# EUROPEAN PATENT APPLICATION

(11) **EP 4 813 978 A1**
(43) Date of publication of application: **30.09.2026**
(21) Application number: 24892744.4
(22) Date of filing: 06.06.2024
(51) Int. Cl.: C07D 401/14, C07D 471/04, C07D 487/04, A61K 31/517, A61K 31/519, A61P 35/00

(54) **COMPOUND TARGETING UBIQUITIN KRAS PROTEIN DEGRADATION AGENT, AND USE THEREOF**

(30) Priority: 20.11.2023 CN 202311543432; 24.01.2024 CN 202410097043; 02.02.2024 CN 202410145511; 17.05.2024 CN 202410621599
(71) Applicant: Betta Pharmaceuticals Co., Ltd, Yuhang Hangzhou Zhejiang 311100 (CN)
(72) Inventor: XU, Renqi, Hangzhou, Zhejiang 311100 (CN); YANG, Xiaofeng, Hangzhou, Zhejiang 311100 (CN); ZHANG, Hongbo, Hangzhou, Zhejiang 311100 (CN); LU, Lu, Hangzhou, Zhejiang 311100 (CN); WANG, Ze, Hangzhou, Zhejiang 311100 (CN); ZHENG, Hang, Hangzhou, Zhejiang 311100 (CN); HE, Jiangqi, Hangzhou, Zhejiang 311100 (CN); LI, Tengfei, Hangzhou, Zhejiang 311100 (CN); FANG, Longcheng, Hangzhou, Zhejiang 311100 (CN); LIN, Yuanwang, Hangzhou, Zhejiang 311100 (CN); LU, Yuan, Hangzhou, Zhejiang 311100 (CN); WU, Hao, Hangzhou, Zhejiang 311100 (CN); ZHOU, Quan, Hangzhou, Zhejiang 311100 (CN); LAN, Hong, Hangzhou, Zhejiang 311100 (CN); WANG, Jiabing, Beijing 100176 (CN); DING, Lieming, Hangzhou, Zhejiang 311100 (CN)
(74) Representative: Mathys & Squire
(86) International application number: PCT/CN2024/097697
(87) International publication number: WO 2025/107579

(57) **Abstract**

A compound targeting KRAS protein degradation agent and a use thereof. The compound is a compound having an F-L-M structure, or a tautomer, racemate, enantiomer, or diastereomer thereof, or a mixture thereof or a pharmaceutically acceptable salt thereof, wherein F is a KRAS protein binding fragment, L is a linking unit for linking F and M, and M is a VHL binding fragment. The compound can be used for treating diseases caused by KRAS mutation or amplification.

## Description

### TECHNICAL FIELD

The present application relates to the technical field of medicines, and specifically relates to a compound targeting pan-KRAS protein degradation agent and use thereof.

### BACKGROUND

PROTAC is a heterobifunctional molecule. One end of the molecule is a ligand that binds to a target protein, and the other end is a ligand of an E3 ligase, with the two ends connected by an appropriate chain. Degradation of the target protein by PROTAC is achieved through a ubiquitin-proteasome system (UPS). The general process is as follows: the PROTAC molecule binds to the protein of interest (POI) and the E3 ligase to form a ternary complex, so that the protein of interest is marked with a ubiquitin molecule, and then the ubiquitinated protein is recognized and degraded by the intracellular proteasome 26S.

The traditional small molecules and antibodies all inhibit the function of target proteins through an action mode of "occupancy-driven" to exert therapeutic effects on diseases. This action mode requires a relatively high concentration of inhibitor or monoclonal antibody in order to occupy the active site of the target and block the transduction of downstream signaling pathways. In contrast, PROTAC is "event-driven", which does not affect the function of the protein but mediates the degradation of a pathogenic target protein. As long as PROTAC mediates the formation of the ternary complex and marks the target protein with ubiquitination, PROTAC can theoretically be recycled and reused, and thus a catalytic dose can exert its effect. It has characteristics such as a wider range of action, higher activity, the ability to target "undruggable" targets, improved selectivity, activity and safety, and the ability to overcome drug resistance.

The activity of KRAS is altered due to the conversion of its binding with GDP or GTP. KRAS is in an inactive state when binding to GDP and is activated when binding to GTP. When KRAS protein is mutated, GAP is restricted from entering GTP, thereby preventing hydrolysis and forming a continuously activated GTP-bound state. Due to the high affinity of mutant KRAS for guanosine triphosphate (GTP), as well as factors such as small catalytic sites and smooth protein surfaces making it difficult to target, the development of small molecule inhibitors has always been challenging, resulting in an "undruggable" characteristic of KRAS. In one aspect, KRAS binds to GDP and GTP with an affinity of picomolar concentrations, which seriously hinders the development of a nucleotide-competitive inhibitor. In another aspect, KRAS protein lacks an ideal small molecule binding pocket, making it difficult to design a high-affinity allosteric inhibitor.

Cancers caused by RAS mutations account for 25% to 30% of all human cancers. Among these, KRAS mutations account for 86% of the three RAS mutations. Among all KRAS mutations, there are multiple mutations such as G12D (35%), G12V (29%), G12C (21%), G13D (6.7%), G12R (4.3%), G12A (4.1%), etc. If these different KRAS mutations can be targeted simultaneously, it would greatly satisfy clinical needs and exhibit great commercial value.

However, available KRAS inhibitors are facing a predicament of limited efficacy and high susceptibility to drug resistance in clinical practice. If the characteristics, including anti-resistance, high efficacy, etc., of PROTAC can be combined to develop novel pan-KRAS PROTAC degrader drugs for the treatment of diseases caused by KRAS mutation or amplification, it will have great social significance and commercial value.

### SUMMARY

The present application provides a novel pan-KRAS PROTAC degrader, which can be used for treating a disease caused by KRAS mutations or amplification.

In one aspect, the present application provides a compound having an F-L-M structure, or a tautomer, racemate, enantiomer, diastereomer thereof, or a mixture thereof, or a pharmaceutically acceptable salt thereof, wherein:
F is a KRAS protein binding fragment represented by general formula (I); 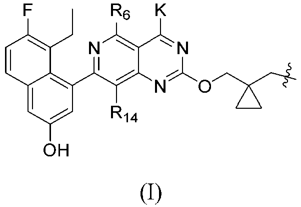
K is N-containing 6- to 12-membered heterocyclyl; K is optionally further substituted by one or more Rₐ;
R₁₄ is halogen;
R₆ is selected from the group consisting of H, hydroxy, amino, cyano, halogen, C₁₋₆ alkyl, C₁₋₆ haloalkyl, C₁₋₆ haloalkoxy, and C₁₋₆ alkoxy; R₆ is preferably H; or
R₆ and K, together with the atoms to which they are bound, form 3- to 14-membered heterocyclyl, wherein the 3- to 14-membered heterocyclyl is optionally further substituted by one or more Rₐ;
L is a linking unit for linking F and M; L is selected from the group consisting of 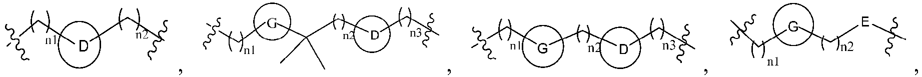 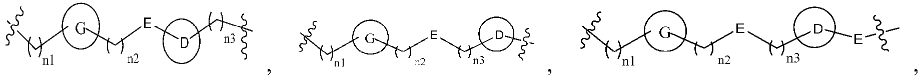 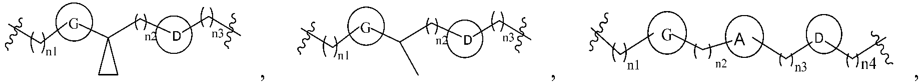 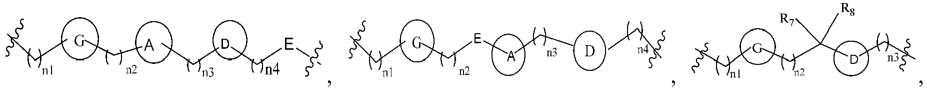 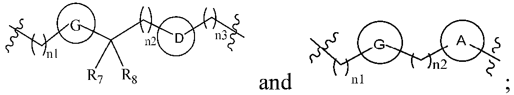
wherein,
ring G and ring D are each independently selected from the group consisting of C₆₋₁₄ aryl, 5-to 14-membered heteroaryl, C₃₋₁₄ cycloalkyl and 3- to 14-membered heterocyclyl, wherein the C₆₋₁₄ aryl, 5- to 14-membered heteroaryl, C₃₋₁₄ cycloalkyl or 3- to 14-membered heterocyclyl is optionally further substituted by one or more Rₐ;
each ring A is independently 3- to 7-membered N-containing heterocyclyl; the 3- to 7-membered N-containing heterocyclyl is optionally further substituted by one or more Rₐ; preferably, each ring A is independently 3- to 6-membered N-containing heterocyclyl; the 3- to 6-membered N-containing heterocyclyl is optionally further substituted by one or more Rₐ;
each E is independently absent or selected from the group consisting of -O-, -NH-, 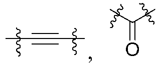 and -NCH₃-;
R₇ and R₈ are each independently selected from the group consisting of H, halogen, hydroxy, amino, oxo, cyano, halogen, C₁₋₆ alkyl, C₁₋₆ haloalkyl, C₁₋₆ haloalkoxy and C₁₋₆ alkoxy; or
R₇ and R₈, together with the atoms to which they are bound, form C₃₋₁₄ cycloalkyl or 3- to 14-membered heterocyclyl; the C₃₋₁₄ cycloalkyl or 3- to 14-membered heterocyclyl is optionally further substituted by one or more Rₐ;
n1, n2, n3 and n4 are each independently selected from the group consisting of integers ranging from 0 to 10, and are preferably each independently 0, 1, 2, 3 or 4;
M is a VHL binding fragment represented by general formula (II); 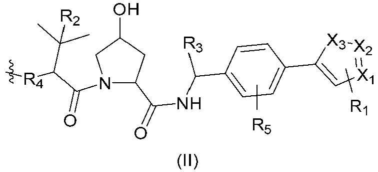
wherein, X₁ is selected from the group consisting of C, CH and N;
X₂ is selected from the group consisting of C, CH and N;
X₃ is selected from the group consisting of N, NH, O and S;
R₁ is selected from the group consisting of H, C₁₋₆ alkyl, C₁₋₆ haloalkyl, C₁₋₆ alkoxy, C₁₋₆ haloalkoxy, halogen and cyano, and is preferably methyl, ethyl, F or cyano;
R₂ is selected from the group consisting of H, C₁₋₆ alkyl and C₁₋₆ haloalkyl, and is preferably H or methyl;
R₃ is selected from the group consisting of H, C₁₋₆ alkyl, C₁₋₆ haloalkyl, C₁₋₆ hydroxyalkyl, -(CH₂)ₙN(R₉)₂, -(CH₂)ₙ-CO-OR₉ and -(CH2) ₙ-CO-N(R₉)₂, wherein each R₉ is independently selected from the group consisting of H, C₁₋₆ alkyl, C₁₋₆ haloalkyl and C₁₋₆ alkoxy; preferably, the -(CH₂)ₙ-CO-N(R₉)₂ is -(CH₂)ₙ-CO-NH-(CH₂)ₙ-CH₃;
n is selected from the group consisting of 0, 1, 2 and 3;
R₄ is selected from the group consisting of 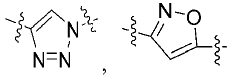 and -NH-;
R₅ is selected from the group consisting of H, C₁₋₆ alkyl and C₁₋₆ haloalkyl;
Rₐ is independently selected from the group consisting of H, hydroxy, amino, oxo, cyano, halogen, C₁₋₆ alkyl, C₁₋₆ haloalkyl, C₁₋₆ alkoxy, -C₀₋₃ alkylene-OR_{b}, -OC(=O)C₁₋₆ alkyl, -C₀₋₃ alkylene-SR_{b}, -C₀₋₃ alkylene-N(R_{b})₂, -C₀₋₃ alkylene-S(=O)R_{b}, -C₀₋₃ alkylene-S(=O)₂R_{d}, -C₀₋₃ alkylene-SR_{b}, -C₀₋₃ alkylene-S(R_{b})₅, -C₀₋₃ alkylene-C(=O)R_{b}, -C₀₋₃ alkylene-C(=O)OR_{b}, -C₀₋₃ alkylene-C(=O)N(R_{b})₂, C₂₋₆ alkenyl, 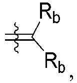 C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl, and -C₀₋₃ alkylene-(5- to 18-membered heteroaryl), wherein hydroxy, amino, C₁₋₆ alkyl, C₁₋₆ haloalkyl, C₁₋₆ alkoxy, C₀₋₃ alkylene, C₂₋₆ alkenyl, C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl or -C₀₋₃ alkylene-(5- to 18-membered heteroaryl) is optionally further substituted by one or more R_{b};
each R_{b} is independently H, halogen, hydroxy, amino, cyano, C₁₋₆ alkyl, C₃₋₆ cycloalkyl, C₁₋₆ haloalkyl, C₃₋₁₄ cycloalkyl, 3- to 14-membered heterocyclyl, C₆₋₁₈ aryl or 5- to 18-membered heteroaryl, wherein hydroxy, amino, C₁₋₆ alkyl, C₃₋₆ cycloalkyl, C₁₋₆ haloalkyl, C₃₋₁₄ cycloalkyl, 3- to 14-membered heterocyclyl, C₆₋₁₈ aryl or 5- to 18-membered heteroaryl is optionally further substituted by one or more halogen, C₁₋₆ alkyl or C₁₋₆ haloalkyl; or two R_{b} bound to one atom, together with the atom to which they are bound, form C₃₋₁₄ cycloalkyl, 3- to 14-membered heterocyclyl, C₆₋₁₈ aryl or 5- to 18-membered heteroaryl, wherein the C₃₋₁₄ cycloalkyl, 3- to 14-membered heterocyclyl, C₆₋₁₈ aryl or 5- to 18-membered heteroaryl is optionally further substituted by one or more halogen, amino, hydroxy, cyano, C₁₋₆ alkyl, C₃₋₆ cycloalkyl or C₁₋₆ haloalkyl.

In some embodiments of the present application, K is selected from the group consisting of

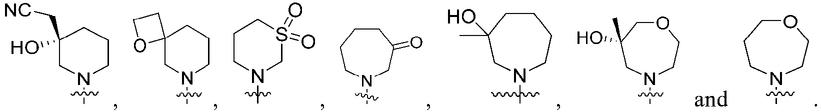

In some embodiments of the present application, F is

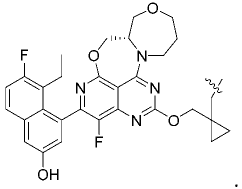

In some embodiments of the present application, R₄ is

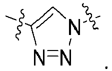

In some embodiments of the present application, R₄ is

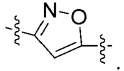

In some embodiments of the present application, R₄ is -NH-.

In some embodiments of the present application, ring G or ring D in L is each independently selected from the group consisting of C₆₋₁₄ aryl, 5- to 14-membered heteroaryl, C₃₋₁₄ cycloalkyl and 3- to 14-membered heterocyclyl, wherein the C₆₋₁₄ aryl, 5- to 14-membered heteroaryl, C₃₋₁₄ cycloalkyl or 3- to 14-membered heterocyclyl is optionally further substituted by one or more halogen or C₁₋₃ alkyl;
C₆₋₁₄ aryl is preferably 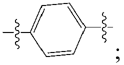
5- to 14-membered heteroaryl is preferably selected from the group consisting of 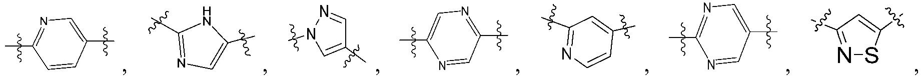 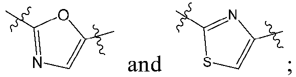
3- to 14-membered heterocyclyl is preferably selected from the group consisting of 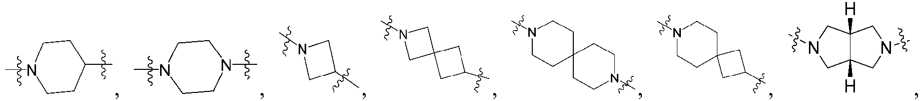 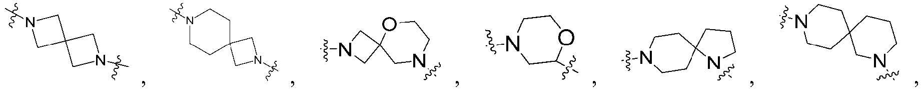 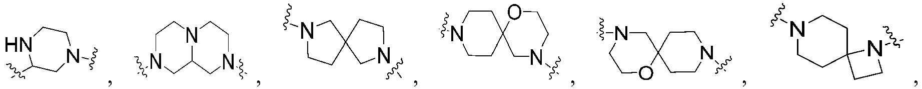 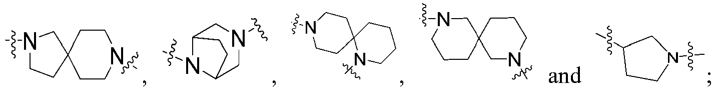
C₃₋₁₄ cycloalkyl is preferably selected from the group consisting of 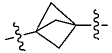 and

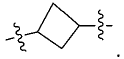

In some embodiments of the present application, each ring D is independently 5- to 6-membered nitrogen-containing heteroaryl, wherein the 5- to 6-membered nitrogen-containing heteroaryl is optionally further substituted by one or more Rₐ;
each ring G is independently selected from the group consisting of C₆₋₁₄ aryl, 5- to 14-membered heteroaryl, C₃₋₁₄ cycloalky and 3- to 14-membered heterocyclyl, wherein the C₆₋₁₄ aryl, 5- to 14-membered heteroaryl, C₃₋₁₄ cycloalkyl or 3- to 14-membered heterocyclyl is optionally further substituted by one or more Rₐ;
each ring A is independently 3- to 7-membered N-containing heterocyclyl; the 3- to 7-membered N-containing heterocyclyl is optionally further substituted by one or more Rₐ;
each E is independently absent or selected from the group consisting of -O-, -NH-, 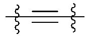 and -NCH₃-;
R₇ and R₈ are each independently selected from the group consisting of H, halogen, hydroxy, amino, oxo, cyano, halogen, C₁₋₆ alkyl, C₁₋₆ haloalkyl and C₁₋₆ alkoxy; or
R₇ and R₈, together with the atoms to which they are bound, form C₃₋₁₄ cycloalkyl or 3- to 14-membered heterocyclyl; the C₃₋₁₄ cycloalkyl or 3- to 14-membered heterocyclyl is optionally further substituted by one or more Rₐ;
n1, n2, n3 and n4 are each independently selected from the group consisting of integers ranging from 0 to 10, and are preferably each independently 0, 1, 2, 3 or 4.

In some embodiments of the present application, each ring A is independently 3- to 7-membered N-containing heterocyclyl; the 3- to 7-membered N-containing heterocyclyl is optionally further substituted by one or more halogen or C₁₋₃ alkyl.

Further, 3- to 7-membered N-containing heterocyclyl is

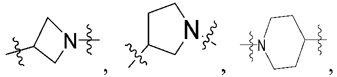

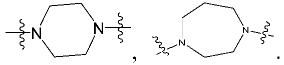

In some embodiments of the present application, E is -O-.

In some embodiments of the present application, E is -NH-.

In some embodiments of the present application, E is -NCH₃-.

In some embodiments of the present application, ring D is independently 5- to 6-membered nitrogen-containing heteroaryl, the 5- to 6-membered heteroaryl is selected from the group consisting of 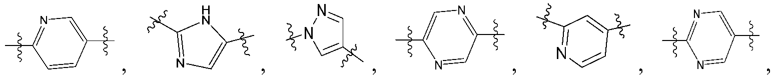 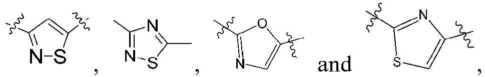 and the 5- to 6-membered nitrogen-containing heteroaryl is optionally further substituted by one or more Rₐ; Rₐ is selected from the group consisting of H, hydroxy, amino, oxo, cyano, halogen, C₁₋₆ alkyl, C₁₋₆ haloalkyl and C₁₋₆ alkoxy.

In some embodiments of the present application, ring G is independently selected from the group consisting of C₆₋₁₄ aryl, 5- to 14-membered heteroaryl, C₃₋₁₄ cycloalkyl and 3- to 14-membered heterocyclyl, and the C₆₋₁₄ aryl, 5- to 14-membered heteroaryl, C₃₋₁₄ cycloalkyl or 3- to 14-membered heterocyclyl is optionally further substituted by one or more Rₐ;
C₆₋₁₄ aryl is preferably 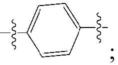
5- to 14-membered heteroaryl is preferably selected from the group consisting of 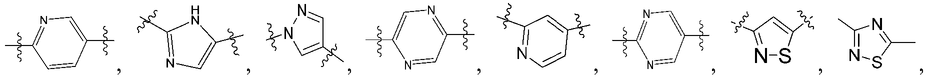 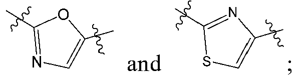
3- to 14-membered heterocyclyl is preferably selected from the group consisting of 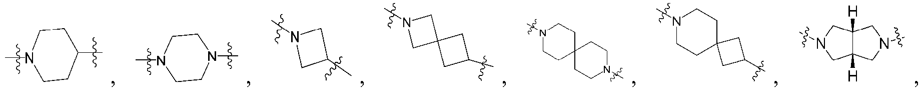 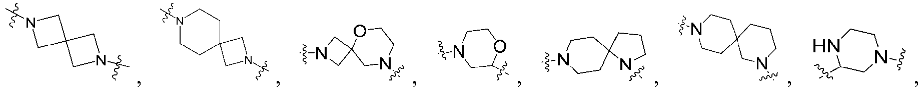 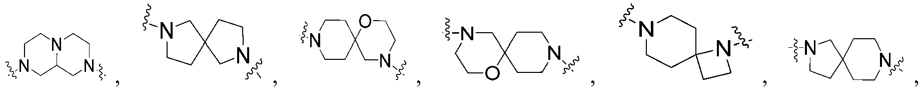 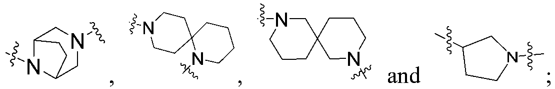
C₃₋₁₄ cycloalkyl is preferably selected from the group consisting of 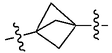 and 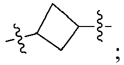
Rₐ is selected from the group consisting of H, hydroxy, amino, oxo, cyano, halogen, C₁₋₆ alkyl, C₁₋₆ haloalkyl and C₁₋₆ alkoxy.

In some embodiments of the present application, each ring G is independently 3- to 14-membered heterocyclyl, and the 3- to 14-membered heterocyclyl is optionally further substituted by one or more Rₐ; and the 3- to 14-membered heterocyclyl is preferably selected from the group consisting of

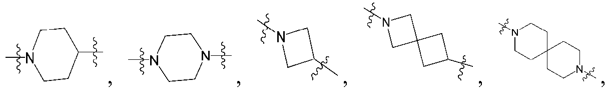

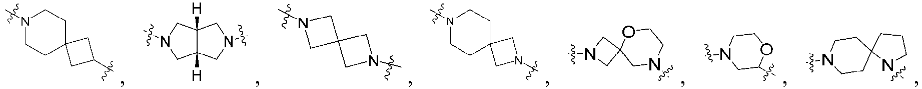

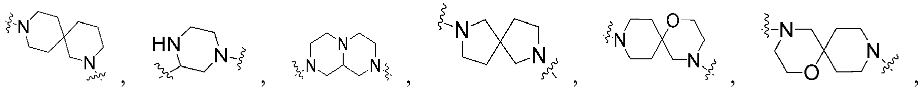

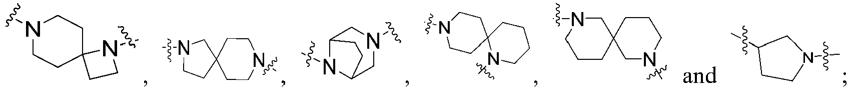

Rₐ is selected from the group consisting of H, hydroxy, amino, oxo, cyano, halogen, C₁₋₆ alkyl, C₁₋₆ haloalkyl and C₁₋₆ alkoxy.

In some embodiments of the present application, L is selected from the group consisting of

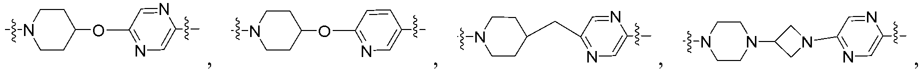

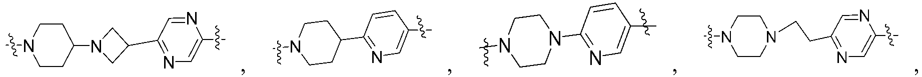

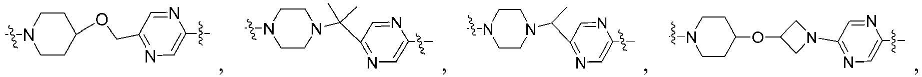

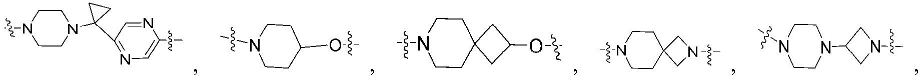

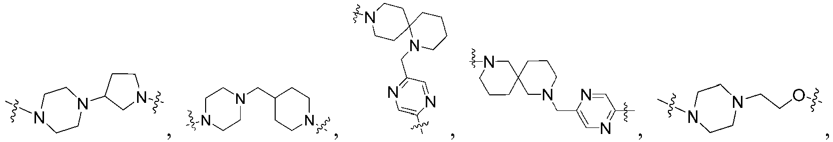

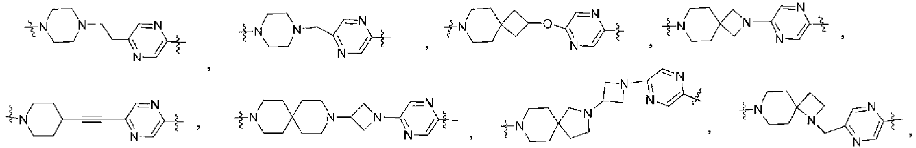

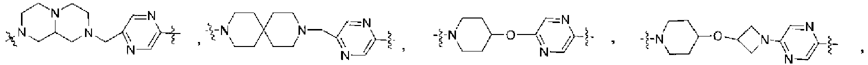

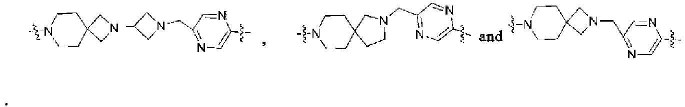

In some embodiments of the present application, L is selected from the group consisting of

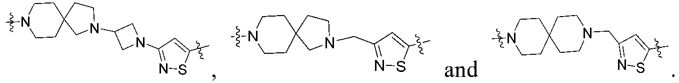

In some embodiments of the present application, R₃ is selected from the group consisting of H, methyl, hydroxymethyl, -CH₂-CO-O-CH₃, -CH₂-N(CH₃)₂ and -CH₂-CO-NH-CH₃.

In some embodiments of the present application, M is selected from the group consisting of

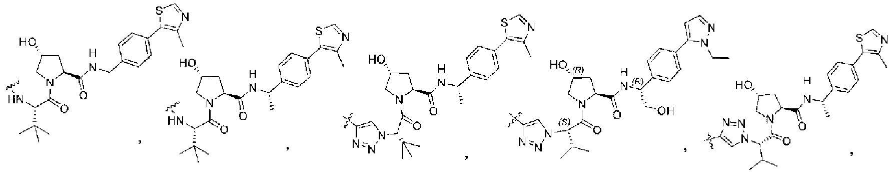

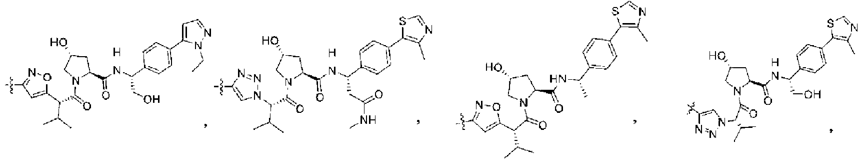

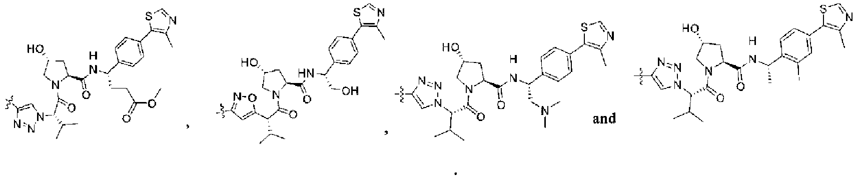

In some embodiments of the present application, the compound having the F-L-M structure is selected from the group consisting of the following compounds:

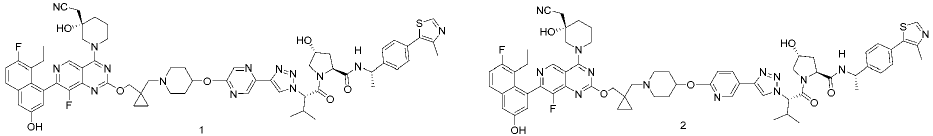

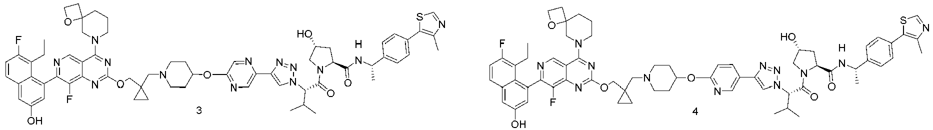

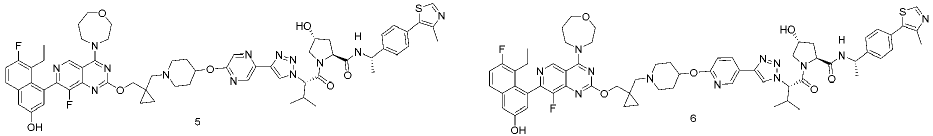

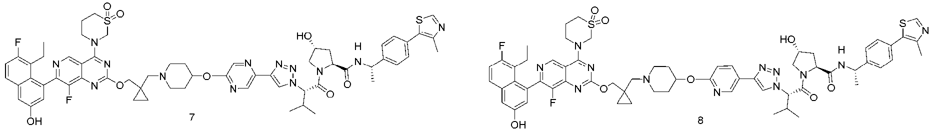

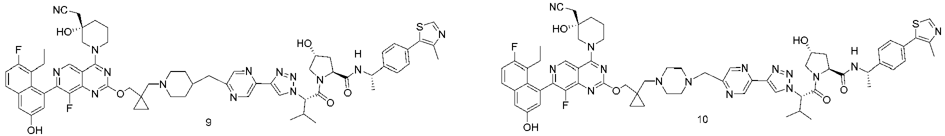

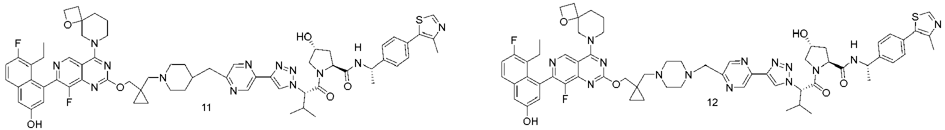

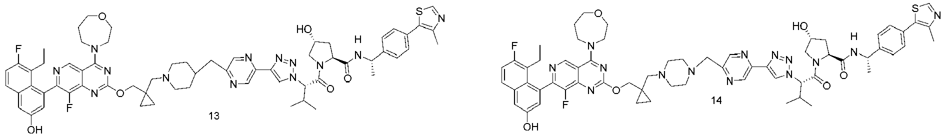

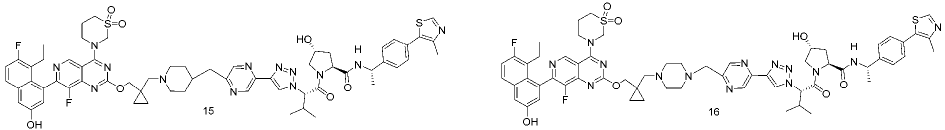

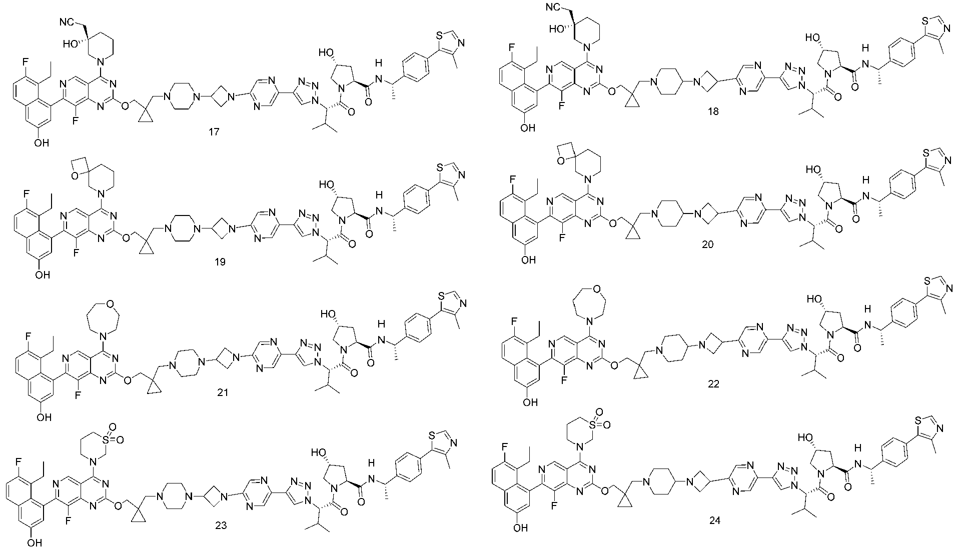

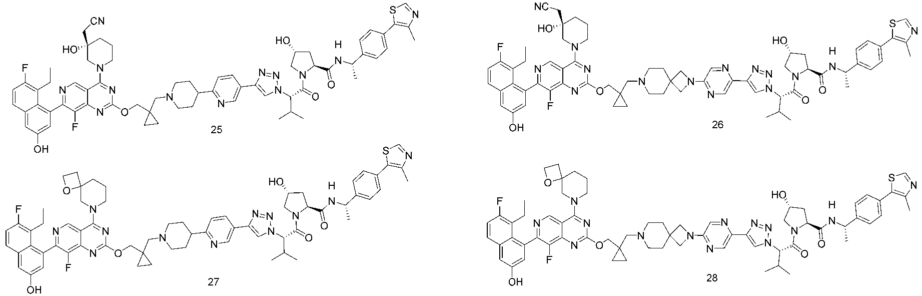

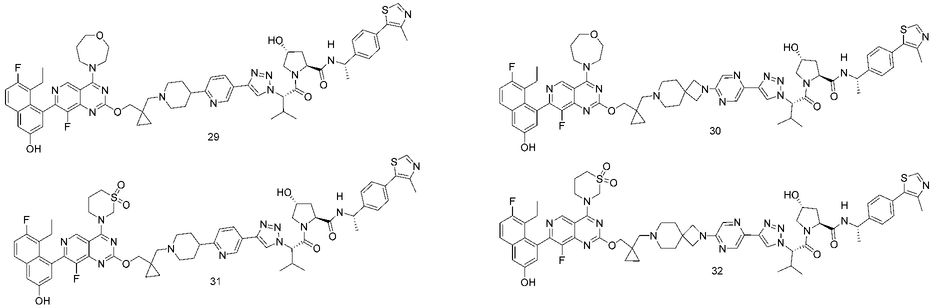

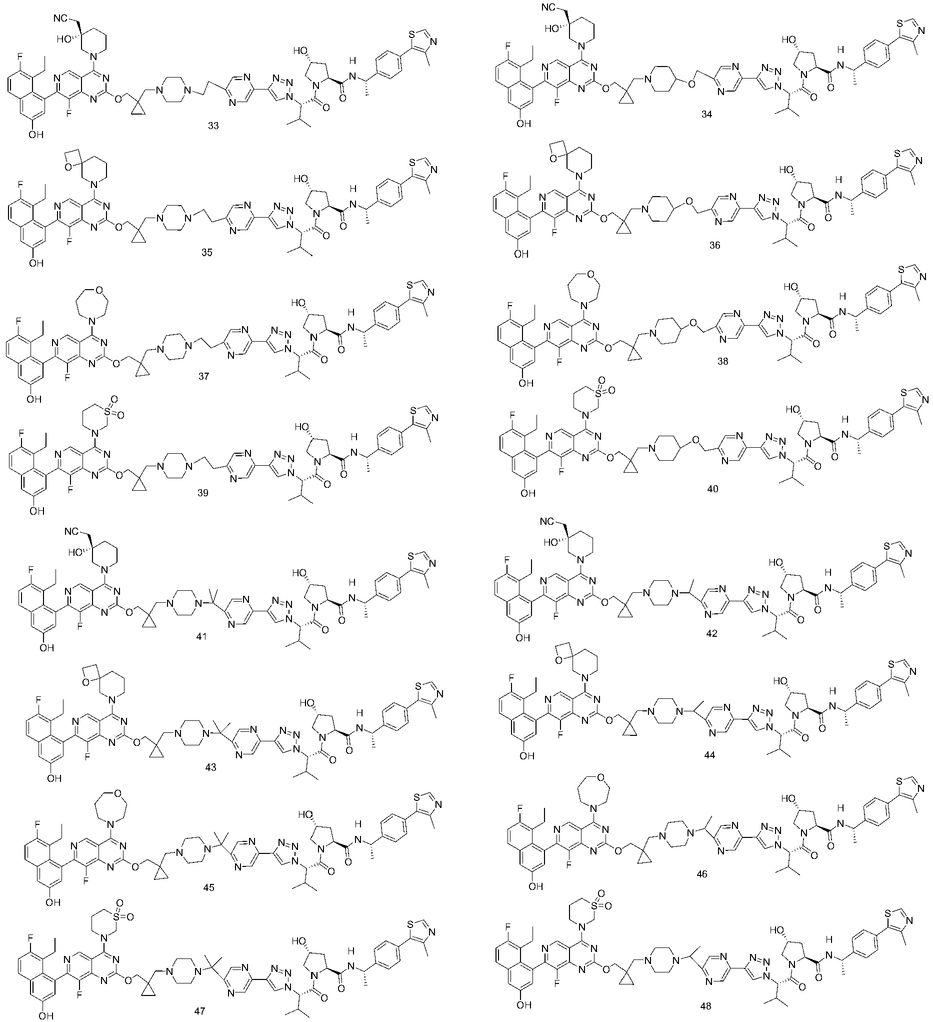

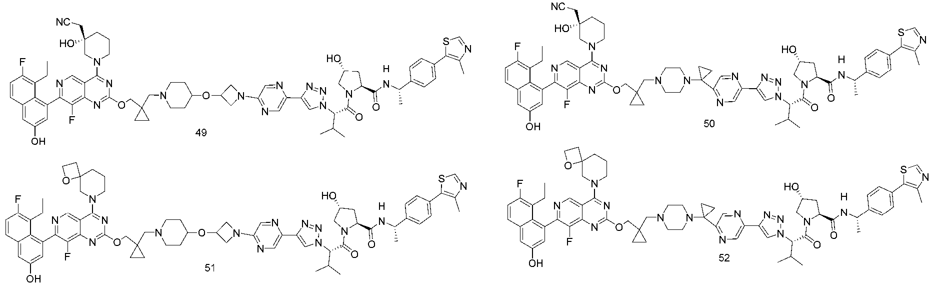

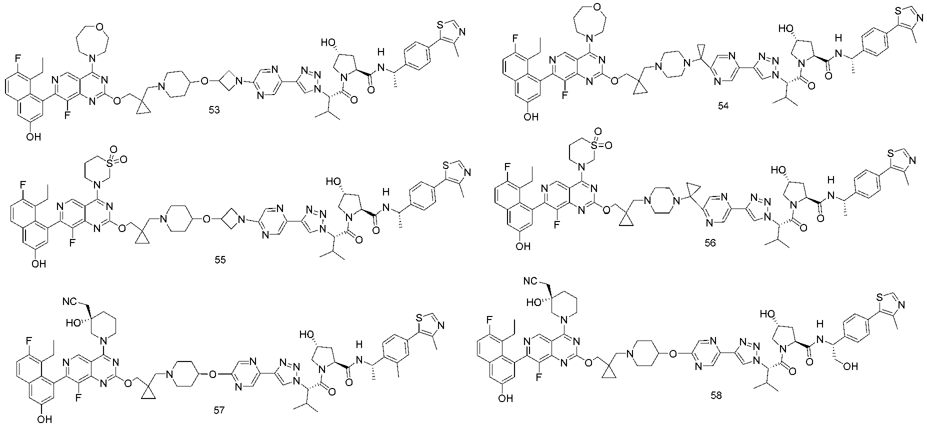

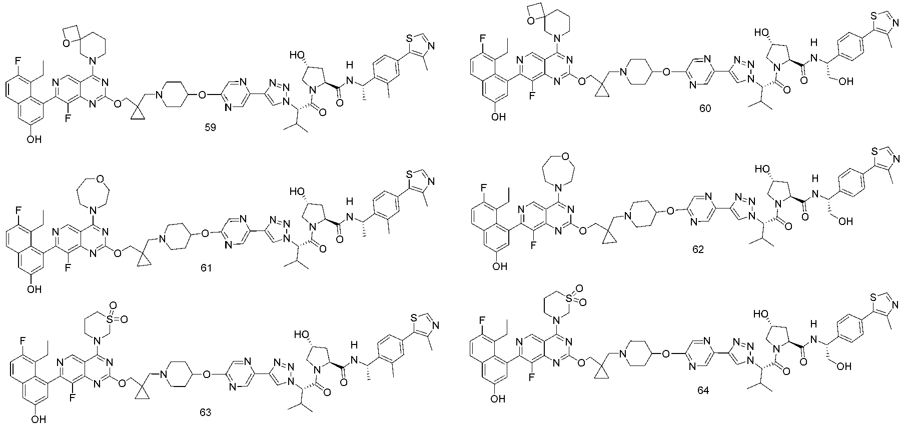

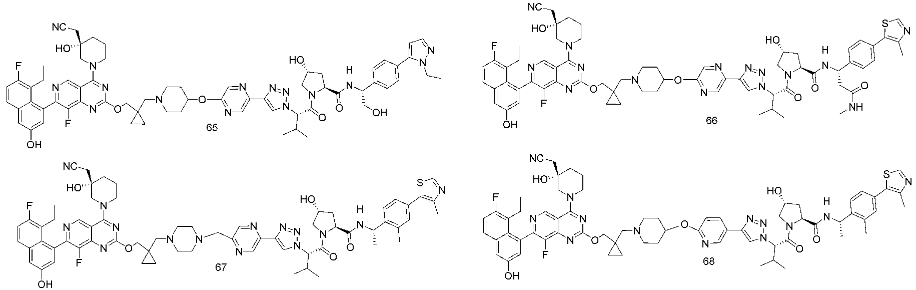

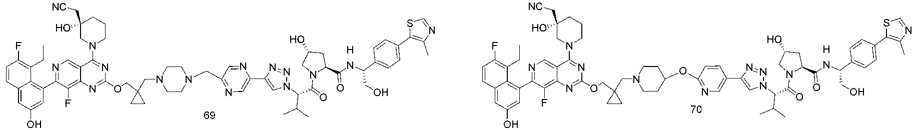

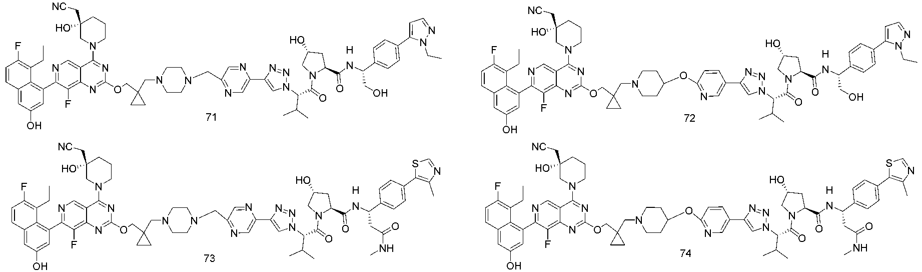

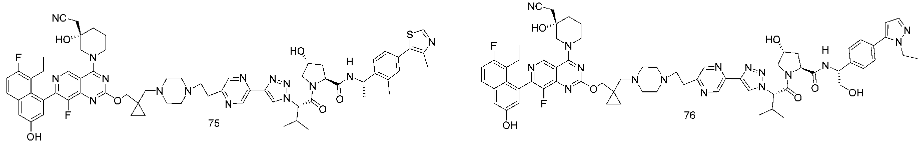

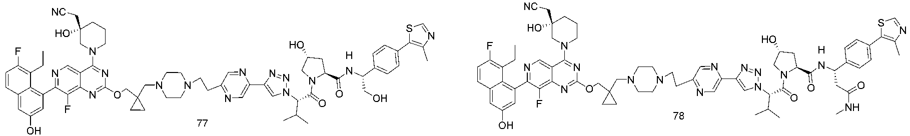

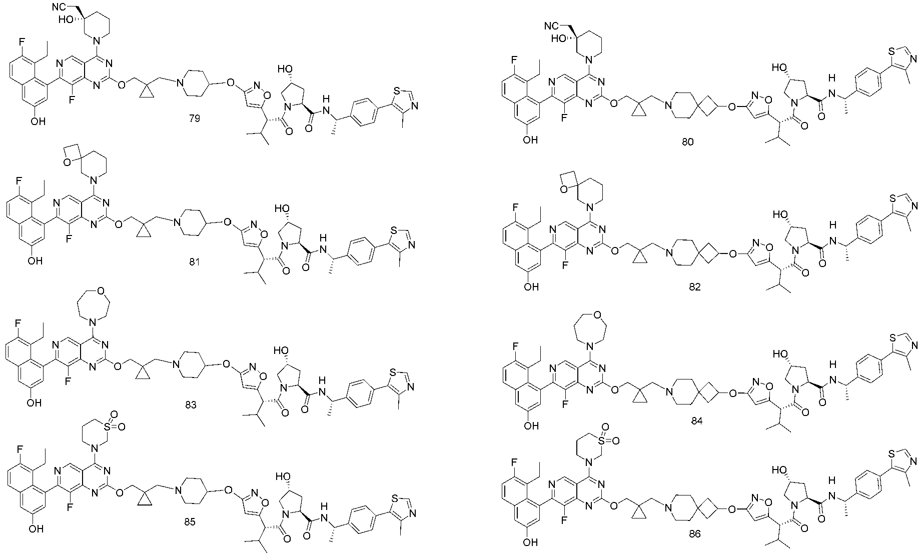

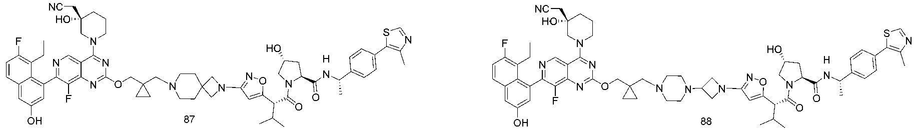

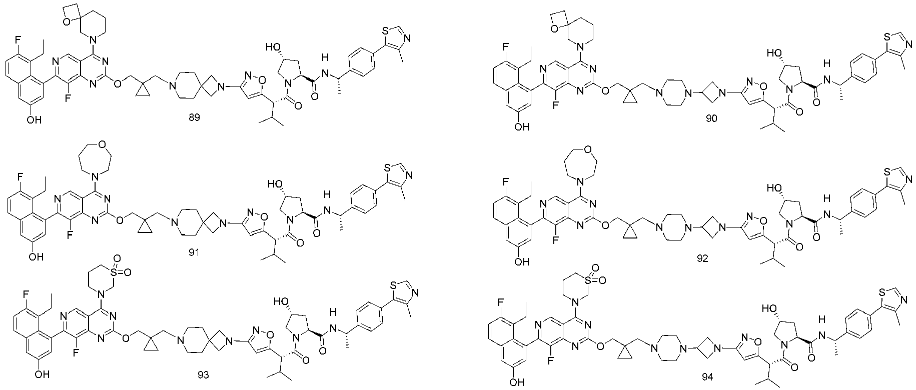

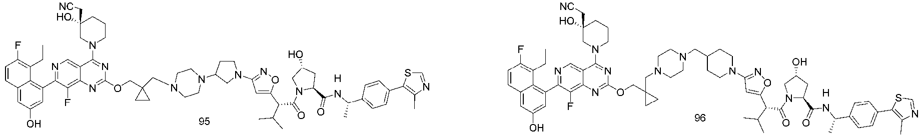

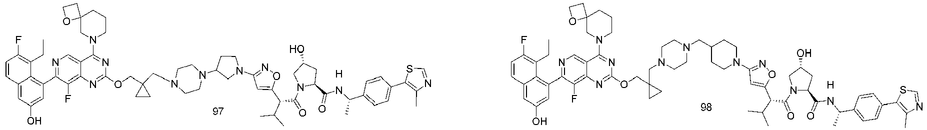

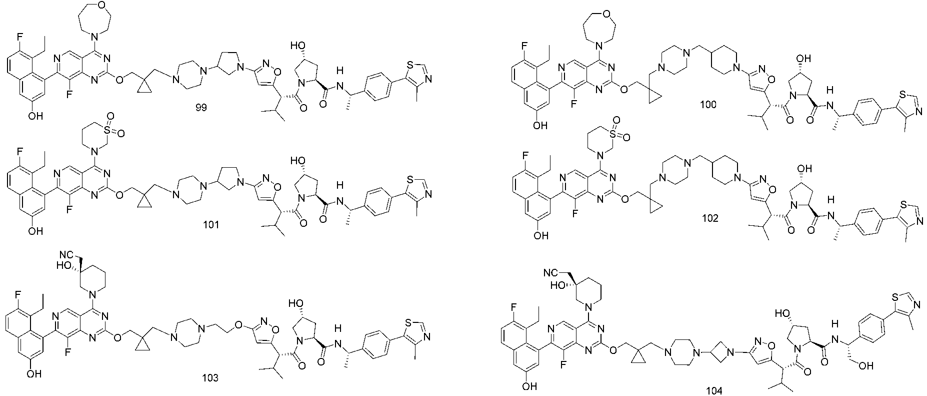

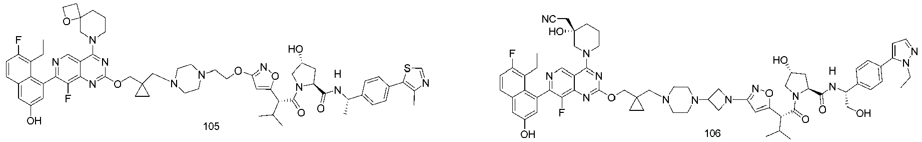

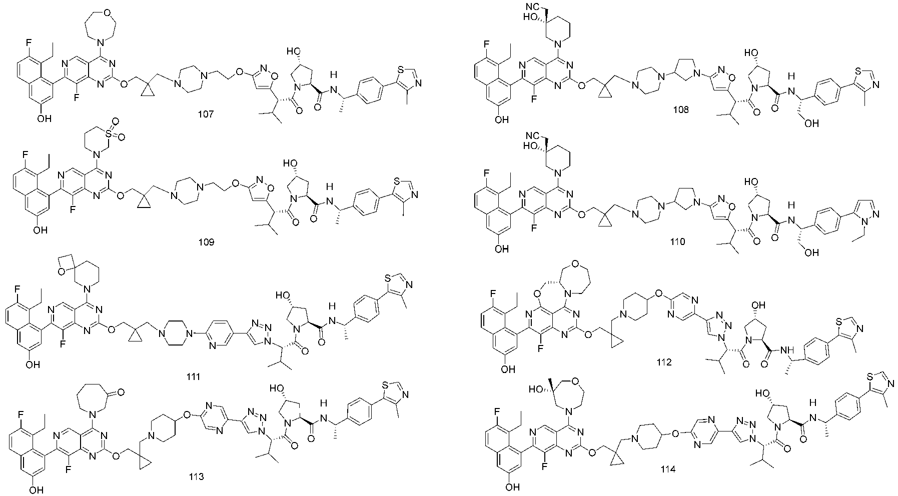

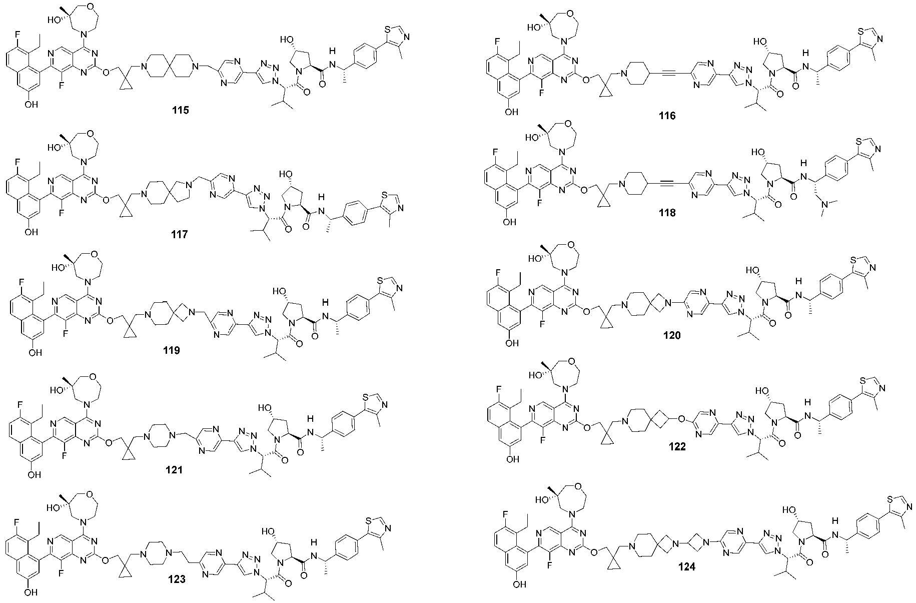

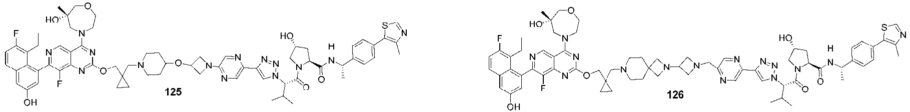

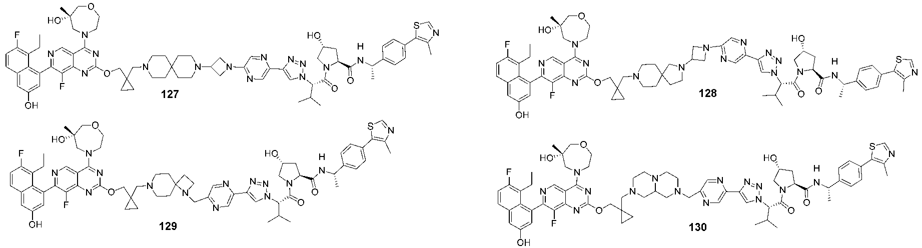

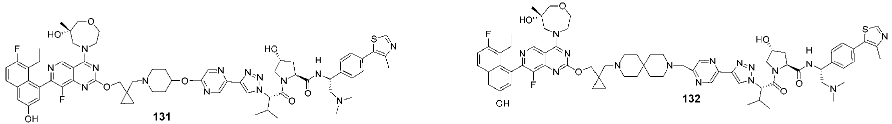

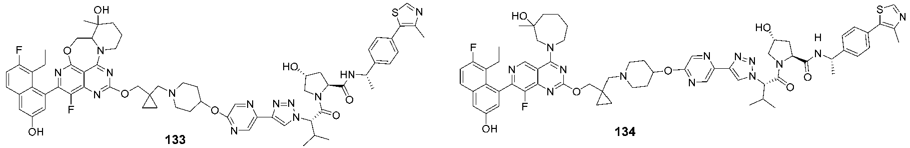

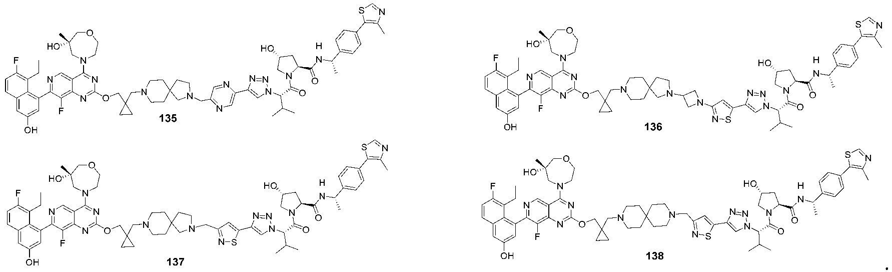

In another aspect, the present application provides a pharmaceutical composition comprising any one of the above compounds represented by the general formula having the F-L-M structure of the present application, or the tautomer, racemate, enantiomer, diastereomer thereof, or the mixture thereof, or the pharmaceutically acceptable salt thereof, and one or more pharmaceutically acceptable carriers, diluents, or excipients.

The present application further provides use of any one of the above compounds represented by the general formula having the F-L-M structure, or the tautomer, racemate, enantiomer, diastereomer thereof, or the mixture thereof, or the pharmaceutically acceptable salt thereof, or the pharmaceutical composition comprising the same in the manufacture of a medicament for regulating ubiquitination and degradation of KRAS protein in a subject.

The present application further provides use of any one of the above compounds represented by the general formula having the F-L-M structure, or the tautomer, racemate, enantiomer, diastereomer thereof, or the mixture thereof, or the pharmaceutically acceptable salt thereof, or the pharmaceutical composition comprising the same in the manufacture of a medicament for treating and/or preventing a KRAS-mediated disease or a KRAS-dependent disease, wherein the KRAS-mediated disease is preferably a tumor.

In certain embodiments, the disease is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumor, intrahepatic cholangiocarcinoma, myelodysplastic syndrome, malignant glioma, prostate cancer, thyroid cancer, Schwann cell tumor, pulmonary squamous cell carcinoma, lichenoid keratosis, synovial sarcoma, skin cancer, pancreatic cancer, testicular cancer and liposarcoma.

Unless otherwise specified, the general chemical term used in the structural general formulae has its common meaning.

For example, unless otherwise specified, the term "halogen" used in the present application refers to fluorine, chlorine, bromine or iodine.

Unless otherwise specified, when the linking group involved in the present application does not indicate its connection direction, the connection direction is arbitrary. For example, when the linking group L in F-L-M in the compound is 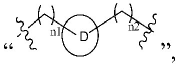 L can link F and M according to the left-to-right direction to form 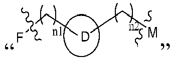 and can also link F and M according to the right-to-left direction to form

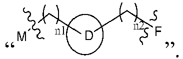

Preferably, in the linking order of L as described in the present application, ring D is close to M (VHL end), and ring G is close to F (KRAS end).

In the present application, unless otherwise specified, "alkyl" includes a linear or branched chain monovalent saturated hydrocarbon group. For example, alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl, 2-methylpentyl, etc. Similarly, "1-6" in "C₁₋₆ alkyl" means that the group comprises 1, 2, 3, 4, 5 or 6 carbon atoms arranged in a linear or branched chain form.

The term "alkoxy" refers to the oxygen ether form of the aforementioned linear or branched chain alkyl, i.e., -O-alkyl.

The term "alkylene" refers to a divalent alkyl linking group. Alkylene is formally an alkane in which two C-H bonds are replaced by points of attachment of the alkylene to the rest of the compound. Similarly, "C₁₋₃" in "C₁₋₃ alkylene" means the alkylene group containing 1, 2, or 3 carbon atoms, including but not limited to methylene, 1,2-ethylene, 1,3-propylene, or 1,2-isopropylene.

The term "haloalkyl" refers to an alkyl group in which one or more H atoms are replaced by halogen atoms.

The term "oxo" or "oxo group" refers to an oxygen atom in the form of a divalent substituent, which forms a carbonyl group when attached to C, and forms a sulfoxide group, a sulfone group, or an N-oxide group when attached to a heteroatom.

In the present application, unless otherwise specified, the terms "aryl ring", "aromatic ring" or "aromatic heterocyclic ring" refer to a polyunsaturated carbon ring or heterocyclic ring with aromatic characteristics (having (4n+2) non-localized π electrons, where n is an integer).

The term "aryl", in the present application, unless otherwise specified, refers to an unsubstituted or substituted monocyclic or fused ring aromatic group containing carbon ring atoms. C₆₋₁₈ aryl is preferred. More preferably, aryl is a C₆₋₁₀ monocyclic or bicyclic aromatic ring group. Phenyl or naphthalenyl is preferred; and naphthalenyl is the most preferred. The aryl ring may be fused to a heteroaryl, heterocyclyl, or cycloalkyl group, wherein the ring attached to the parent structure is an aryl ring; non-limiting examples include, but are not limited to, benzocyclopentyl.

The term "heterocyclyl" refers to a ring system having at least one cyclized alkyl or cyclic alkenyl group containing a heteroatom selected from the group consisting of N, O and/or S. The heterocyclyl may include monocyclic or polycyclic rings (e.g., having 2, 3, or 4 fused rings, spiro rings, bridged rings, etc.). Heterocyclyl may be linked to another part of the compound via a ring-forming carbon atom or a ring-forming heteroatom. 3- to 14-membered heterocyclyl is preferred. 3- to 8-membered heterocyclyl, 3- to 7-membered heterocyclyl and 3- to 6-membered heterocyclyl are more preferred; wherein "3- to 14-membered" in 3- to 14-membered heterocyclyl refers to a heterocyclyl composed of 3 to 14 ring-forming atoms selected from the group consisting of C, N, O and S; wherein nitrogen or sulfur heteroatoms can be oxidized selectively, and nitrogen heteroatoms can be quaternized selectively. Examples of these heterocyclyl include, but are not limited to 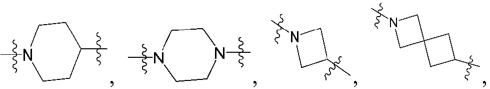 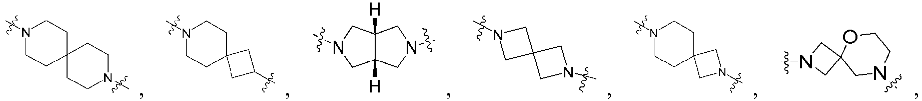 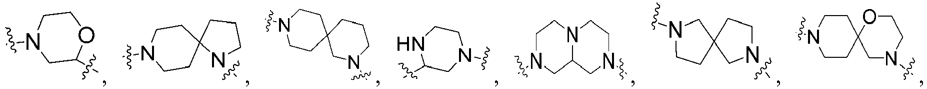 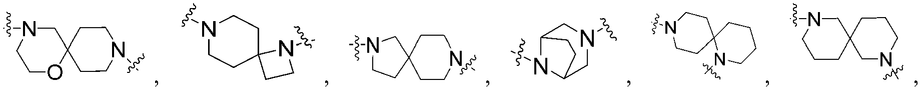 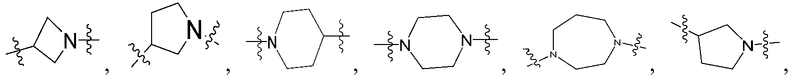 pyrrolidinyl, piperidinyl, piperazinyl, oxopiperazinyl, oxopiperidinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiomorpholinyl sulfoxide, thiomorpholinyl sulfonyl and tetrahydrooxadiazolyl. The heterocyclyl can be fused to an aryl, heteroaryl, or cycloalkyl ring, wherein the ring attached to the parent structure is the heterocyclyl.

The term "heteroaryl", in the present application, unless otherwise specified, refers to a monocyclic or polycyclic (e.g., having 2, 3 or 4 fused rings, spiro rings, bridged rings, etc.) heteroaryl ring with at least one heteroatom selected from the group consisting of N, O and/or S, wherein the nitrogen or sulfur heteroatoms can be oxidized selectively, and the nitrogen heteroatoms can be quaternized selectively. 5- to 18-membered heteroaryl is preferred. 5- to 10-membered heteroaryl and 5- to 6-membered heteroaryl are more preferred; wherein "5- to 18-membered" in 5- to 18-membered heteroaryl refers to a heteroaryl composed of 5 to 18 ring-forming atoms selected from the group consisting of C, N, O and S. 5- to 10-membered heteroaryl groups are more preferred; 5- to 6-membered heteroaryl groups are much more preferred. Examples of heteroaryl include, but are not limited to, thiophenyl, furanyl, imidazolyl, isoxazolyl, oxazolyl, pyrazolyl, pyrrolyl, thiazolyl, thiadiazolyl, triazolyl, pyridinyl, pyridazinyl, indolyl, azaindolyl, indazolyl, benzimidazolyl, benzofuranyl, benzothienyl, benzisoxazolyl, benzothiazolyl, benzothiazolyl, benzothiadiazolyl, benzotriazolyl, adenine, quinolinyl or isoquinolinyl. The heteroaryl can be fused to an aryl, heterocyclyl or cycloalkyl ring, wherein the ring attached to the parent structure is the heteroaryl ring.

The term "cycloalkyl" refers to a ring system having at least one cyclized alkyl group. C₃₋₁₄ cycloalkyl is preferred, and C₃₋₈ cycloalkyl, C₃₋₆ cycloalkyl and C₅₋₇ cycloalkyl are more preferred; wherein "C₃₋₁₄" means that the cycloalkyl can have 3, 4, 5, 6, 7, 8, 9, 10, 11, 12, 13 or 14 ring-forming atoms. Cycloalkyl can include monocyclic ring and polycyclic ring (e.g., having 2, 3 or 4 fused rings, spiro rings, bridged rings, etc.). In some embodiments, cycloalkyl includes, but is not limited to, cyclopropyl, cyclobutyl, cyclopentyl, 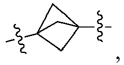 etc.; the cycloalkyl can also be fused to an aryl, heterocyclyl, or heteroaryl ring, wherein the ring attached to the parent structure is the cycloalkyl.

The term "substituted" means that one or more hydrogen atoms in a group are substituted by the same or different substituents, respectively. Typical substituents include, but are not limited to, halogen (F, Cl, Br or I), C₁₋₈ alkyl, C₃₋₁₂ cycloalkyl, -OR¹, -SR¹, =O, =S, -C(O)R¹, -C(S)R¹, =NR¹, -C(O)OR¹, -C(S)OR¹, -NR¹R², -C(O)NR¹R², cyano, nitro, -S(O)₂R¹, -O-S(O₂)OR¹, -O-S(O)₂R¹, -OP(O)(OR¹)(OR²); wherein R¹ and R² are independently selected from the group consisting of -H, C₁₋₆ alkyl, C₁₋₆ haloalkyl and C₃₋₆ cycloalkyl. In some embodiments, the substituent is independently selected from the groups comprising -F, -Cl, -Br, -I, -OH, trifluoromethoxy, ethoxy, propoxy, isopropoxy, n-butoxy, isobutoxy, tert-butoxy, -SCH₃, -SC₂H₅, formyl, -COCH₃, cyano, nitro, -CF₃, amino, dimethylamino, sulfonyl and acetyl.

When the number of a linking group is 0, for example, -(CH₂)₀- indicates that the linking group is a bond.

The term "pharmaceutically acceptable salt" refers to a salt prepared from a pharmaceutically acceptable non-toxic base or acid.

When the compound provided by the present application is an acid, its corresponding salt can be conveniently prepared from a pharmaceutically acceptable non-toxic base, including inorganic bases and organic bases. Salts derived from inorganic bases include salts of aluminum, ammonium, calcium, copper (with high and low valence), ferric iron, ferrous iron, lithium, magnesium, manganese (with high and low valence), potassium, sodium, zinc, etc. Salts of ammonium, calcium, magnesium, potassium and sodium are particularly preferred. Non-toxic organic bases that can be derivatized into pharmaceutically acceptable salts include primary, secondary, and tertiary amines, as well as cyclic amines and substituted amines, such as naturally occurring and synthetic substituted amines. Other pharmaceutically acceptable non-toxic organic bases that can form salts include ion exchange resins and arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, reduced glucosamine, glucosamine, histidine, isopropylamine, lysine, methylglucamine, morpholine, piperazine, piperidine, polyamine resins, procaine, chloroprocaine, purines, theobromine, triethylamine, trimethylamine, tripropylamine, tromethamine, etc.

When the compound provided by the present application is a base, its corresponding salt can be conveniently prepared from pharmaceutically acceptable non-toxic acids, including inorganic acids and organic acids. Such acids include, for example, acetic acid, benzenesulfonic acid, benzoic acid, camphorsulfonic acid, citric acid, ethanesulfonic acid, formic acid, fumaric acid, gluconic acid, glutamic acid, hydrobromic acid, hydrochloric acid, hydroxyethanesulfonic acid, lactic acid, maleic acid, malic acid, mandelic acid, methanesulfonic acid, mucic acid, nitric acid, pamoic acid, pantothenic acid, phosphoric acid, succinic acid, sulfuric acid, oxalic acid, propionic acid, glycolic acid, hydroiodic acid, perchloric acid, cyclohexylsulfamic acid, salicylic acid, 2-naphthalenesulfonic acid, saccharinic acid, trifluoroacetic acid, tartaric acid and p-toluenesulfonic acid, etc. Citric acid, hydrobromic acid, formic acid, hydrochloric acid, maleic acid, phosphoric acid, sulfuric acid and tartaric acid are preferred. Formic acid and hydrochloric acid are more preferred.

Prodrugs of the compounds of the present application are included within the scope of protection of the present application. Generally, the prodrug refers to a functional derivative that can be easily converted into the desired compound in a body. Such derivatives include, for example, any pharmaceutically acceptable salt, ester, salt of an ester or other derivative of the compound of the present application that, after administration to a subject, can directly or indirectly provide the compound of the present application or a pharmaceutically active metabolite or residue thereof.

The compounds of the present application may contain one or more asymmetric centers and may thereby give rise to diastereomers and optical isomers. The present application includes all possible diastereomers and a racemic mixture thereof, an essentially pure separated enantiomer, all possible geometric isomers and pharmaceutically acceptable salts thereof.

When tautomers of the compound represented by formula (I) exist, unless otherwise specified, the present application includes any possible tautomers and pharmaceutically acceptable salts thereof, as well as mixtures thereof.

The term "pharmaceutical composition" refers to a mixture of one or more compounds of the present application or pharmaceutically acceptable salts thereof with pharmaceutically acceptable excipients. The purpose of a pharmaceutical composition is to facilitate administration of the compound of the present application to an organism.

In the present application, "a", "an", "the", "at least one" and "one or more" can be used interchangeably. Thus, for example, a mixture comprising "a" pharmaceutically acceptable excipient can be interpreted to mean that the pharmaceutical composition includes "one or more" pharmaceutically acceptable excipients.

The term "pharmaceutically acceptable excipient" refers to those excipients that have no obvious stimulating effect on an organism and do not impair the biological activity and performance of the active compound. Suitable excipients are well known to those skilled in the art, such as carbohydrates, waxes, water-soluble and/or water-swellable polymers, hydrophilic or hydrophobic materials, gelatin, oils, solvents, water, etc.

The pharmaceutical composition of the present application can be prepared by combining the compound of the present application with suitable pharmaceutically acceptable excipients, and can be formulated, for example, into a solid, semi-solid, liquid, or gaseous formulation, such as tablets, pills, capsules, powders, granules, ointments, emulsions, suspensions, suppositories, injections, inhalants, gels, microspheres and aerosols.

Typical routes of administration of the compound of the present application or a pharmaceutically acceptable salt thereof or a pharmaceutical composition thereof include, but are not limited to, oral, rectal, topical, inhalation, parenteral, sublingual, intravaginal, intranasal, intraocular, intraperitoneal, intramuscular, subcutaneous, and intravenous administration.

The term "treatment" generally refers to obtaining a desired pharmacological and/or physiological effect. The effect may be therapeutic in terms of partially or completely stabilizing or curing a disease and/or side effects resulting from the disease. "Treatment" as used herein covers any treatment of a disease in a patient, including: (a) inhibiting the symptoms of the disease, i.e., arresting its development; or (b) alleviating the symptoms of the disease, i.e., causing regression of the disease or symptoms.

The term "effective amount" means an amount of the compound of the present application for (i) treating or preventing a specific disease, condition, or disorder, (ii) alleviating, improving or eliminating one or more symptoms of a particular disease, condition, or disorder, or (iii) preventing or delaying the onset of one or more symptoms of a specific disease, condition, or disorder described herein. The amount of the compound of the present application that constitutes a "therapeutically effective amount" will vary depending on the compound, the disease state and its severity, the route of administration, and the age of the mammal to be treated, but can be routinely determined by one of ordinary skill in the art based on their own knowledge and the disclosure of the present application.

Room temperature as used in the present application refers to the indoor environment temperature, which is generally 18°C to 25°C.

The compounds of the present application have excellent performance in biological activities such as KRAS degradation ability, cell proliferation inhibitory activity and in vivo pharmacokinetic properties, and have high drug developability. Furthermore, research has found that the linking chain of the compound of the present application has a significant impact on the KRAS degradation ability and cell proliferation inhibitory activity of the compound. When 5- to 6-membered N-containing heteroaryl group (especially pyrazine ring or pyridine ring) is added to the linking chain, close to the VHL ligand side, the degradation ability of the compound for KRAS is enhanced, and the cell proliferation inhibitory activity of the compound is increased, achieving unexpected effects. We speculate that N-containing heteroaromatic ring occupies a special spatial position, and may have interactions such as hydrogen bonds with the VHL protein, increasing the binding force of the compound to VHL, thereby enhancing the degradation ability of the compound for the KRAS protein. Particularly, when a specific linking chain 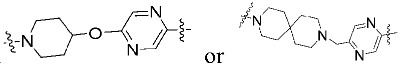 is used (wherein the pyrazine end is bonded to the VHL ligand), the resulting compound of the present application exhibits more excellent KRAS degradation ability and cell proliferation inhibitory activity.

### BRIEF DESCRIPTION OF THE DRAWINGS

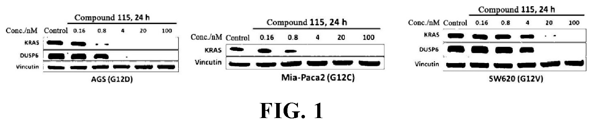

Figure 1 shows the results of western blotting experiments for KRAS protein degradation by compound 115 in different cell lines in Biological Experiment 2.

### DETAILED DESCRIPTION

In order to make the above content clearer and more definite, the present application will further illustrate the technical solution of the present application with the following examples. The following examples are only used to illustrate specific embodiments of the present application, so that those skilled in the art can understand the present application, but are not intended to limit the protection scope of the present application. In specific embodiments of the present application, techniques or methods not specifically described are conventional techniques or methods in the art.

Unless otherwise specified, all temperatures in the present application refer to degrees Celsius.

The following abbreviations are used in the examples:
DMF: N,N-dimethylformamide;
NIS: N-iodosuccinimide;
THF: tetrahydrofuran;
EA: ethyl acetate;
PE: petroleum ether;
DCM: dichloromethane;
MeOH: methanol;
DPPA: diphenylphosphoryl azide;
CDI: N,N'-carbonyldiimidazole;
DIEA/DIPEA: N,N-diisopropylethylamine;
POCl₃: phosphorus oxychloride;
DMSO: dimethyl sulfoxide;
HATU: 2-(7-azabenzotriazol-1-yl)-*N,N,N',N'*-tetramethyluronium hexafluorophosphate;
TFA: trifluoroacetic acid;
DABCO: triethylenediamine;
TBAF: tetrabutylammonium fluoride;
TEA: triethylamine;
TMSOI: trimethylsulfoxonium iodide;
ACN/MeCN: acetonitrile;
Dioxane: 1,4-dioxane;
PdCl₂(PPh₃)₂: bis(triphenylphosphine)palladium(II) dichloride;
Cs₂CO₃: cesium carbonate;
DMP: Dess-Martin periodinane;
CuI: cuprous iodide;
K₃PO₄: potassium phosphate;
ZnCl₂: zinc chloride;
CataCXium A Pd G3: methanesulfonato[di(1-adamantyl)-n-butylphosphine](2-amino-1,1'-biphenyl-2-yl)palladium(II).

### Synthesis of Intermediate M1:

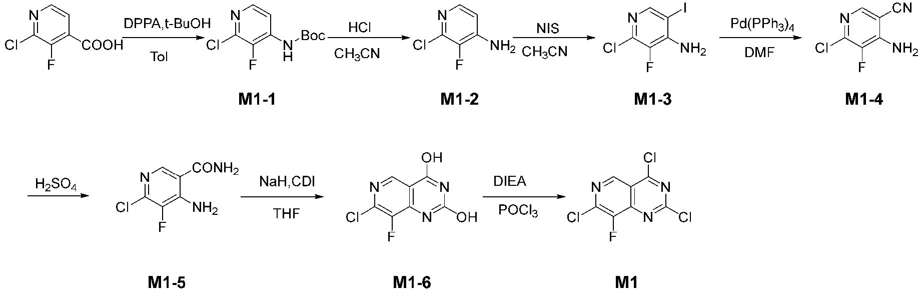

### Step 1: Synthesis of Compound M1-1

At room temperature, 2-chloro-3-fluoro-pyridine-4-carboxylic acid (54.00 g), toluene (390.00 mL), tert-butanol (390.00 mL), triethylamine (128.27 mL), and powdered 4Å molecular sieves (90.00 mL) (pre-activated) were sequentially added. The temperature was maintained at reflux under nitrogen protection for half an hour (internal temperature 87°C). After natural cooling to room temperature, DPPA (99.44 mL) was added. The mixture was heated to reflux, and reacted under maintained temperature for 5 hours. The reaction mixture was cooled to below 40°C, then diluted with 500 mL EA, and further cooled to room temperature. The mixture was filtered with diatomaceous earth to remove the added molecular sieves. The filter residue was rinsed several times with 1500 mL EA and suction-dried. The filtrate was collected and washed successively with 700 mL water and 700 mL saturated brine, and subjected to phase separation. The organic phase was dried with anhydrous sodium sulfate, filtered to remove the drying agent, and concentrated. The concentrate was separated and purified by column chromatography (PE/EA = 30:1 to 20:1). The eluent was concentrated to finally obtain Compound M1-1 (68.2 g, 89.88% yield).

ESI-MS m/z: 247.1 [M+H]⁺.

### Step 2: Synthesis of Compound M1-2

At room temperature, Compound M1-1 (65.00 g) was dissolved in CH₃CN (82.00 mL). The solution was cooled in a water bath, and hydrochloric acid (4M in dioxane) (264 mL) was slowly added. The mixture was reacted with stirring at room temperature for about 16 hours, during which a white solid precipitated in a suspended state. The reaction mixture was filtered, and the filter cake was rinsed with a small amount of acetonitrile, suction-dried, and the filtrate was discarded. The filter cake was collected and added to a mixture of 700 mL saturated sodium bicarbonate aqueous solution and 700 mL ethyl acetate. The mixture was alkalized, extracted, and subjected to phase separation. The aqueous phase was further extracted with 350 mL ethyl acetate and subjected to phase separation. The ethyl acetate phases were combined, washed with 300 mL saturated sodium chloride aqueous solution, and subjected to phase separation. The organic phase was dried with anhydrous sodium sulfate, filtered to remove the drying agent, and concentrated to obtain Compound M1-2 (36.3 g, 94.0% yield).

ESI-MS m/z: 147.1 [M+H]⁺.

### Step 3: Synthesis of Compound M1-3

At room temperature, Compound M1-2 (36.00 g) was dissolved in acetonitrile (180.00 mL). NIS (66.32 g) and p-toluenesulfonic acid (2.12 g) were added. Under nitrogen protection, the mixture was heated and maintained at 70°C for reaction. The reaction solution was cooled to 50°C, and 900 mL water was added. A pinkish-white solid powder was precipitated and was slurried for half an hour. The mixture was filtered, and the filter cake was rinsed with water and suction-dried. The filter cake was collected and dissolved completely in 1200 mL ethyl acetate. The solution was then washed twice with 350 mL saturated sodium sulfite aqueous solution, followed by washing with 350 mL saturated brine and subjected to phase separation. The organic phase was dried with anhydrous sodium sulfate, filtered, and concentrated to obtain Compound M1-3 (63.2 g, 94.43% yield).

ESI-MS m/z: 272.9 [M+H]⁺.

### Step 4: Synthesis of Compound M1-4

At room temperature, Compound M1-3 (57.50 g) was dissolved in DMF (22.00 mL). Zinc cyanide (32.22 g), tetrakis(triphenylphosphine)palladium (12.19 g) and powdered 4Å molecular sieves (20.00 mL) were added. Under a nitrogen atmosphere, the mixture was heated and maintained at 100°C for reaction about 7 hours. The oil bath was removed, and the mixture was naturally cooled to room temperature for work-up. The reaction mixture was filtered with diatomaceous earth, and suction-dried. The filtrate was collected and concentrated at 60°C to 70°C to obtain a crude product as a pale yellow solid. The filter residue was rinsed with 500 mL ethyl acetate and suction-dried. The rinsing solution was collected, combined with the crude product, and then concentrated until no liquid was distilled out. The obtained solid crude product was dissolved in 700 mL ethyl acetate, and washed three times with 250 mL saturated sodium chloride solution each time, and subjected to phase separation. The organic phase was dried with anhydrous sodium sulfate, filtered, and concentrated to obtain a pale yellow solid. 160 mL PE/EA=3/1 mixture was added. The mixture was slurried for half an hour, filtered, and suction-dried. The filter cake was collected, concentrated in a 45°C water bath, and then further evacuated under high vacuum with an oil pump to constant weight to obtain Compound M1-4 (36.1 g, 99.7% yield).

ESI-MS m/z: 172.0 [M+H]⁺.

### Step 5: Synthesis of Compound M1-5

At room temperature, concentrated sulfuric acid (61.37 mL) was added to a 500 mL single-neck flask. The mixture was cooled to below 10°C in an ice-water bath. Compound M1-4 (39.30 g) was added in batches. After the addition was complete, the mixture was stirred for 10 minutes under nitrogen atmosphere, maintained at 60°C in an oil bath, and reacted for about 1 hour. The reaction solution was cooled to room temperature, then carefully added to 1100 mL ice-water mixture for dilution and quenching. A small amount of yellow solid was precipitated. After stirring for 10 minutes, the mixture was filtered. The filter cake was collected, mixed with 50 mL saturated sodium bicarbonate aqueous solution, slurried for 20 minutes, and filtered again. The two filtrates were collected and combined. Then, solid sodium carbonate was slowly added to adjust the pH to about 7, causing an off-white solid powder to precipitate. The mixture was stirred for half an hour, filtered, and suction-dried. The filter cake was rinsed twice with 100 mL water, followed by suction-drying, each time. The filter cake was collected, placed in a vacuum oven, and dried at 55°C to constant weight to obtain Compound M1-5 (33.6 g, 77.37% yield).

ESI-MS m/z: 190.0 [M+H]⁺.

### Step 6: Synthesis of Compound M1-6

At room temperature, tetrahydrofuran (470.00 mL) was added. After nitrogen displacement, under a slight nitrogen flow, sodium hydride (10.00 g) was added. The mixture was heated in an oil bath, maintained at 40°C to 45°C, and stirred for 15 minutes. Then, Compound M1-5 (18.95 g) was added in batches. After the addition was complete, the mixture was stirred mechanically for 20 minutes at the same temperature. Then, CDI (24.31 g) was carefully added in batches. After the addition was complete, the mixture was stirred for 15 minutes, then heated in an oil bath and maintained under reflux for reaction. The reaction solution was cooled to below 10°C in an ice-water bath. Then 500 mL saturated ammonium chloride aqueous solution was added. A pale yellow solid was precipitated. 1000 mL water was added. The mixture was transferred to a 5 L beaker, and an additional 3000 mL water was added. The mixture was stirred for 1 hour, filtered, and suction-dried. The filter cake was collected, placed in a vacuum oven, and dried at 50°C to 55°C to constant weight to obtain Compound M1-6 (18.3 g, 84.93% yield).

ESI-MS m/z: 216.0 [M+H]⁺.

### Step 7: Synthesis of Compound M1

At room temperature, Compound M1-6 (18.00 g) and DIEA (36.00 mL) were dissolved in POCl₃ (180.00 mL). Under a nitrogen atmosphere, the mixture was heated and maintained at 100°C for about 2.5 hours. The mixture was concentrated under reduced pressure to remove phosphorus oxychloride, and the residue was co-evaporated twice with 100 mL DCM. The concentrated residue was dissolved in 400 mL DCM, and the solution was slowly added to 500 mL saturated sodium bicarbonate aqueous solution and cooled with an ice-water bath. After stirring for 15 minutes, phase separation was performed. The aqueous phase was further extracted with 300 mL dichloromethane and subjected to phase separation. The dichloromethane phases were combined, washed with 300 mL saturated sodium chloride aqueous solution, and subjected to phase separation. The organic phase was dried with anhydrous sodium sulfate, filtered, and concentrated. The concentrate was purified by silica gel column chromatography (PE/EA = 90/10 to 75/25) to obtain Compound M1 (10.95 g, 51.94% yield).

ESI-MS m/z: 251.9 [M+H]⁺.

### Synthesis of Intermediate M2

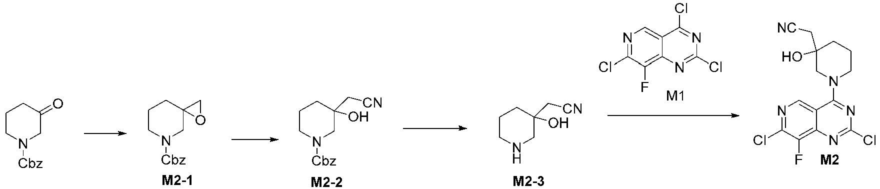

### Step 1: Synthesis of Compound M2-1

Compound TMSOI (8.5 g) was dissolved in DMSO (20 mL), and the mixture was cooled to 0°C. NaH (1.6 g) was added. After N₂ displacement three times, the mixture was reacted with stirring at room temperature for 1.0 h. A solution of benzyl 3-oxopiperidine-1-carboxylate (9.0 g) dissolved in DMSO (5 mL) was slowly added dropwise, and the reaction mixture was reacted with stirring at room temperature overnight. After the reaction was complete, the mixture was extracted with EA and H₂O, washed with saturated brine, dried with anhydrous sodium sulfate, filtered, dried by rotary evaporation, and purified by column chromatography to obtain Compound M2-1 (3.2 g). ESI-MS m/z: 248.3 [M+H]⁺.

### Step 2: Synthesis of Compound M2-2

Compound M2-1 (3.2 g), sodium cyanide (1.3 g), anhydrous ethanol (10 mL) and water (10 mL) were added. After N₂ displacement three times, the mixture reacted with stirring at room temperature for 5 h. After the reaction was complete, the mixture was extracted with EA and H₂O, washed with saturated brine, dried with anhydrous sodium sulfate, filtered, dried by rotary evaporation, and purified by column chromatography (PE:EA 43%) to obtain Compound M2-2 (3.0 g). ESI-MS m/z: 275.3 [M+H]⁺.

### Step 3: Synthesis of Compound M2-3

Compound M2-2 (1.5 g), palladium hydroxide (0.2 g) and anhydrous ethanol (10 mL) were added. After hydrogen displacement five times, the mixture was reacted with stirring at room temperature for 1.5 h. After the reaction was complete, the mixture was filtered, and the solvent was dried by rotary evaporation to obtain Compound M2-3 (0.9 g), which was used directly in the next step. ESI-MS m/z: 141.3 [M+H]⁺.

### Step 4: Synthesis of Compound M2

Compound M1 (1.6 g) and DIPEA (3.5 mL) were dissolved in DCM (15 mL), and the mixture was cooled to below -40°C and reacted with stirring for 10 min. Then, Compound M2-3 (0.9 g) was added, and the reaction mixture was reacted with stirring at this temperature for 1 h. After the reaction was complete, the mixture was quenched with saturated ammonium chloride solution, extracted with EA and H₂O, washed with saturated brine, dried with anhydrous sodium sulfate, filtered, dried by rotary evaporation, and purified by column chromatography (EA:DCM 12%) to obtain Compound M2 (1.2 g). ESI-MS m/z: 356.3 [M+H]⁺.

### Synthesis of Intermediate M3:

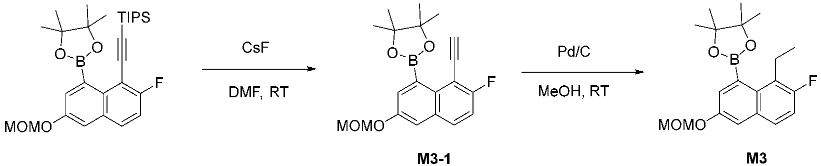

### Step 1: Synthesis of Compound M3-1

((2-Fluoro-6-(methoxymethoxy)-8-(4,4,5,5-tetramethyl-1,3,2-dioxaborolan-2-yl)naphthalen -1-yl)ethynyl)triisopropylsilane (1.0 g) was added to a 50 mL single-neck flask, followed by the addition of 6 mL DMF. Then, cesium fluoride (5.9 g) was added. The mixture was reacted with stirring at room temperature for 1 h. TLC monitoring indicated that the raw materials were completely reacted. The reaction solution was slowly added dropwise to water (20 mL). A white solid was precipitated. The mixture was filtered, and the filter cake was collected and dried to obtain 0.65 g of a white solid, which was Compound M3-1.

### Step 2: Synthesis of Compound M3

Compound M3-1 (0.65 g) was added to a 50 mL single-neck flask, followed by the addition of 6 mL of MeOH and Pd/C (600 mg, 10% purity). The mixture was reacted at room temperature under a hydrogen atmosphere for 20 min. After the reaction was complete, the mixture was filtered, and the filtrate was collected and concentrated. The concentrate was purified by column chromatography to obtain Compound M3 (496 mg, yield 75.5%).

¹H NMR (500 MHz, Methanol-d₄) δ 7.56 (dd, J = 8.9, 5.8 Hz, 1H), 7.39 (d, J = 2.7 Hz, 1H), 7.36 (d, J = 2.7 Hz, 1H), 7.20 (t, J = 9.2 Hz, 1H), 5.27 (s, 2H), 3.50 (s, 3H), 3.12 (qd, J = 7.5, 2.5 Hz, 2H), 1.44 (s, 12H), 1.26 (t, J = 7.5 Hz, 3H).

### Synthesis of Intermediate M4:

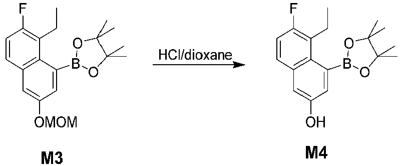

Compound M3 (1.0 g) was added to a 50 mL single-neck flask, followed by the addition of 10 mL hydrochloric acid (4 M in dioxane). After reacting at room temperature for half an hour, the reaction solution was neutralized by adding saturated sodium bicarbonate solution, extracted twice with EA. The organic phases were combined and dried with anhydrous sodium sulfate and concentrated. The concentrate was purified by column chromatography (DCM:ammonia in methanol = 10:1) to obtain Compound M4 (0.82 g). ¹H NMR (500 MHz, CDCl₃) δ 7.46 (dd, J = 8.9, 5.8 Hz, 1H), 7.27 (d, J = 2.7 Hz, 1H), 7.17 (dd, J = 12.5, 5.9 Hz, 1H), 7.12 (d, J = 2.7 Hz, 1H), 3.17 - 3.07 (m, 2H), 1.45 (s, 12H), 1.29 - 1.25 (m, 3H).

### Synthesis of Intermediate M5:

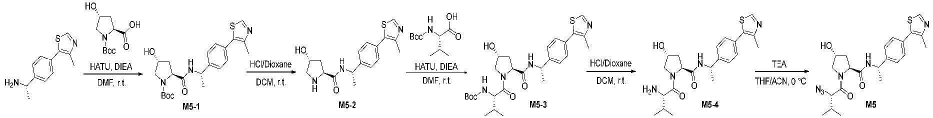

### Step 1: Synthesis of Compound M5-1

(*S*)-1-(4-(4-methylthiazol-5-yl)phenyl)ethanamine (12.7 g), Boc-*L*-hydroxyproline (13.5 g), and HATU (23.2 g) were dissolved in DMF (100 mL). DIEA (38.5 mL) was added, and the mixture was reacted at room temperature for 10 minutes. LCMS monitoring indicated the reaction was complete. EA (100 mL) and saturated brine (100 mL) were added to the reaction solution. The organic phase was collected, dried with anhydrous sodium sulfate, filtered, concentrated, and purified by column chromatography to obtain 22.0 g of a white solid, which was Compound M5-1.

ESI-MS m/z: 432 [M+H]⁺.

### Step 2: Synthesis of Compound M5-2

Compound M5-1 (22.0 g) was added to DCM (100 mL). A solution of hydrochloric acid in dioxane (100 mL) was added under an ice-water bath. The mixture was reacted at room temperature for 60 minutes. LCMS monitoring indicated the reaction was complete. The reaction solution was directly concentrated. 1 M NaOH solution was added until the pH of the reaction solution reached 8. DCM (100 mL) was added to the reaction solution. The organic phase was collected, washed once with saturated brine, dried with anhydrous sodium sulfate, filtered, and concentrated to obtain 16.8 g of a white solid, which was Compound M5-2.

ESI-MS m/z: 332 [M+H]⁺.

### Step 3: Synthesis of Compound M5-3

Compound M5-2 (19.0 g), Boc-L-valine (12.5 g) and HATU (22.9 g) were dissolved in DMF (100 mL). DIEA (28.4 mL) was added, and the mixture was reacted at room temperature for 10 minutes. LCMS monitoring indicated the reaction was complete. EA (100 mL) and saturated brine (100 mL) were added to the reaction solution. The organic phase was collected, dried with anhydrous sodium sulfate, filtered, concentrated, and purified by column chromatography to obtain 25.0 g of a white solid, which was Compound M5-3.

ESI-MS m/z: 531 [M+H]⁺.

### Step 4: Synthesis of Compound M5-4

Compound M5-3 (25.0 g) was added to DCM (100 mL). A solution of hydrochloric acid in dioxane (100 mL) was added under an ice-water bath. The mixture was reacted at room temperature for 60 minutes. LCMS monitoring indicated the reaction was complete. The reaction solution was directly concentrated. 1 M NaOH solution was added until the pH of the reaction solution reached 8. DCM (100 mL) was added to the reaction solution. The organic phase was collected, washed once with saturated brine, dried with anhydrous sodium sulfate, filtered, and concentrated to obtain 17.0 g of a white solid, which was Compound M5-4.

ESI-MS m/z: 431 [M+H]⁺.

### Step 5: Synthesis of Compound M5

Compound M5-4 (17.0 g) and TEA (32.9 mL) were dissolved in ACN (100 mL) and THF (130 mL). Under an ice-water bath, 2-azido-1,3-dimethylimidazolinium hexafluorophosphate (22.5 g) was dissolved in ACN (30 mL) and slowly added to the system. The mixture was maintained at this temperature and reacted for 3 hours. LCMS monitoring indicated the reaction was complete. The reaction solution was directly concentrated. DCM (100 mL) was added to the reaction solution. The organic phase was collected, washed once with saturated brine, dried with anhydrous sodium sulfate, filtered, concentrated, and purified by column chromatography to obtain 15.3 g of a pale yellow powder, which was Compound M5.

ESI-MS m/z: 457 [M+H]⁺.

¹H NMR (500 MHz, Methanol-d₄) δ 8.87 (s, 1H), 7.46-7.40 (m, 4H), 7.94 (q, J = 7.0 Hz, 1H), 4.64-4.60 (m, 1H), 4.45-4.44 (m, 1H), 3.75 (d, J = 8.0 Hz, 1H), 3.71-3.64 (m, 2H), 2.48 (s, 3H), 2.26-2.15 (m, 2H), 1.96-1.91 (m, 1H), 1.52 (d, J = 7.0 Hz, 3H), 1.09-0.98 (m, 6H).

### Synthesis of Intermediate M6

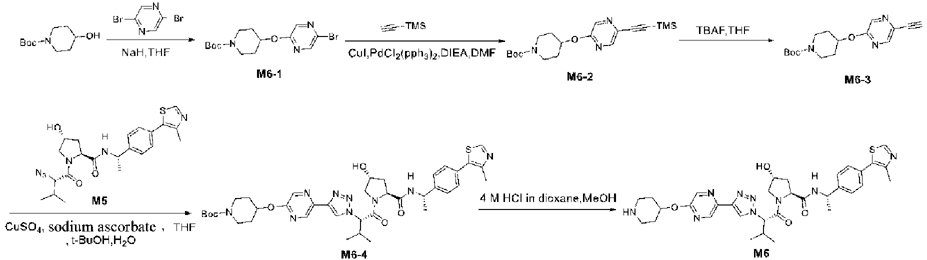

### Step 1: Synthesis of Compound M6-1

Compound N-Boc-4-hydroxypiperidine (1.0 g) was added to a 25 mL single-neck flask and dissolved in THF (10 mL). NaH (300.0 mg) and 2,5-dibromopyrazine (1.18 g) were added. The mixture was heated to 50°C and reacted for 5 h. The reaction solution was directly evaporated to dryness. Purification by column chromatography (PE:EA = 6:1) obtained 1.38 g of a pale yellow solid, which was Compound M6-1.

### Step 2: Synthesis of Compound M6-2

Compound M6-1 (690.0 mg) was added to a 25 mL single-neck flask and dissolved in DMF (10 mL). Trimethylsilylacetylene (283.7 mg), CuI (73.3 mg), PdCl₂(PPh₃)₂ (135.2 mg) and DIEA (0.95 mL) were added. After nitrogen protection, the mixture was heated to 50°C and reacted for 3 h. Saturated brine (5 mL) was added to the reaction solution, followed by extraction with EA. The organic phase was collected, dried with anhydrous sodium sulfate, and the product was separated by column chromatography (PE:EA = 6:1) to obtain 522.2 mg of a pale yellow solid, which was Compound M6-2.

### Step 3: Synthesis of Compound M6-3

Compound M6-2 (522.2 mg) was added to a 25 mL single-neck flask and dissolved in THF (10 mL). 1 M TBAF/THF solution (2.2 mL) was added, and the mixture was reacted at room temperature for 2 h. The reaction solution was directly evaporated to dryness. Purification by column chromatography (PE:EA = 4:1) obtained 378.2 mg of a pale yellow solid, which was Compound M6-3.

### Step 4: Synthesis of Compound M6-4

Compound M5 (100.0 mg) was added to a 25 mL single-neck flask and dissolved in THF (2 mL). Compound M6-3 (80.2 mg), copper sulfate (35.2 mg), water (2 mL), tert-butanol (2 mL) and sodium ascorbate (160.0 mg) were added. The mixture was reacted at room temperature for 0.5 h. The reaction solution was directly evaporated to dryness. Purification by column chromatography (DCM:MeOH = 12:1) obtained 132.3 mg of a pale yellow solid, which was Compound M6-4.

### Step 5: Synthesis of Compound M6

Compound M6-4 (132.3 mg) was added to a 25 mL single-neck flask and dissolved in HCl/dioxane (5 mL). Methanol (1 mL) was added, and the mixture was reacted at room temperature for 1 h. The reaction solution was directly evaporated to dryness. Saturated sodium bicarbonate solution (5 mL) was added to the reaction solution to adjust the pH to 7-8, followed by extraction with dichloromethane and isopropanol. The organic phase was dried with anhydrous sodium sulfate to obtain 92.2 mg of a yellow solid, which was Compound M6.

### Synthesis of Intermediate M7

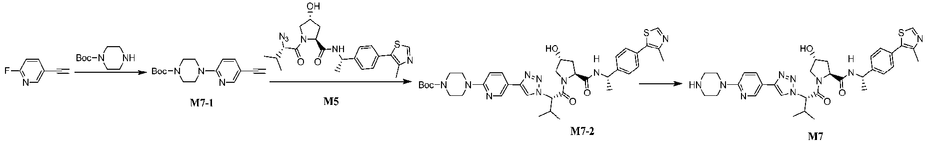

### Step 1: Synthesis of Compound M7-1

5-ethynyl-2-fluoropyridine (500 mg), tert-butyl piperazine-1-carboxylate (922 mg), acetone (5 mL) and DIEA (1.6 g) were added to a 50 mL flask. The mixture was heated to 60°C for 5 h, then the solvent was removed. The residue was dissolved in EA, washed with water, washed with brine, dried with sodium sulfate, and filtered. The filtrate was subjected to solvent removal under reduced pressure to obtain a crude product, which was purified by column chromatography to obtain Compound M7-1 (1.02 g).

### Step 2: Synthesis of Compound M7-2

Compound M7-1 (200 mg), M5 (250 mg), sodium ascorbate (226 mg), copper sulfate (108 mg), tert-butanol (3 mL), THF (3 mL) and water (3 mL) were added to a 50 mL flask. The mixture was reacted at room temperature for 1 hour. The reaction was complete as detected. The mixture was extracted with EA. The organic phase was washed with water, washed with brine, dried with sodium sulfate, and filtered. The filtrate was concentrated under reduced pressure to obtain a crude product, which was purified by column chromatography to obtain Compound M7-2 (198 mg).

### Step 3: Synthesis of Compound M7

Compound M7-2 (198 mg) and 4N HCl/dioxane (5 mL) were added to a 50 mL flask. The mixture was reacted at room temperature for 1 hour, and then subjected to solvent removal under reduced pressure to obtain crude Compound M7 (200 mg).

### Synthesis of Intermediate M8:

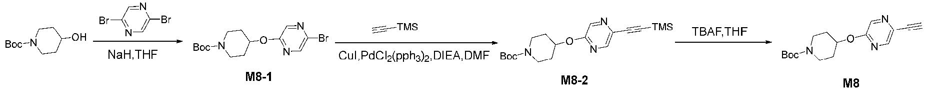

### Step 1: Synthesis of Compound M8-1

Compound N-Boc-4-hydroxypiperidine (1.0 g) was added to a 25 mL single-neck flask and dissolved in THF (10 mL). NaH (300.0 mg) and 2,5-dibromopyrazine (1.18 g) were added. The mixture was heated to 50°C and reacted for 5 h. The reaction solution was directly evaporated to dryness. Purification by column chromatography (PE:EA = 6:1) obtained 1.38 g of a pale yellow solid, which was Compound M8-1.

### Step 2: Synthesis of Compound M8-2

Compound M8-1 (690.0 mg) was added to a 25 mL single-neck flask and dissolved in DMF (10 mL). Trimethylsilylacetylene (283.7 mg), CuI (73.3 mg), PdCl₂(PPh₃)₂ (135.2 mg) and DIPEA (0.95 mL) were added. After nitrogen protection, the mixture was heated to 50°C and reacted for 3 h. Saturated brine (5 mL) was added to the reaction solution, followed by extraction with EA. The organic phase was collected, dried with anhydrous sodium sulfate, and the product was separated by column chromatography (PE:EA = 6:1) to obtain 522.2 mg of a pale yellow solid, which was Compound M8-2.

### Step 3: Synthesis of Compound M8

Compound M8-2 (522.2 mg) was added to a 25 mL single-neck flask and dissolved in THF (10 mL). 1 M TBAF in tetrahydrofuran solution (2.2 mL) was added, and the mixture was reacted at room temperature for 2 h. The reaction solution was directly evaporated to dryness. Purification by column chromatography (PE:EA = 4:1) obtained 378.2 mg of a pale yellow solid, which was Compound M8.

### Synthesis of Intermediate M9

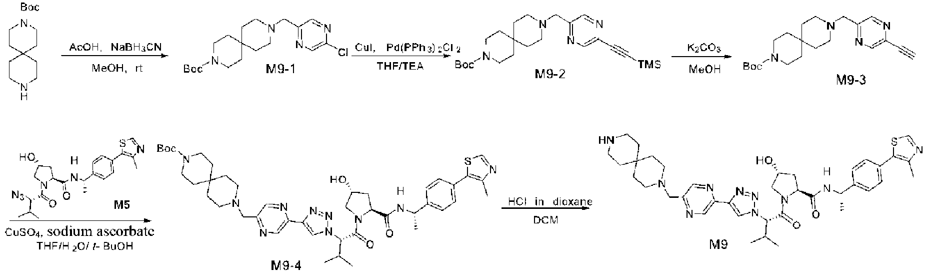

### Step 1: Synthesis of Compound M9-1

5-Chloropyrazine-2-carbaldehyde (336.23 mg), tert-butyl 3,9-diazaspiro[5.5]undecane-3-carboxylate (500.00 mg), methanol (8.00 mL) and acetic acid (0.02 mL) were added to a reaction flask. The mixture was stirred at room temperature for 1 h, then cooled to 0°C. Sodium borohydride (148.72 mg) was added, and the mixture was reacted at room temperature for 0.5 h. The mixture was diluted with water and then extracted with DCM. The organic phase was dried and concentrated. Purification by column chromatography (PE:EA = 2:1) obtained 250 mg of a yellow oil, which was Compound M9-1.

### Step 2: Synthesis of Compound M9-2

Compound M9-1 (240.00 mg), trimethylsilylacetylene (0.18 mL), bis(triphenylphosphine)palladium(II) dichloride (44.22 mg), cuprous iodide (12.0 mg), triethylamine (0.53 mL) and tetrahydrofuran (5.00 mL) were added to a reaction flask. After nitrogen displacement, the mixture was reacted at 50°C for 2 h under nitrogen protection. The reaction solution was filtered, and the filter cake was rinsed three times with EA. The mother liquor was concentrated, and the residue was purified by column chromatography (PE:EA = 1:1) to obtain 220 mg of a brown liquid, which was Compound M9-2.

### Step 3: Synthesis of Compound M9-3

Compound M9-2 (220.00 mg), methanol (4.00 mL), and potassium carbonate (137.38 mg) were added to a reaction flask. The mixture was reacted at room temperature for 0.2 h, then filtered. The filter cake was washed several times with EA. The mother liquor was concentrated, and the residue was purified by column chromatography (DCM:MeOH = 10:1) to obtain 50 mg of a brown liquid, which was Compound M9-3.

### Step 4: Synthesis of Compound M9-4

Compound M9-3 (50.00 mg), M5 (61.6 mg), copper sulfate (19.39 mg), sodium ascorbate (69.54 mg), tetrahydrofuran (1.00 mL), tert-butanol (1.00 mL) and water (1.00 mL) were added to a reaction flask. The mixture was reacted at room temperature for 0.5 h, then diluted with water and extracted with DCM. The organic phase was dried, concentrated, and purified by column chromatography (DCM:ammonia in methanol = 14:1) to obtain 65 mg of a yellow solid, which was Compound M9-4.

### Step 5: Synthesis of Compound M9

Compound M9-4 (65.00 mg) and dichloromethane (2.00 mL) were added to a reaction flask. Under stirring, 4M HCl in dioxane solution (0.40 mL) was added, and the mixture was reacted at room temperature for 0.2 h. The pH was then adjusted to 8 with sodium bicarbonate aqueous solution, and the mixture was extracted with DCM. The organic phase was dried and concentrated to obtain 45 mg of a yellow solid, which was Compound M9.

### Synthesis of Intermediate M10:

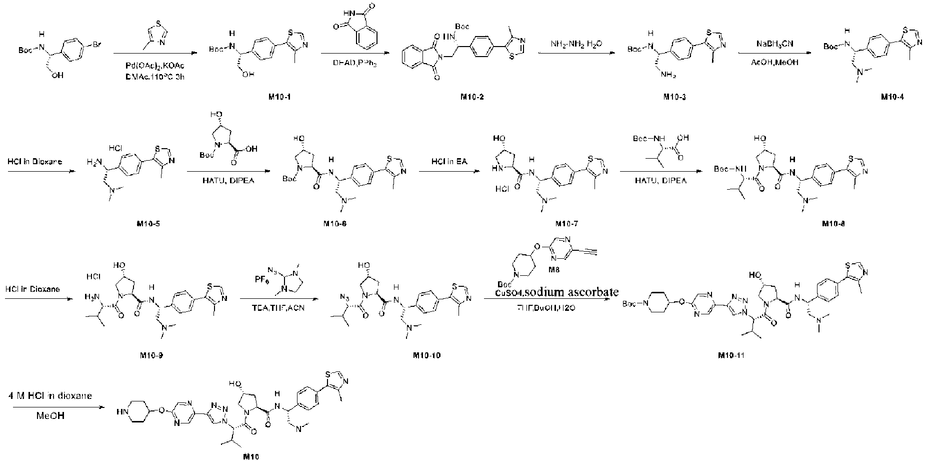

### Step 1: Synthesis of Compound M10-1

Compound (*R*)-tert-butyl (1-(4-bromophenyl)-2-hydroxyethyl)carbamate (1.0 g), 4-methylthiazole (0.63 g), palladium acetate (0.07 g) and potassium carbonate (0.62 g) were dissolved in 10.0 mL of DMAc. The mixture was reacted at 120°C for 2.0 h. The reaction solution was cooled to room temperature, then quenched by dropwise addition of water. The mixture was extracted three times with ethyl acetate. The organic phases were combined, dried with anhydrous sodium sulfate, and subjected to solvent removal under reduced pressure. Purification by column chromatography obtained 1.05 g of the product, which was Compound M10-1.

### Step 2: Synthesis of Compound M10-2

Compound M10-1 (0.9 g), phthalimide (0.395 g), and triphenylphosphine (0.74 g) were dissolved in 10.0 mL of THF. After N₂ displacement 2 to 3 times, diethyl azodicarboxylate (0.468 g) was added under an ice bath. The mixture was reacted at room temperature for 1 h. The reaction solution was concentrated under reduced pressure to obtain 1.10 g of the product, which was Compound M10-2. The crude product was used directly in the next step.

### Step 3: Synthesis of Compound M10-3

Compound M10-2 (1.1 g) was dissolved in 10.0 mL of methanol. Hydrazine hydrate (0.74 mL) was added, and the mixture was reacted at 90°C for 1.0 h. The reaction solution was filtered to remove insoluble impurities. The filtrate was concentrated under reduced pressure to obtain 0.78 g of crude product, which was used directly in the next step. This crude product was Compound M10-3.

### Step 4: Synthesis of Compound M10-4

Compound M10-3 (0.78 g) was added to a 25 mL single-neck flask and dissolved in anhydrous methanol (10 mL). Formaldehyde aqueous solution (1.9 g, 37%) and acetic acid (0.13 mL) were added. The mixture was reacted at room temperature for 30 min, then sodium cyanoborohydride (0.44 g) was added. Saturated brine (5 mL) was added to the reaction solution, followed by extraction with EA. The organic phase was collected, dried with anhydrous sodium sulfate, and the product was separated by column chromatography (PE:EA = 6:1) to obtain 0.80 g of a pale yellow solid, which was Compound M10-4.

### Step 5: Synthesis of Compound M10-5

Compound M10-4 (0.8 g) was dissolved in 4M HCl in dioxane solution (10.00 mL). The mixture was reacted at room temperature for 1.0 h. The reaction solution was concentrated under reduced pressure. The residue was re-dissolved in methanol and concentrated again under reduced pressure to obtain 0.8 g of a yellow product, which was used directly in the next step. This yellow product was Compound M10-5.

### Step 6: Synthesis of Compound M10-6

Compound M10-5 (0.8 g), (2S,4R)-1-tert-butoxycarbonyl-2-carbamoyl-4-hydroxypyrrolidine (0.5 g) and DIPEA (2.3 mL) were dissolved in 10.00 mL of DCM. HATU (0.9 g) was added, and the mixture was reacted at room temperature for 1.0 h. Silica gel was added to the reaction solution for sand making. Purification by column chromatography obtained 1.0 g of the product, which was Compound M10-6.

### Step 7: Synthesis of Compound M10-7

Compound M10-6 (1.0 g) was dissolved in 4M HCl in dioxane solution (10.00 mL). The mixture was reacted at room temperature for 1.0 h. The reaction solution was evaporated for solvent removal under reduced pressure. The residue was re-dissolved in methanol and evaporated for solvent removal again under reduced pressure to obtain 0.76 g of a yellow product, which was used directly in the next step. This yellow product was Compound M10-7.

### Step 8: Synthesis of Compound M10-8

Compound M10-7 (1.1 g), *N*-tert-butoxycarbonyl-L-valine (0.4 g) and DIPEA (3.22 mL) were dissolved in 15.00 mL of DMF. HATU (0.7 g) was added, and the mixture was reacted at room temperature for 1.0 h. The reaction solution was quenched with water and extracted three times with ethyl acetate. The organic phases were combined, dried with anhydrous sodium sulfate, and evaporated for solvent removal under reduced pressure. Purification by column chromatography obtained 0.86 g of the product, which was Compound M10-8.

### Step 9: Synthesis of Compound M10-9

Compound M10-8 (0.86 g) was dissolved in 4M HCl in dioxane solution (10.00 mL). The mixture was reacted at room temperature for 1.0 h. The reaction solution was evaporated for solvent removal under reduced pressure. The residue was re-dissolved in methanol and evaporated for solvent removal again under reduced pressure to obtain 0.70 g of a yellow product, which was used directly in the next step. This yellow product was Compound M10-9.

### Step 10: Synthesis of Compound M10-10

Compound M10-9 (0.70 g) was dissolved in a mixed solvent of anhydrous acetonitrile (10.00 mL) and tetrahydrofuran (10.0 mL). Triethylamine (1.91 mL) was added, followed by 2-azido-1,3-dimethylimidazolinium hexafluorophosphate (0.78 g) under an ice bath. The mixture was reacted at room temperature for 2.0 h. The reaction solution was quenched with water and extracted three times with ethyl acetate. The organic phases were combined, dried with anhydrous sodium sulfate, and evaporated for solvent removal under reduced pressure. Purification by column chromatography obtained 0.35 g of the product, which was Compound M10-10.

### Step 11: Synthesis of Compound M10-11

At room temperature, M10-10 (150 mg), M8 (150 mg), sodium ascorbate (155 mg) and anhydrous copper sulfate (43 mg) were dissolved in a mixed solvent of 2.00 mL tert-butanol, 2.00 mL tetrahydrofuran and 2.00 mL water. The mixture was reacted at room temperature for 1 h. The reaction solution was quenched by adding 50 mL of water, extracted three times with a mixed solvent of DCM/MeOH = 10/1. The organic phases were combined, dried with anhydrous sodium sulfate, and evaporated for solvent removal under reduced pressure. Purification by column chromatography (DCM/MeOH = 10/1) obtained 208 mg of a yellow solid product, which was Compound M10-11.

### Step 12: Synthesis of Compound M10

Compound M10-11 (208 mg) was added to a 25 mL single-neck flask and dissolved in 4M HCl in dioxane solution (5 mL). Methanol (1 mL) was added, and the mixture was reacted at room temperature for 1 h. The reaction solution was directly evaporated to dryness. Saturated sodium bicarbonate solution (5 mL) was added to the reaction solution to adjust the pH to 7-8, followed by extraction with dichloromethane/isopropanol. The organic phase was dried with anhydrous sodium sulfate to obtain 133 mg of a yellow solid, which was Compound M10.

### Examples

### Example 5: Synthesis of Compound (2S,4R)-1-((S)-2-(4-(5-((1-((1-(((7-(8-ethyl-7-fluoro-3-hydroxynaphthalen-1-yl)-8-fluoro-4-(1,4-oxazepan-4-yl)pyrido[4,3-d]pyrimidin-2-yl)oxy)methyl)cyclopropyl)methyl)piperidin-4-yl)oxy) pyrazin-2-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

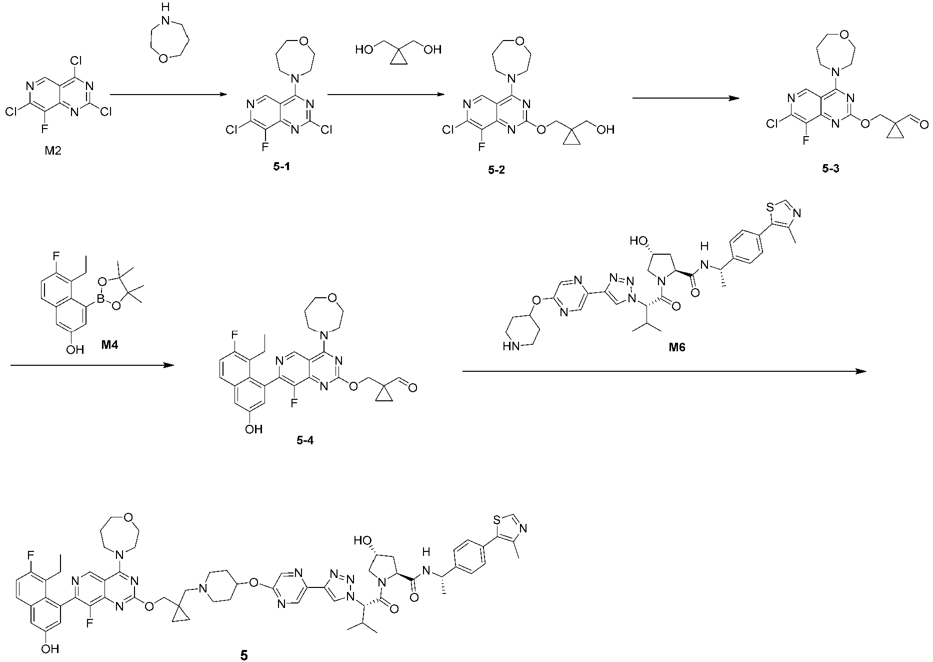

### Step 1: Synthesis of Compound 5-1

Compound M2 and 2,4,7-trichloro-8-fluoropyrido[4,3-d]pyrimidine (500.0 mg) were dissolved in dichloromethane (20 mL). Under nitrogen protection, the mixture was cooled to -60°C. DIPEA (690 µL) was added, followed by the slow addition of a solution of homomorpholine (200.0 mg) in dichloromethane. The temperature was maintained, and the mixture was reacted for 0.5 h. LCMS monitoring indicated the reaction was complete. Saturated ammonium chloride (5 mL) was added to the reaction solution to quench the reaction, followed by extraction with EA. The organic phase was washed once with saturated brine, dried with anhydrous sodium sulfate, filtered, concentrated, and purified by column chromatography to obtain 610.0 mg of a white solid, which was Compound 5-1. ESI-MS m/z: 317.1 [M+H]⁺.

### Step 2: Synthesis of Compound 5-2

Compound 5-1 (610 mg) was dissolved in DMF (20 mL). Compound 1,1-cyclopropanedimethanol (393 mg), Cs₂CO₃ (1.88 g) and DABCO (43.0 mg) were slowly added. The mixture was reacted at room temperature for 2 h. LCMS monitoring indicated the reaction was complete. EA was added to the reaction solution for extraction, and the organic phase was washed once with saturated brine, dried with anhydrous sodium sulfate, filtered, concentrated, and purified by column chromatography to obtain 757 mg of a white solid, which was Compound 5-2. ESI-MS m/z: 383.2 [M+H]⁺.

### Step 3: Synthesis of Compound 5-3

Compound 5-2 (757 mg) was dissolved in DCM (20 mL). Under ice bath conditions, DMP reagent (1.26 g) was slowly added. The mixture was slowly warmed to room temperature and reacted for 1 h. LCMS monitoring indicated the reaction was complete. DCM was added to the reaction solution for extraction, and the organic phase was washed once with saturated brine, dried with anhydrous sodium sulfate, filtered, concentrated, and purified by column chromatography to obtain 638 mg of a white solid, which was Compound 5-3. ESI-MS m/z: 381.1 [M+H]⁺.

### Step 4: Synthesis of Compound 5-4

Compound 5-3 (638 mg), Compound M4 (1.06 g), CataCXium A Pd G3 (244 mg) and K₃PO₄ (1.07 g) were weighed and added to 1,4-dioxane (20 mL) and water (5 mL). Under nitrogen protection, the mixture was reacted at 90°C for 2 h. LCMS monitoring indicated the reaction was complete. EA was added to the reaction solution for extraction, and the organic phase was washed once with saturated brine, dried with anhydrous sodium sulfate, filtered, concentrated, and purified by column chromatography to obtain 460 mg of a white solid, which was Compound 5-4. ESI-MS m/z: 535.3 [M+H]⁺.

### Step 5: Synthesis of Compound 5

Compound 5-4 (120.0 mg) and Compound M6 (148 mg) were dissolved in methanol (10 mL). Zinc chloride solution (0.45 mL, 1.0 M in THF) was added. The mixture was reacted at 40°C for 1 hour, then sodium cyanoborohydride (42.3 mg) was added. The mixture was reacted at 60°C for 12 hours. LCMS monitoring indicated the reaction was complete. Saturated sodium bicarbonate solution (5.0 mL) was added to quench the reaction. DCM (20 mL) was added to the reaction solution. The organic phase was collected, concentrated, and separated by preparative liquid chromatography to obtain 88.0 mg of Compound 5. ESI-MS m/z: 590.3 1/2[M+2H]⁺.

¹H NMR (500 MHz, DMSO-d₆) δ 9.90 (s, 1H), 9.17 (s, 1H), 8.99 (s, 1H), 8.76 (d, J = 1.3 Hz, 1H), 8.57 (s, 1H), 8.52 (d, J = 7.9 Hz, 1H), 8.29 (d, J = 1.3 Hz, 1H), 7.77 (dd, J = 9.1, 6.0 Hz, 1H), 7.48 - 7.42 (m, 2H), 7.39 - 7.32 (m, 4H), 7.03 (d, J = 2.5 Hz, 1H), 5.40 (d, J = 10.2 Hz, 1H), 5.18 (d, J = 3.8 Hz, 1H), 5.00 (d, J = 16.3 Hz, 1H), 4.93 (p, J = 7.0 Hz, 1H), 4.44 - 4.30 (m, 4H), 4.13 (t, J = 15.4 Hz, 4H), 3.95 (t, J = 4.5 Hz, 2H), 3.77 - 3.73 (m, 3H), 2.80 (s, 2H), 2.46 (s, 2H), 2.36 (s, 3H), 2.25 (s, 2H), 2.12 - 2.05 (m, 3H), 2.02 - 1.94 (m, 3H), 1.79 (ddd, J = 12.8, 8.5, 4.5 Hz, 1H), 1.67 (d, J = 9.2 Hz, 2H), 1.39 (d, J = 7.0 Hz, 3H), 1.08 (d, J = 6.4 Hz, 3H), 0.72 (dt, J = 32.8, 18.5 Hz, 11H), 0.44 (s, 2H).

### Example 7: Synthesis of Compound (2S,4R)-1-((S)-2-(4-(5-((1-((1-(((4-(1,1-dioxido-1,3-thiazinan-3-yl)-7-(8-ethyl-7-fluoro-3-hydrox ynaphthalen-1-yl)-8-fluoropyrido[4,3-d]pyrimidin-2-yl)oxy)methyl)cyclopropyl)methyl)piperidi n-4-yl)oxy)pyrazin-2-yl)-1H-1,2,3-triazol-1-yl)-3-methylbutanoyl)-4-hydroxy-N-((S)-1-(4-(4-me thylthiazol-5-yl)phenyl)ethyl)pyrrolidine-2-carboxamide

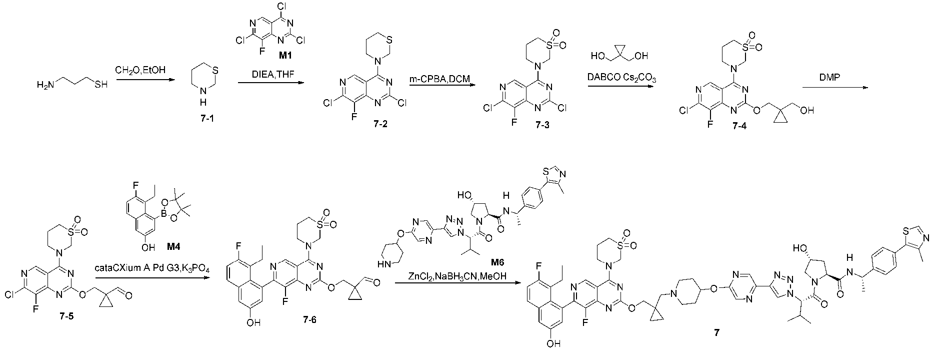

### Step 1: Synthesis of Compound 7-1

3-Aminopropanethiol hydrochloride (500 mg) was dissolved in 5 mL of ethanol, followed by the addition of formaldehyde aqueous solution (319 mg). The mixture was stirred at 20°C for 12 hours. LCMS monitoring indicated the reaction was complete. The mixture was concentrated under reduced pressure to obtain crude Compound 7-1 (520 mg). ESI-MS m/z: 104.1 [M+H]⁺.

### Step 2: Synthesis of Compound 7-2

Compound 7-1 (240 mg) was added to a 25 mL single-neck flask, followed by the addition of 4 mL of tetrahydrofuran. Then, M1 (450 mg) was added. 1.18 mL N,N-diisopropylethylamine was added dropwise to the reaction solution at -20°C. The mixture was stirred at 0°C for 2 hours. LCMS monitoring indicated the reaction was complete. The mixture was concentrated under reduced pressure and purified by column chromatography to obtain Compound 7-2 (441 mg). ESI-MS m/z: 319.1 [M+H]⁺.

### Step 3: Synthesis of Compound 7-3

Compound 7-2 (441 mg) was added to a 25 mL single-neck flask, followed by the addition of 6 mL of dichloromethane. Then, m-chloroperoxybenzoic acid (701 mg) was added, and the mixture was stirred at 20°C for 0.5 hours. LCMS monitoring indicated the reaction was complete. The mixture was concentrated under reduced pressure and purified by column chromatography to obtain Compound 7-3 (374 mg). ESI-MS m/z: 351.1 [M+H]⁺.

### Step 4: Synthesis of Compound 7-4

Compound 7-3 (374 mg) was added to a 50 mL single-neck flask, followed by the addition of 5 mL of *N,N-*dimethylformamide, 1,1-cyclopropanedimethanol (218 mg), triethylenediamine (24 mg) and cesium carbonate (694 mg). The mixture was stirred at 20°C for 12 hours. LCMS monitoring indicated the reaction was complete. The reaction solution was diluted with water, then extracted twice with ethyl acetate. The organic phases were collected and combined, dried with anhydrous sodium sulfate, and purified by column chromatography to obtain Compound 7-4 (70 mg). ESI-MS m/z: 417.1 [M+H]⁺.

### Step 5: Synthesis of Compound 7-5

Compound 7-4 (70 mg) was added to a 10 mL single-neck flask, followed by the addition of 2 mL of dichloromethane and Dess-Martin periodinane (142 mg). The mixture was stirred at 20°C for 12 hours. LCMS monitoring indicated the reaction was complete. The mixture was concentrated under reduced pressure and purified by column chromatography to obtain Compound 7-5 (36 mg). ESI-MS m/z: 415.1 [M+H]⁺.

### Step 6: Synthesis of Compound 7-6

Compound 7-5 (10 mg) and M4 (15 mg) were added to a 10 mL single-neck flask, followed by the addition of 1 mL 1,4-dioxane, 0.2 mL water, methanesulfonato[di(1-adamantyl)-n-butylphosphine](2-amino-1,1'-biphenyl-2-yl)palladium(II) (4 mg) and potassium phosphate (15 mg). After N₂ displacement three times, the mixture was stirred at 90°C for 10 hours under a N₂ atmosphere. LCMS monitoring indicated the reaction was complete. The mixture was concentrated under reduced pressure and purified by column chromatography to obtain Compound 7-6 (5 mg). ESI-MS m/z: 569.2 [M+H]⁺.

### Step 7: Synthesis of Compound 7

M6 (9 mg) was added to a 10 mL single-neck flask, followed by the addition of 0.5 mL methanol, Compound 7-6 (5 mg) and 0.01 mL zinc chloride (1 mol/L THF). The mixture was stirred at 40°C for 2 hours. Sodium cyanoborohydride (3 mg) was added, and the mixture was stirred at 50°C for 12 hours. LCMS monitoring indicated the reaction was complete. The reaction was quenched with saturated ammonium chloride solution, and then neutralized with saturated sodium bicarbonate solution. The mixture was extracted twice with dichloromethane/methanol (5:1). The organic phases were collected and combined, dried with anhydrous sodium sulfate, concentrated under reduced pressure, and purified by pre-HPLC to obtain Compound 7 (1.7 mg). ESI-MS m/z: 607.07 1/2[M+2H]⁺.

### Example 57: Synthesis of Compound (2S,4R)-1-((S)-2-(4-(5-((1-((1-(((4-((S)-3-(cyanomethyl)-3-hydroxypiperidin-1-yl)-7-(8-ethyl-7-f luoro-3-hydroxynaphthalen-1-yl)-8-fluoropyrido[4,3-d]pyrimidin-2-yl)oxy)methyl)cyclopropyl) methyl)piperidin-4-yl)oxy)pyrazin-2-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

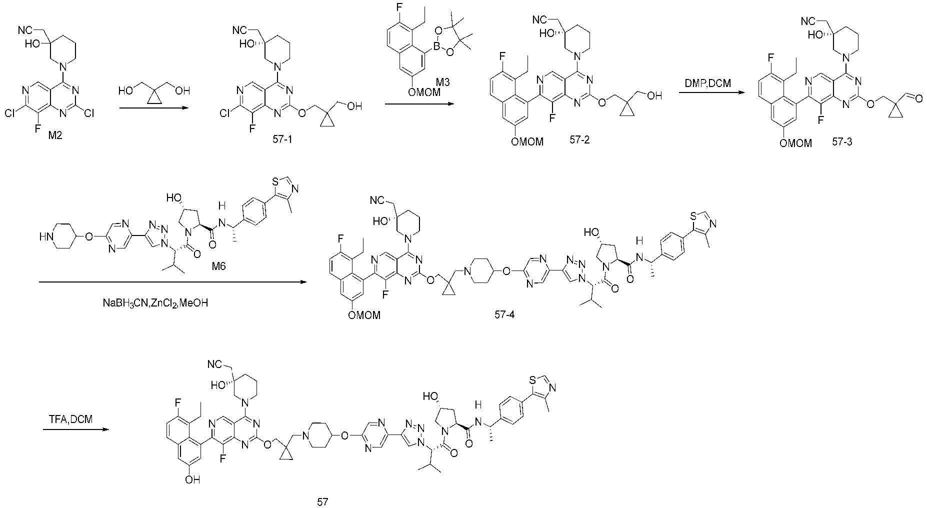

### Step 1: Synthesis of Compound 57-1

Compound M2 (1.2 g), 1,1-cyclopropanedimethanol (0.7 g), cesium carbonate (2.2 g) and DABCO (0.1 g) were dissolved in DMF (10 mL). The mixture was stirred at room temperature overnight. After the reaction was complete, the mixture was diluted with water, extracted twice with EA, washed with saturated brine, dried with anhydrous sodium sulfate, filtered, dried by rotary evaporation, and purified by column chromatography (PE:EA = 100:67) to obtain the racemate (0.8 g). ESI-MS m/z: 422.1 [M+H]⁺. A chiral Compound 57-1 was obtained by liquid chromatography separation under the following conditions: Column: CHIRAL ART IA (20.0 mm × 250 mm, 5 µm); Column temperature: 35°C; Wavelength: 254 nm; Flow rate: 25 mL/min; Retention time: 4.217 min; Mobile phase: 0.1% diethylamine in n-hexane: 0.1% diethylamine in ethanol (40:60).

### Step 2: Synthesis of Compound 57-2

Compound 57-1 (130.0 mg), Compound M4 (2-(8-ethyl-7-fluoro-3-(methoxymethoxy)naphthalen-1-yl)-4,4,5,5-tetramethyl-1,3,2-dioxaborola ne) (229.7 mg), CataCXium A Pd G3 (46.5 mg), K₃PO₄ (203.1 mg), 1,4-dioxane (4 mL) and H₂O (1 mL) were added. The temperature was raised to 90°C, and the mixture was reacted with stirring for 1 h. After the reaction was complete, the mixture was cooled to room temperature. The mixture was diluted with water, and then extracted twice with EA. The organic phases were washed with saturated brine, and then dried with anhydrous sodium sulfate, filtered, concentrated under reduced pressure, and purified by column chromatography (DCM:MeOH = 100:7) to obtain Compound 57-2 (185 mg). ESI-MS m/z: 620.3 [M+H]⁺.

### Step 3: Synthesis of Compound 57-3

DMP (259.0 mg) was added to a solution of Compound 57-2 (185 mg) in DCM (10 mL). The mixture was stirred at room temperature for 30 min. After the reaction was complete, the mixture was diluted with water, extracted twice with DCM. The organic phases were combined, dried with anhydrous sodium sulfate, filtered, dried by rotary evaporation, and purified by column chromatography (PE:EA = 50:50) to obtain Compound 57-3 (110 mg). ESI-MS m/z: 618.4 [M+H]⁺.

### Step 4: Synthesis of Compound 57-4

M6 (87 mg) and ZnCl₂ (0.6 mL) were added to a solution of Compound 57-3 (80 mg) in methanol (10 mL). The temperature was raised to 40°C, and the mixture was stirred for 1 h. Then, sodium cyanoborohydride (33 mg) was added, the temperature was raised to 70°C, and the mixture was stirred for 4 h. After the reaction was complete, the solvent was dried by rotary evaporation, and the residue was purified by column chromatography (DCM:MeOH = 100:13) to obtain Compound 57-4 (100 mg). ESI-MS m/z: 631.74 [M/2+H]⁺.

### Step 5: Synthesis of Compound 57

Trifluoroacetic acid (2 mL) was slowly added to a solution of Compound 57-4 (100 mg) in DCM (8 mL). The mixture was stirred at room temperature for 20 min. After the reaction was complete, the solvent was dried by rotary evaporation. The concentrate was purified by preparative liquid chromatography (Column: Kinetex EVO C18 (21 × 150 mm, 5 µm); Mobile phase: ACN + 0.05% TFA (20%-53%); Retention time: 9.1-9.65 min) to obtain Compound 57 (26 mg). ESI-MS m/z: 609.72 1/2[M+2H]⁺.

¹H NMR (500 MHz, DMSO-d₆) δ 9.20 (s, 1H), 8.99 (d, J = 4.4 Hz, 1H), 8.76 (s, 1H), 8.59 - 8.49 (m, 2H), 8.30 (s, 1H), 7.80 - 7.73 (m, 1H), 7.44 (d, J = 8.1 Hz, 2H), 7.35 (dd, J = 12.8, 8.2 Hz, 4H), 7.03 (s, 1H), 5.54 (s, 1H), 5.40 (d, J = 10.0 Hz, 1H), 5.18 (d, J = 3.6 Hz, 1H), 5.02 (s, 1H), 4.95 - 4.90 (m, 1H), 4.40 (t, J = 8.1 Hz, 1H), 4.35 - 4.27 (m, 3H), 4.11 (dd, J = 28.6, 13.1 Hz, 1H), 3.74 (dd, J = 41.9, 13.7 Hz, 3H), 3.50 - 3.40 (m, 2H), 2.81 (s, 4H), 2.37 (s, 3H), 2.25 (s, 3H), 2.08 - 1.95 (m, 5H), 1.76 (dd, J = 47.7, 31.5 Hz, 8H), 1.55 (d, J = 6.6 Hz, 1H), 1.39 (dt, J = 22.5, 11.3 Hz, 5H), 1.24 (s, 6H), 1.08 (d, J = 6.3 Hz, 4H), 0.45 (s, 2H).

### Example 111: Synthesis of Compound (2S,4R)-1-((2S)-2-(4-(6-(4-((1-(((7-(8-ethyl-7-fluoro-3-hydroxynaphthalen-1-yl)-8-fluoro-4-(1-o xa-6-azaspiro[3.5]nonan-6-yl)pyrido[4,3-d]pyrimidin-2-yl)oxy)methyl)cyclopropyl)methyl)piper azin-1-yl)pyridin-3-yl)-1H-1,2,3-triazol-1-yl)-3-methylbutanoyl)-4-hydroxy-N-((S)-1-(4-(4-meth ylthiazol-5-yl)phenyl)ethyl)pyrrolidine-2-carboxamide

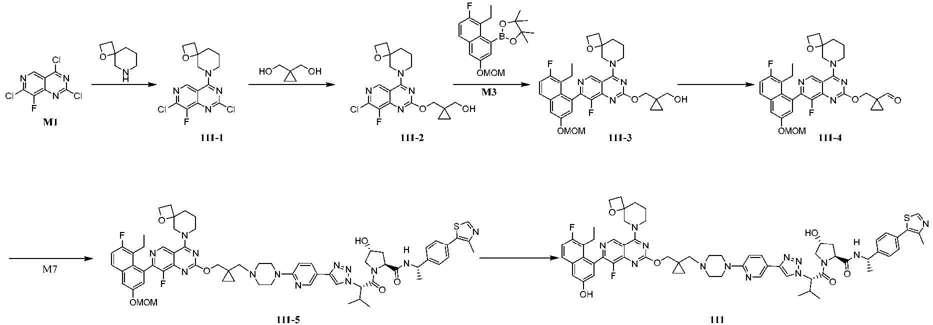

### Step 1: Synthesis of Compound 111-1

1-Oxa-6-azaspiro[3.5]nonane (185 mg), DCM (5 mL) and DIEA (280 mg) were added to a 50 mL flask. The mixture was cooled to -40°C, and Compound M1 (250 mg) was added. The mixture was maintained at this temperature and reacted for 1 hour. The mixture was dissolved in DCM, washed with water, washed with brine, dried with sodium sulfate, and filtered. The filtrate was subjected to solvent removal under reduced pressure to obtain a crude product, which was purified by column chromatography to obtain Compound 111-1 (250 mg).

### Step 2: Synthesis of Compound 111-2

Compound 111-1 (250 mg), cyclopropane-1,1-diyldimethanol (149 mg), DMF (5 mL), DABCO (15 mg) and cesium carbonate (400 mg) were added to a 50 mL flask. The mixture was reacted at room temperature for 6 hours. The mixture was dissolved in EA, washed with water, washed with brine, dried with sodium sulfate, and filtered. The filtrate was subjected to solvent removal under reduced pressure to obtain a crude product, which was purified by column chromatography to obtain Compound 111-2 (225 mg).

### Step 3: Synthesis of Compound 111-3

Compound 111-2 (225 mg), M3 (300 mg), dioxane (2 mL), water (0.2 mL), potassium phosphate (350 mg) and CataCXium A Pd G3 (40 mg) were added to a 50 mL flask. After nitrogen displacement and protection, the mixture was heated to 90°C and reacted for 2 hours. The mixture was dissolved in EA, washed with water, washed with brine, dried with sodium sulfate, and filtered. The filtrate was subjected to solvent removal under reduced pressure to obtain a crude product, which was purified by column chromatography to obtain Compound 111-3 (229 mg).

### Step 4: Synthesis of Compound 111-4

Compound 111-3 (100 mg), DCM (5 mL) and DMP (84 mg) were added to a 50 mL flask. The mixture was reacted at room temperature for 1 hour. The mixture was dissolved in DCM, washed with water, washed with brine, dried with sodium sulfate, and filtered. The filtrate was subjected to solvent removal under reduced pressure to obtain a crude product, which was purified by column chromatography to obtain Compound 111-4 (95 mg).

### Step 5: Synthesis of Compound 111-5

Compound 111-4 (95 mg), M7 (100 mg), MeOH (5 mL) and 1N ZnCl₂ solution (0.3 µL) were added to a 50 mL flask. The mixture was heated to 45°C and reacted for 1 hour. Sodium cyanoborohydride (100 mg) was added, and the reaction was continued at 45°C for 12 hours. The mixture was diluted with DCM, washed with water, washed with brine, dried with sodium sulfate, and filtered. The filtrate was subjected to solvent removal under reduced pressure to obtain a crude product, which was purified by column chromatography to obtain Compound 111-5 (60 mg).

### Step 6: Synthesis of Compound 111

Compound 111-5 (60 mg), DCM (3 mL) and TFA (1 mL) were added to a 50 mL flask. The mixture was reacted at room temperature for 0.5 hour. After detection, the mixture was concentrated under reduced pressure. The residue was dissolved in DCM, and the pH was adjusted to 7-8 with saturated aqueous sodium bicarbonate solution. The mixture was stirred, and then layered. The organic phase was washed with brine, dried with sodium sulfate, and filtered. The filtrate was subjected to solvent removal under reduced pressure to obtain a crude product, which was purified by Pre-HPLC to obtain Compound 111 (25.0 mg). ESI-MS m/z: 595.27 1/2[M+2H]⁺.

¹H NMR (500 MHz, DMSO-d₆) δ ppm 9.94 (s, 1H), 9.21 (d, J = 4.09 Hz, 1H), 8.99 (s, 1H), 8.68-8.46 (m, 1H), 8.00 (dd, J = 8.76, 1.96 Hz, 1H), 7.77 (dd, J = 9.12, 6.02 Hz, 1H), 7.52-7.17 (m, 1H), 7.06 (dd, J = 4.46, 2.62 Hz, 1H), 6.88 (d, J = 8.85 Hz, 1H), 5.32 (d, J = 10.26 Hz, 1H), 5.19 (d, J = 3.72 Hz, 1H), 5.04-4.80 (m, 1H), 4.62-4.08 (m, 1H), 3.98-3.26 (m, 1H), 3.25-3.17 (m, 1H), 2.63-1.97 (m, 1H), 1.96-1.63 (m, 1H), 1.56 (d, J = 6.98 Hz, 1H), 1.47-1.17 (m, 1H), 1.16-0.60 (m, 1H), 0.46 (s, 1H).

### Example 112: Synthesis of Compound (2S,4R)-1-((S)-2-(4-(5-((1-((1-((((S)-2-(8-ethyl-7-fluoro-3-hydroxynaphthalen-1-yl)-1-fluoro-5a, 6,9,10-tetrahydro-5H,8H-4,7-dioxa-3,10a,11,13-tetraazanaphtho[1,8-ab]heptalen-12-yl)oxy)met hyl)cyclopropyl)methyl)piperidin-4-yl)oxy)pyrazin-2-yl)-1H-1,2,3-triazol-1-yl)-3-methylbutano yl)-4-hydroxy-N-((S)-1-(4-(4-methylthiazol-5-yl)phenyl)ethyl)pyrrolidine-2-carboxamide

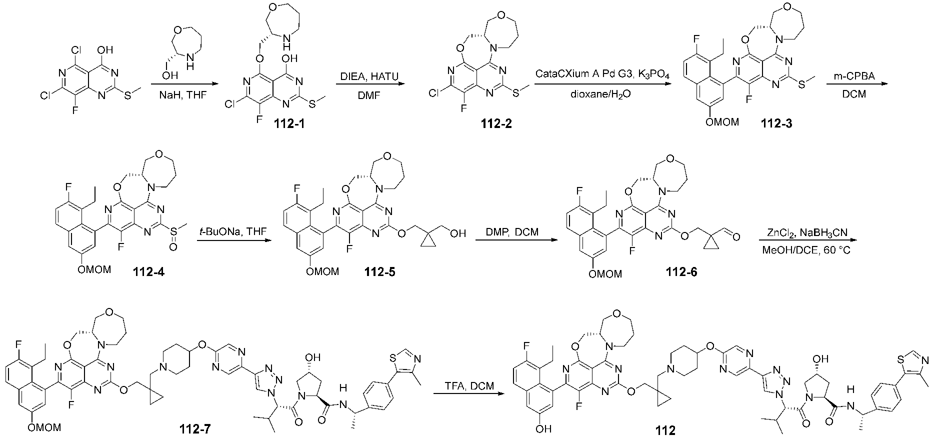

### Step 1: Synthesis of Compound 112-1

7,9-Dichloro-10-fluoro-3-methylsulfanyl-2,4,8-triazabicyclo[4.4.0]deca-2,4,6,8,10-pentaen-5-ol (400.00 mg), tetrahydrofuran (8.00 mL) and sodium hydride (102.82 mg) were added to a reaction flask. The mixture was reacted at room temperature for 5 min. Then, [(3*R*)-1,4-oxazepan-3-yl]methanol (196.68 mg) was added, and the reaction was continued at room temperature for 1 h. The reaction was quenched with ammonium chloride aqueous solution. A solid was precipitated and was collected by filtration. The filter cake was washed three times with water, and then washed once with EA. The filter cake was dried to obtain 450 mg of a yellow solid, which was Compound 112-1.

### Step 2: Synthesis of Compound 112-2

Compound 112-1 (440.00 mg), N,N-dimethylformamide (6.00 mL) and *N,N*-diisopropylethylamine (0.58 mL) were added to a reaction flask. HATU (670.00 mg) was added under stirring, and the mixture was reacted at room temperature for 0.5 h. Water was added, and a solid was precipitated. The mixture was filtered, and the filter cake was washed three times with water and dried to obtain 310 mg of a yellow solid, which was Compound 112-2.

### Step 3: Synthesis of Compound 112-3

Compound 112-2 (300.00 mg), 2-[8-ethyl-7-fluoro-3-(methoxymethoxy)naphthalen-1-yl]-4,4,5,5-tetramethyl-1,3,2-dioxaborola ne (605.76 mg), methanesulfonato[di(1-adamantyl)-n-butylphosphine](2-amino-1,1'-biphenyl-2-yl)palladium(II) (91.83 mg), potassium phosphate (535.42 mg), 1,4-dioxane (6.00 mL) and water (1.00 mL) were added to a reaction flask. Under nitrogen protection, the mixture was reacted at 90°C for 2 h. The mixture was diluted with water and extracted with EA. The organic phase was dried and concentrated. Purification by column chromatography (DCM:EA = 5:1) obtained 410 mg of a yellow solid, which was Compound 112-3.

### Step 4: Synthesis of Compound 112-4

Compound 112-3 (410.00 mg), dichloromethane (6.00 mL), and m-chloroperoxybenzoic acid (229.64 mg) were added to a reaction flask. The mixture was reacted at room temperature for 0.5 h. The pH was adjusted to 8 with aqueous sodium bicarbonate solution. The mixture was extracted with DCM. The organic phase was dried and concentrated to obtain 415 mg of a crude yellow solid, which was Compound 112-4.

### Step 5: Synthesis of Compound 112-5

[1-(Hydroxymethyl)cyclopropyl]methanol (114.55 mg), tetrahydrofuran (6.00 mL), Compound 112-4 (320.00 mg) and sodium tert-butoxide (107.79 mg) were added to a reaction flask. The mixture was reacted at room temperature for 10 min. The reaction was quenched with ammonium chloride aqueous solution under an ice bath, then extracted with EA. The organic phase was dried and concentrated. Purification by column chromatography (DCM:EA = 3:1) obtained 180 mg of a yellow solid, which was Compound 112-5.

### Step 6: Synthesis of Compound 112-6

Compound 112-5 (170.00 mg), dichloromethane (5.00 mL) and Dess-Martin periodinane (154.00 mg) were added to a reaction flask. The mixture was reacted at room temperature for 0.5 h. The mixture was diluted with saturated sodium bicarbonate aqueous solution, then extracted with DCM. The organic phase was dried and concentrated. Purification by column chromatography (DCM:EA = 3:1) obtained 95 mg of a yellow solid, which was Compound 112-6.

### Step 7: Synthesis of Compound 112-7

Compound 112-6 (45.00 mg), Intermediate M6 (53.84 mg), methanol (1.50 mL), 1,2-dichloroethane (1.50 mL) and 1 M zinc chloride in THF (0.07 mL) were added to a reaction flask. The mixture was reacted at 40°C for 1 h. Then, sodium cyanoborohydride (37.30 mg) was added, and the mixture was reacted at 60°C for 3 h. The mixture was diluted with water, then extracted with DCM and MeOH. The organic phase was dried and concentrated. Purification by Pre-TLC obtained 52 mg of a pale yellow solid, which was Compound 112-7.

### Step 8: Synthesis of Compound 112

Compound 112-7 (45.00 mg), dichloromethane (1.00 mL) and trifluoroacetic acid (1.00 mL) were added to a reaction flask. The mixture was reacted at room temperature for 0.5 h. The reaction solution was concentrated. The residue was dissolved in DCM, and the solution was added to ice-cold sodium bicarbonate aqueous solution to adjust the pH to 8. The mixture was extracted with DCM and methanol. The organic phase was dried and concentrated. Purification by Pre-TLC obtained Compound 112 (31 mg). LCMS: 1/2[M+2H]⁺ = 604.21.

¹H NMR (500 MHz, Methanol-d₄) δ 8.86 (d, J = 9.1 Hz, 1H), 8.71 (t, J = 1.3 Hz, 1H), 8.55 (d, J = 13.8 Hz, 1H), 8.17 (dd, J = 4.7, 1.3 Hz, 1H), 7.66 - 7.59 (m, 1H), 7.47 - 7.37 (m, 4H), 7.30 - 7.18 (m, 2H), 7.10 (dd, J = 48.6, 2.3 Hz, 1H), 5.40 (d, J = 10.1 Hz, 1H), 5.20 (m, 1H), 5.06 (m, 2H), 4.76 - 4.68 (m, 1H), 4.63 - 4.52 (m, 2H), 4.49 - 4.41 (m, 3H), 4.37 - 4.25 (m, 1H), 4.16 (m, 1H), 4.01 - 3.85 (m, 3H), 3.76 (m, 1H), 3.60 - 3.53 (m, 1H), 3.48 - 3.36 (m, 1H), 3.31 (m, 3H), 3.00 (d, J = 10.8 Hz, 2H), 2.70 - 2.48 (m, 5H), 2.44 - 2.27 (m, 3H), 2.25 - 2.06 (m, 4H), 1.94 (m, 4H), 1.52 (d, J = 7.0 Hz, 3H), 1.15 (t, J = 6.9 Hz, 3H), 0.86 (m, 6H), 0.76 (s, 2H), 0.57 (s, 2H).

### Example 113: Synthesis of Compound (2S,4R)-1-((S)-2-(4-(5-((1-((1-(((7-(8-ethyl-7-fluoro-3-hydroxynaphthalen-1-yl)-8-fluoro-4-(3-ox oazepan-1-yl)pyrido[4,3-d]pyrimidin-2-yl)oxy)methyl)cyclopropyl)methyl)piperidin-4-yl)oxy)p yrazin-2-yl)-1H-1,2,3-triazol-1-yl)-3-methylbutanoyl)-4-hydroxy-N-((S)-1-(4-(4-methylthiazol-5 -yl)phenyl)ethyl)pyrrolidine-2-carboxamide

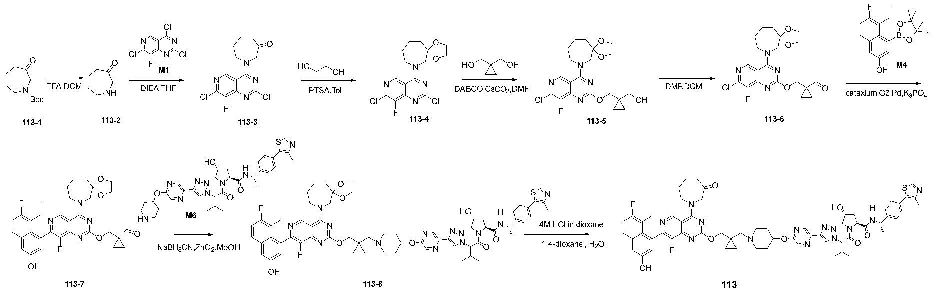

### Step 1: Synthesis of Compound 113-2

Compound 113-1 (500.0 mg) was added to a 100 mL single-neck flask and dissolved in DCM (5 mL). TFA (2.5 mL) was added dropwise, and the mixture was reacted at room temperature for 1 h. The reaction solution was directly evaporated to dryness to obtain 450 mg of a pale yellow oil product, which was set aside to be used. This product was Compound 113-2.

### Step 2: Synthesis of Compound 113-3

Compound M1 (709.0 mg) was added to a 100 mL single-neck flask and dissolved in THF (10 mL). DIEA (1.94 mL) was added. Then, the obtained pale yellow oily Compound 113-2 was dissolved in THF (2 mL) and added dropwise to the above reaction solution under an ice bath. The mixture was reacted for 1 h. The reaction solution was added to 30 mL water, and extracted twice with EA. The organic phases were combined, washed once with saturated brine, dried with anhydrous sodium sulfate, and purified by column chromatography (DCM:MeOH = 32:1) to obtain 510 mg of a pale yellow solid, which was Compound 113-3.

### Step 3: Synthesis of Compound 113-4

Compound 113-3 (510 mg) and ethylene glycol (200 mg) were added to a 100 mL single-neck flask and dissolved in toluene (15 mL). p-Toluenesulfonic acid (26.7 mg) was added. The mixture was heated to 110°C and reacted for 5 h. After cooling, the reaction solution was concentrated with silica gel and purified by column chromatography (DCM:MeOH = 50:1) to obtain 175 mg of a white solid, which was Compound 113-4.

### Step 4: Synthesis of Compound 113-5

Compound 113-4 (175 mg), cyclopropanedimethanol (95.4 mg), DABCO (10.5 mg) and cesium carbonate (305.4 mg) were added to a 50 mL single-neck flask and dissolved in DMF (8 mL). The mixture was stirred at room temperature for 1 h. The reaction solution was added to water (50 mL), and extracted twice with EA. The organic phases were combined, washed once with saturated brine, dried with anhydrous sodium sulfate, and purified by column chromatography (DCM:MeOH = 32:1) to obtain 210 mg of a yellow oil, which was Compound 113-5.

### Step 5: Synthesis of Compound 113-6

Compound 113-5 (210 mg) was added to a 50 mL single-neck flask and dissolved in DCM (6 mL). DMP (250 mg) was added, and the mixture was reacted with stirring at room temperature for 1 h. The reaction solution was filtered through a filter head. The filtrate was washed once with saturated sodium bicarbonate solution, once with saturated brine, dried with anhydrous sodium sulfate, and purified by column chromatography (DCM:MeOH = 25:1) to obtain 170 mg of a yellow oil, which was Compound 113-6.

### Step 6: Synthesis of Compound 113-7

Compound 113-6 (130 mg), Intermediate M4 (188 mg), CataCXium A Pd G3 (21.7 mg) and potassium phosphate (190 mg) were added to a 50 mL three-neck flask and dissolved in 1,4-dioxane (7 mL). Water (0.7 mL) was added. After nitrogen displacement three times, the mixture was stirred at 100°C and was reacted for 3 h. The reaction solution was cooled to room temperature and purified by column chromatography (DCM:MeOH = 20:1) to obtain 90 mg of a yellow solid, which was Compound 113-7.

### Step 7: Synthesis of Compound 113-8

Compound 113-7 (90 mg) was added to a 25 mL single-neck flask and dissolved in MeOH (5 mL). M6 (120 mg) and 1 M zinc chloride in THF (0.1 mL) were added. The mixture was heated to 40°C and reacted for 2 h. Then, NaBH₃CN (50 mg) was added, and the reaction was continued overnight. The reaction solution was added to an appropriate amount of brine, extracted twice with a DCM:MeOH = 5:1 solution. The organic phases were combined, washed once with saturated brine, dried with anhydrous sodium sulfate, and purified by column chromatography (DCM:MeOH = 10:1) to obtain 95 mg of a brown solid, which was Compound 113-8.

### Step 8: Synthesis of Compound 113

Compound 113-8 (95 mg) was added to a 25 mL single-neck flask and dissolved in 1,4-dioxane (2 mL). Water (0.4 mL) and 4M HCl in dioxane (0.4 mL) were added. The mixture was reacted for 1 h. Saturated sodium bicarbonate solution was added to the reaction solution to adjust the pH to ~8. The mixture was extracted twice with DCM:MeOH = 5:1. The organic phases were combined, dried with anhydrous sodium sulfate, and concentrated. The product was separated by Pre-HPLC (CH₃CN/H₂O = 60%:40%) to obtain Compound 113 (19.5 mg, purity 98.02%). LCMS: 1/2[M+2H]⁺ = 596.2.

### Example 114: Synthesis of Compound (2S,4R)-1-((S)-2-(4-(5-((1-((1-(((7-(8-ethyl-7-fluoro-3-hydroxynaphthalen-1-yl)-8-fluoro-4-((S)-6-hydroxy-6-methyl-1,4-oxazepan-4-yl)pyrido[4,3-d]pyrimidin-2-yl)oxy)methyl)cyclopropyl)me thyl)piperidin-4-yl)oxy)pyrazin-2-yl)-1H-1,2,3-triazol-1-yl)-3-methylbutanoyl)-4-hydroxy-N-((S )-1-(4-(4-methylthiazol-5-yl)phenyl)ethyl)pyrrolidine-2-carboxamide

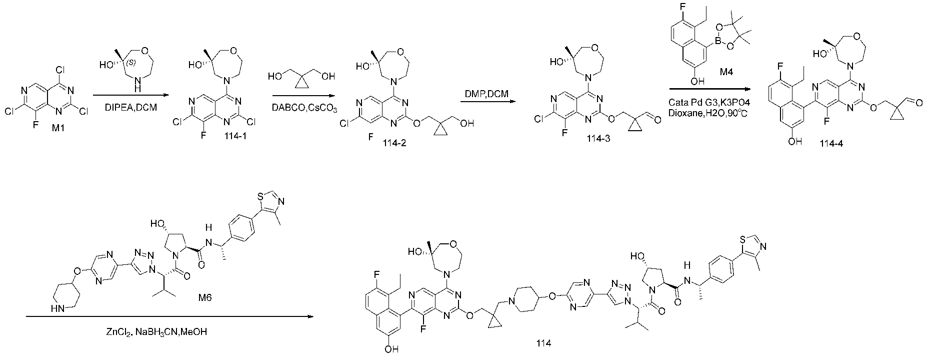

### Step 1: Synthesis of Compound 114-1

(*S*)-6-Methyl-1,4-oxazepan-6-ol (390 mg) and DIPEA (1.1 g) were added to a 100 mL single-neck flask and dissolved in DCM (30 mL). The mixture was cooled to below -40°C. Compound M1 (670 mg) was added in batches, and the mixture was reacted with stirring for 1.0 h. After the reaction was complete, the mixture was quenched with saturated ammonium chloride, and extracted with dichloromethane, washed with saturated brine, dried with anhydrous sodium sulfate, filtered, dried by rotary evaporation, and purified by column chromatography (PE:EA = 1:1) to obtain a pale yellow solid, which was Compound 114-1 (800 mg). ESI-MS m/z: 347.1 [M+H]⁺.

### Step 2: Synthesis of Compound 114-2

Compound 114-1 (400 mg), 1,1-cyclopropanedimethanol (240 mg), cesium carbonate (750 mg), and DABCO (30 mg) were added to a 50 mL single-neck flask and dissolved in DMF (15 mL). The mixture was stirred at room temperature overnight. After the reaction was complete, the mixture was diluted with water, extracted with ethyl acetate, washed with saturated brine, dried with anhydrous sodium sulfate, filtered, dried by rotary evaporation, and purified by column chromatography (DCM:EA = 9:1) to obtain a pale yellow solid, which was Compound 114-2 (250 mg). ESI-MS m/z: 413.2 [M+H]⁺.

### Step 3: Synthesis of Compound 114-3

DMP (379 mg) was added to a solution of Compound 114-2 (250 mg) in DCM (10 mL). The mixture was stirred at room temperature for 30 min. After the reaction was complete, the mixture was diluted with water, extracted with dichloromethane. The organic phases were combined, dried with anhydrous sodium sulfate, filtered, dried by rotary evaporation, and purified by column chromatography (DCM:EA = 6:1) to obtain a pale yellow solid, which was Compound 114-3 (210 mg). ESI-MS m/z: 410.2 [M+H]⁺.

### Step 4: Synthesis of Compound 114-4

Compound 114-3 (210 mg), 2-(8-ethyl-7-fluoro-3-(methoxymethoxy)naphthalen-1-yl)-4,4,5,5-tetramethyl-1,3,2-dioxaborola ne (310.7 mg), CataCXium A Pd G3 (70.5 mg), K₃PO₄ (208.1 mg), 1,4-dioxane (8 mL), and H₂O (2 mL) were combined. The temperature was raised to 90°C, and the mixture was reacted with stirring for 1.0 h. After the reaction was complete, the mixture was cooled to room temperature, extracted with ethyl acetate, washed with saturated brine, dried with anhydrous sodium sulfate, filtered, dried by rotary evaporation, and purified by column chromatography (DCM:MeOH = 15:1) to obtain a pale yellow solid, which was Compound 114-4 (150 mg). ESI-MS m/z: 565.4 [M+H]⁺.

### Step 5: Synthesis of Target Compound 114

M6 (128 mg) and ZnCl₂ (0.8 mL) were added to a solution of Compound 114-4 (150 mg) in methanol (40 mL). The temperature was raised to 40°C, and the mixture was stirred for 1.0 h. Then, sodium cyanoborohydride (58 mg) was added, the temperature was raised to 70°C, and the mixture was stirred for 4.0 h. After the reaction was complete, the solvent was dried by rotary evaporation, and the concentrate was purified by Pre-HPLC (C18 column, A: 0.05% diethylamine in water, B: acetonitrile, Gradient: 20% B to 85% B, 10.41 min, Flow rate: 20 mL/min, 254 nm) to obtain target Compound 114 (24.5 mg). ESI-MS m/z: 604.72 1/2[M+2H]⁺.

¹H NMR (500 MHz, DMSO-d₆) δ 9.96 (s, 1H), 9.48 (s, 1H), 8.99 (s, 1H), 8.76 (t, J = 2.4 Hz, 1H), 8.59 - 8.50 (m, 2H), 8.30 (s, 1H), 7.77 (dd, J = 9.0, 6.1 Hz, 1H), 7.45 (d, J = 8.2 Hz, 2H), 7.35 (dd, J = 11.5, 9.1 Hz, 4H), 7.03 (dd, J = 18.0, 2.4 Hz, 1H), 5.40 (d, J = 10.1 Hz, 1H), 5.18 (dd, J = 10.0, 6.2 Hz, 2H), 5.02 (s, 1H), 4.97 - 4.89 (m, 1H), 4.36 (dt, J = 30.0, 11.3 Hz, 6H), 4.15 (dd, J = 29.2, 14.7 Hz, 1H), 4.08 - 3.94 (m, 3H), 3.74 (dd, J = 34.7, 9.1 Hz, 2H), 3.62 - 3.51 (m, 2H), 2.33 (t, J = 38.0 Hz, 6H), 2.19 - 1.93 (m, 6H), 1.82 - 1.73 (m, 1H), 1.67 (s, 2H), 1.60 - 1.43 (m, 1H), 1.38 (d, J = 7.0 Hz, 3H), 1.24 (s, 3H), 1.10 (ddd, J = 22.3, 18.5, 7.1 Hz, 8H), 0.85 (t, J = 6.9 Hz, 1H), 0.78 - 0.63 (m, 9H), 0.44 (s, 2H).

### Example 115: Synthesis of Compound (2S,4R)-1-((S)-2-(4-(5-((9-((1-(((7-(8-ethyl-7-fluoro-3-hydroxynaphthalen-1-yl)-8-fluoro-4-((S)-6-hydroxy-6-methyl-1,4-oxazepan-4-yl)pyrido[4,3-d]pyrimidin-2-yl)oxy)methyl)cyclopropyl)me thyl)-3,9-diazaspiro[5.5]undecan-3-yl)methyl)pyrazin-2-yl)-1H-1,2,3-triazol-1-yl)-3-methylbuta noyl)-4-hydroxy-N-((S)-1-(4-(4-methylthiazol-5-yl)phenyl)ethyl)pyrrolidine-2-carboxamide

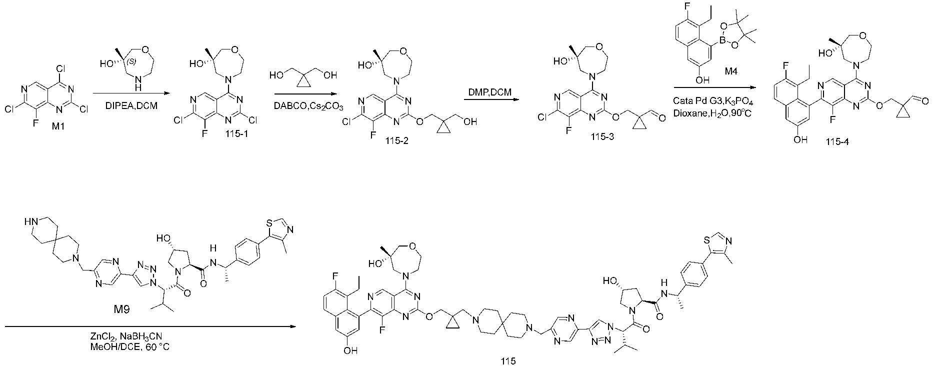

### Step 1: Synthesis of Compound 115-1

(*S*)-6-Methyl-1,4-oxazepan-6-ol (390 mg) and DIPEA (1.1 g) were added to a 100 mL single-neck flask and dissolved in DCM (30 mL). The mixture was cooled to below -40°C. Compound M1 (670 mg) was added in batches, and the reaction mixture was stirred for 1.0 h. After the reaction was complete, the mixture was quenched with saturated ammonium chloride, extracted with dichloromethane, washed with saturated brine, dried with anhydrous sodium sulfate, filtered, dried by rotary evaporation, and purified by column chromatography (PE:EA = 1:1) to obtain a pale yellow solid, which was Compound 115-1 (800 mg). ESI-MS m/z: 347.1 [M+H]⁺.

### Step 2: Synthesis of Compound 115-2

Compound 115-1 (400 mg), 1,1-cyclopropanedimethanol (240 mg), cesium carbonate (750 mg) and DABCO (30 mg) were added to a 50 mL single-neck flask and dissolved in DMF (15 mL). The mixture was stirred at room temperature overnight. After the reaction was complete, the mixture was diluted with water, extracted with ethyl acetate, washed with saturated brine, dried with anhydrous sodium sulfate, filtered, dried by rotary evaporation, and purified by column chromatography (DCM:EA = 9:1) to obtain a pale yellow solid, which was Compound 115-2 (250 mg). ESI-MS m/z: 413.2 [M+H]⁺.

### Step 3: Synthesis of Compound 115-3

DMP (379 mg) was added to a solution of Compound 115-2 (250 mg) in DCM (10 mL). The mixture was stirred at room temperature for 30 min. After the reaction was complete, the mixture was diluted with water, extracted with dichloromethane. The organic phases were combined, dried with anhydrous sodium sulfate, filtered, dried by rotary evaporation, and purified by column chromatography (DCM:EA = 6:1) to obtain a pale yellow solid, which was Compound 115-3 (210 mg). ESI-MS m/z: 410.2 [M+H]⁺.

### Step 4: Synthesis of Compound 115-4

Compound 115-3 (210 mg), 2-(8-ethyl-7-fluoro-3-(methoxymethoxy)naphthalen-1-yl)-4,4,5,5-tetramethyl-1,3,2-dioxaborola ne (310.7 mg), CataCXium A Pd G3 (70.5 mg), K₃PO₄ (208.1 mg), 1,4-dioxane (8 mL) and H₂O (2 mL) were combined. The temperature was raised to 90°C, and the mixture was reacted with stirring for 1.0 h. After the reaction was complete, the mixture was cooled to room temperature, extracted with ethyl acetate, washed with saturated brine, dried with anhydrous sodium sulfate, filtered, dried by rotary evaporation, and purified by column chromatography (DCM:MeOH = 15:1) to obtain a pale yellow solid, which was Compound 115-4 (150 mg). ESI-MS m/z: 565.4 [M+H]⁺.

### Step 5: Synthesis of target Compound 115

M9 (58 mg) and ZnCl₂ (0.2 mL) were added to a solution of Compound 115-4 (45 mg) in methanol (10 mL). The temperature was raised to 40°C, and the mixture was stirred for 1.0 h. Then, sodium cyanoborohydride (20 mg) was added, the temperature was raised to 60°C, and the mixture was stirred for 4.0 h. After the reaction was complete, the solvent was dried by rotary evaporation. An appropriate amount of water and DCM/MeOH (10:1) were added to the residue, and the mixture was extracted. The organic phase was collected, dried with anhydrous sodium sulfate, filtered, and dried by rotary evaporation. The concentrate was purified by Pre-HPLC (C18 column, A: 0.05% diethylamine in water, B: acetonitrile, Gradient: 30% B to 80% B, 9.0 min, Flow rate: 16 mL/min, 254 nm) to obtain target Compound 115 (11.6 mg). ESI-MS m/z: 638.3 1/2[M+2H]⁺.

¹H NMR (500 MHz, MeOD) δ 9.50 (d, J = 10.4 Hz, 1H), 9.18 (t, J = 4.6 Hz, 1H), 8.85 (d, J = 8.5 Hz, 1H), 8.72 (d, J = 11.5 Hz, 1H), 8.66 (s, 1H), 7.63 (dd, J = 9.0, 5.9 Hz, 1H), 7.46 - 7.35 (m, 4H), 7.27 (d, J = 2.0 Hz, 1H), 7.21 (t, J = 9.3 Hz, 1H), 7.05 (dd, J = 20.2, 2.4 Hz, 1H), 5.43 (d, J = 10.0 Hz, 1H), 5.09 - 5.01 (m, 1H), 4.57 - 4.32 (m, 6H), 4.19 - 4.11 (m, 1H), 4.03 - 3.94 (m, 2H), 3.93 - 3.79 (m, 3H), 3.75 - 3.58 (m, 4H), 2.68 - 2.58 (m, 1H), 2.52 - 2.34 (m, 14H), 2.17 (dt, J = 18.5, 9.3 Hz, 2H), 2.00 - 1.92 (m, 1H), 1.56 - 1.39 (m, 11H), 1.26 (d, J = 15.6 Hz, 3H), 1.15 (t, J = 7.8 Hz, 3H), 0.86 - 0.75 (m, 6H), 0.68 (s, 2H), 0.47 (s, 2H).

### Example 131: Synthesis of Compound (2S,4R)-N-((R)-2-(dimethylamino)-1-(4-(4-methylthiazol-5-yl)phenyl)ethyl)-1-((S)-2-(4-(5-((1-(( 1-(((7-(8-ethyl-7-fluoro-3-hydroxynaphthalen-1-yl)-8-fluoro-4-((S)-6-hydroxy-6-methyl-1,4-oxa zepan-4-yl)pyrido[4,3-d]pyrimidin-2-yl)oxy)methyl)cyclopropyl)methyl)piperidin-4-yl)oxy)pyra zin-2-yl)-1H-1,2,3-triazol-1-yl)-3-methylbutanoyl)-4-hydroxypyrrolidine-2-carboxamide

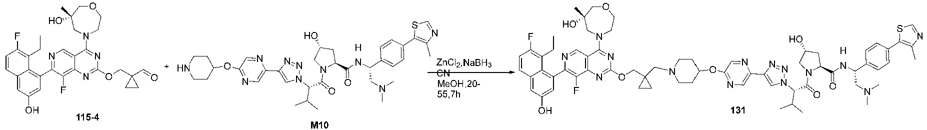

1 M zinc chloride in THF (0.06 mL) was added to a solution of 115-4 (36.13 mg) and Intermediate M10 (45.00 mg) in methanol (1.50 mL). The mixture was reacted at 50°C for 1 h. Sodium cyanoborohydride (16.09 mg) was added, and the reaction was continued at 50°C for 6 h. The reaction solution was added to 30 mL water, and the mixture was extracted with DCM. The organic phase was dried, filtered, and concentrated under reduced pressure. The residue was purified by Pre-TLC (MeOH:DCM = 1:12) to obtain 28.90 mg of Compound 131 (purity 95.64%). LCMS: 1/2[M+2H]⁺ = 626.20.

¹H NMR (500 MHz, Methanol-d₄) δ 9.43 (s, 1H), 8.79 (s, 1H), 8.64 (s, 1H), 8.45 (s, 1H), 8.09 (d, J = 5.0 Hz, 1H), 7.59 - 7.56 (m, 1H), 7.39 - 7.29 (m, 4H), 7.20 (s, 1H), 7.23 (t, J = 10.0 Hz, 1H), 6.95 (s, 1H), 5.32 - 5.30 (m, 1H), 5.06 - 5.02 (m, 3H), 4.55 - 4.44 (m, 3H), 4.37 (d, J = 5.0 Hz, 4H), 4.12 - 4.08 (m, 1H), 3.95 - 3.90 (m, 2H), 3.85 - 3.76 (m, 4H), 3.65 - 3.55 (m, 3H), 2.88 - 2.84 (m, 3H), 2.54 - 2.48 (m, 4H), 2.39 - 2.36 (m, 4H), 2.13 - 2.08 (m, 3H), 1.98 - 1.94 (m, 3H), 1.88 - 1.83 (m, 2H), 1.71 (d, J = 5.0 Hz, 3H), 1.06 (d, J = 5.0 Hz, 3H), 0.74 - 0.72 (m, 7H), 0.66 (s, 2H), 0.46 (s, 2H).

### Example 133: Synthesis of Compound (2S,4R)-1-((2S)-2-(4-(5-((1-((1-(((5-(8-ethyl-7-fluoro-3-hydroxynaphthalen-1-yl)-4-fluoro-9-hyd roxy-9-methyl-8,8a,9,10,11,12-hexahydro-7-oxa-1,3,6,12a-tetraazabenzo[4,5]cyclohepta[1,2,3-d e]naphthalen-2-yl)oxy)methyl)cyclopropyl)methyl)piperidin-4-yl)oxy)pyrazin-2-yl)-1H-1,2,3-tri azol-1-yl)-3-methylbutanoyl)-4-hydroxy-N-((S)-1-(4-(4-methylthiazol-5-yl)phenyl)ethyl)pyrrolid ine-2-carboxamide

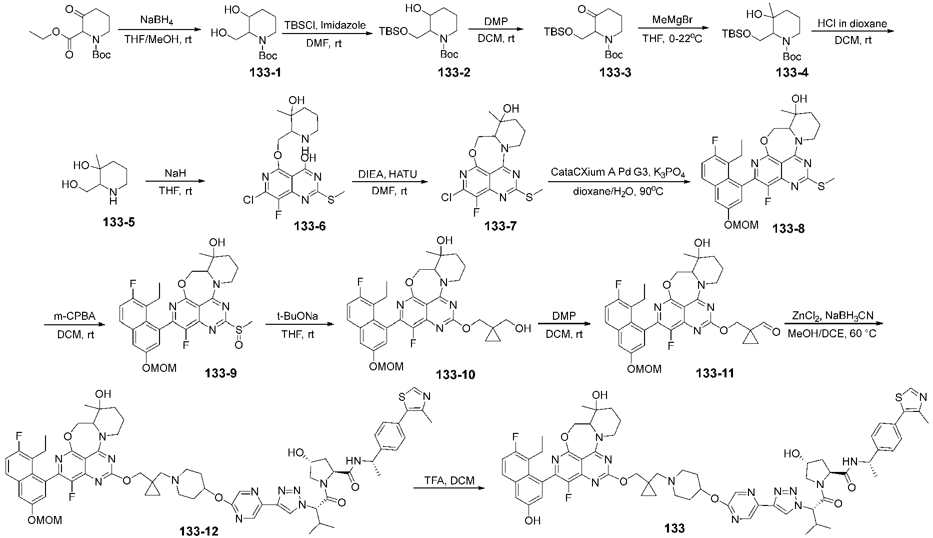

### Step 1: Synthesis of Compound 133-1

tert-Butyl ethyl 3-oxopiperidine-1,2-dicarboxylate (2.20 g), tetrahydrofuran (6.00 mL), and methanol (1.50 mL) were added to a reaction flask. Sodium borohydride (1.84 g) was slowly added, and the mixture was reacted at room temperature for 1 h. The reaction was quenched by adding water under an ice bath, then extracted with EA. The organic phase was washed once with saturated brine, dried, and concentrated. Purification by column chromatography (PE:EA = 4:1) obtained 1.74 g of a colorless liquid, which was Compound 133-1.

### Step 2: Synthesis of Compound 133-2

Compound 133-1 (1.72 g), N,N-dimethylformamide (15.00 mL), and tert-butyldimethylchlorosilane (1.46 g) were added to a reaction flask. Imidazole (1.01 g) was added under stirring, and the mixture was reacted at room temperature for 2 h. The mixture was diluted with water, then extracted with EA, dried, and concentrated. Purification by column chromatography (PE:EA = 10:1) obtained 1.79 g of a colorless liquid, which was Compound 133-2.

### Step 3: Synthesis of Compound 133-3

Compound 133-2 (1.79 g), dichloromethane (1.00 mL) and Dess-Martin periodinane (3.30 g) were added to a reaction flask. The mixture was reacted at room temperature for 0.5 h. The mixture was diluted with saturated sodium bicarbonate aqueous solution, then extracted with DCM, dried, and concentrated. Purification by column chromatography (PE:EA = 10:1) obtained 1.5 g of a colorless liquid, which was Compound 133-3.

### Step 4: Synthesis of Compound 133-4

Compound 133-3 (1.50 g) and tetrahydrofuran (20.00 mL) were added to a reaction flask. After nitrogen displacement, the mixture was cooled to 0°C. Methylmagnesium bromide (1.04 g, 8.73 mmol) was slowly added. After the addition was complete, the mixture was allowed to warm to room temperature and reacted for 10 h. The reaction was quenched by adding ammonium chloride aqueous solution under an ice bath, extracted with EA, dried, and concentrated. Purification by column chromatography (PE:EA = 3:1) obtained 0.79 g of a yellow liquid, which was Compound 133-4.

### Step 5: Synthesis of Compound 133-5

Compound 133-4 (720.00 mg), dichloromethane (8.00 mL) and 4M HCl in dioxane (4.00 mL) were added to a reaction flask. The mixture was reacted at room temperature for 0.5 h. TLC showed the raw materials were completely consumed. The solvent was dried by rotary evaporation to obtain 800 mg of a crude yellow solid, which was Compound 133-5.

### Step 6: Synthesis of Compound 133-6

Compound 133-5 (270.00 mg), tetrahydrofuran (10.00 mL) and sodium hydride (154.22 mg) were added to a reaction flask. After reacting at room temperature for 5 min, 7,9-dichloro-10-fluoro-3-methylsulfanyl-2,4,8-triazabicyclo[4.4.0]deca-2,4,6,8,10-pentaen-5-ol (450.00 mg) was added. The mixture was reacted at room temperature for 0.5 h. The reaction was quenched with ammonium chloride aqueous solution under an ice bath, extracted with EA, dried, and concentrated. Purification by column chromatography (DCM:MeOH = 6:1) obtained 410 mg of a yellow solid, which was Compound 133-6.

### Step 7: Synthesis of Compound 133-7

Compound 133-6 (400.00 mg), N,N-dimethylformamide (0.80 mL), and N,N-diisopropylethylamine (0.51 mL, 3.09 mmol) were added to a reaction flask. 2-(7-azabenzotriazol-1-yl)-*N,N,N',N'*-tetramethyluronium hexafluorophosphate (586.73 mg) was added under stirring, and the mixture was reacted at room temperature for 3 h. Water was slowly added at room temperature, and a solid was precipitated. The mixture was filtered, and the filter cake was washed three more times with water. The filter cake was dried to obtain 230 mg of a pale yellow solid, which was Compound 133-7.

### Step 8: Synthesis of Compound 133-8

Compound 133-7 (220.0 mg), 2-[8-ethyl-7-fluoro-3-(methoxymethoxy)naphthalen-1-yl]-4,4,5,5-tetramethyl-1,3,2-dioxaborola ne (427.44 mg), methanesulfonato[di(1-adamantyl)-n-butylphosphine](2-amino-1,1'-biphenyl-2-yl)palladium(II) (43.19 mg), potassium phosphate (377.81 mg), 1,4-dioxane (5.00 mL) and water (0.90 mL) were added to a reaction flask. After nitrogen displacement, the mixture was reacted at 90°C for 2 h under nitrogen protection. The mixture was diluted with water, then extracted with EA, dried, and concentrated. Purification by column chromatography (DCM:EA = 5:1) obtained 300 mg of a yellow solid, which was Compound 133-8.

### Step 9: Synthesis of Compound 133-9

Compound 133-8 (300.0 mg), dichloromethane (6.00 mL) and m-chloroperoxybenzoic acid (264.03 mg) were added to a reaction flask. The mixture was reacted at room temperature for 0.3 h. The pH was adjusted to 8 with sodium bicarbonate aqueous solution, then extracted with DCM, dried, and concentrated to obtain 280 mg of a crude yellow solid, which was Compound 133-9.

### Step 10: Synthesis of Compound 133-10

Compound 133-9 (270.00 mg), [1-(hydroxymethyl)cyclopropyl]methanol (91.82 mg), tetrahydrofuran (6.00 mL) and sodium tert-butoxide (86.39 mg) were added to a reaction flask. The mixture was reacted at room temperature for 0.2 h. The reaction was quenched with ammonium chloride aqueous solution under an ice bath, extracted with EA, dried, and concentrated. Purification by column chromatography (DCM:EA = 1:1) obtained 180 mg of a yellow solid, which was Compound 133-10.

### Step 11: Synthesis of Compound 133-11

Compound 133-10 (180.00 mg), dichloromethane (5.00 mL), and Dess-Martin periodinane (183.95 mg) were added to a reaction flask. The mixture was reacted at room temperature for 0.5 h. The pH was adjusted to 8 with sodium bicarbonate aqueous solution, extracted with DCM, dried, and concentrated. Purification by column chromatography (DCM:EA = 2:1) obtained 120 mg of a yellow solid, which was Compound 133-11.

### Step 12: Synthesis of Compound 133-12

Compound 133-11 (120.00 mg), Intermediate M6 (140.27 mg), 1,2-dichloroethane (4.00 mL), methanol (MeOH) (4.00 mL) and 1 M zinc chloride in THF (0.19 mL) were added to a reaction flask. The mixture was reacted at 40°C for 1 h. Sodium cyanoborohydride (121.47 mg) was added, and the reaction was continued at 60°C for 3 h. Silica gel was directly added to the mixture. Purification by column chromatography (DCM:MeOH = 15:1) obtained 180 mg of a yellow solid, which was Compound 133-12.

### Step 13: Synthesis of Compound 133

Compound 133-12 (170.00 mg), dichloromethane (2.00 mL) and trifluoroacetic acid (2.00 mL) were added to a reaction flask. The mixture was reacted at room temperature for 0.5 h, then concentrated. The residue was dissolved in DCM, and the solution was added to ice-cold sodium bicarbonate aqueous solution to adjust the pH to 8. The mixture was extracted with DCM and methanol, dried, and concentrated. Purification by Pre-TLC (DCM:MeOH = 10:1) obtained 88.6 mg of Compound 133. LCMS: 1/2[M+2H]⁺ = 611.02.

¹H NMR (500 MHz, Methanol-d₄) δ 8.86 (d, J = 7.7 Hz, 1H), 8.72 (s, 1H), 8.55 (d, J = 13.2 Hz, 1H), 8.17 (d, J = 5.7 Hz, 1H), 7.62 (d, J = 7.6 Hz, 1H), 7.42 (d, J = 5.6 Hz, 4H), 7.23 (dd, J = 21.4, 12.2 Hz, 3H), 5.54 - 5.32 (m, 2H), 5.06 (m, 2H), 4.61 - 4.36 (m, 5H), 3.97 - 3.73 (m, 3H), 3.15 - 2.89 (m, 3H), 2.70 - 2.41 (m, 9H), 2.08 (t, J = 58.0 Hz, 6H), 1.73 (m, 6H), 1.57 - 1.39 (m, 6H), 1.28 (m, 1H), 1.16 (d, J = 6.0 Hz, 3H), 0.95 - 0.73 (m, 8H), 0.56 (s, 2H).

### Example 134: Synthesis of Compound (2S,4R)-1-((2S)-2-(4-(5-((1-((1-(((7-(8-ethyl-7-fluoro-3-hydroxynaphthalen-1-yl)-8-fluoro-4-(3-h ydroxy-3-methylazepan-1-yl)pyrido[4,3-d]pyrimidin-2-yl)oxy)methyl)cyclopropyl)methyl)piperi din-4-yl)oxy)pyrazin-2-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

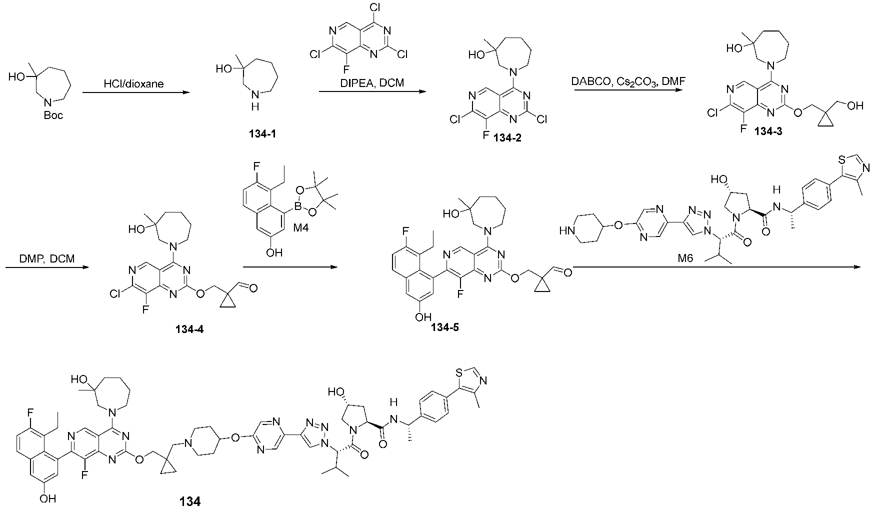

### Step 1: Synthesis of Compound 134-1

tert-Butyl 3-hydroxy-3-methylazetidine-1-carboxylate (1.0 g) was dissolved in DCM (20 mL). 10 mL HCl in dioxane solution was added, and the mixture was stirred for 30 min. After the reaction was complete, the mixture was concentrated to obtain Compound 134-1 (0.56 g). ESI-MS m/z: 130.1 [M+H]⁺.

### Step 2: Synthesis of Compound 134-2

2,4,7-Trichloro-8-fluoropyrido[4,3-d]pyrimidine (1.0 g) was dissolved in DCM (20 mL). The mixture was cooled to about -40°C. DIPEA (1.38 g) was added, and the mixture was stirred at low temperature for 30 min. 134-1 (0.53 g) was slowly added, and the reaction was continued with stirring for 1.0 h. After the reaction was complete, the mixture was diluted with water, extracted with DCM, washed with saturated brine, dried with anhydrous sodium sulfate, filtered, concentrated, and purified by column chromatography (EA:DCM = 1:10) to obtain Compound 134-2 (1.0 g). ESI-MS m/z: 345.3 [M+H]⁺.

### Step 3: Synthesis of Compound 134-3

Compound 134-2 (1.0 g), cesium carbonate (2.1 g), DABCO (0.16 g) and 1,1-cyclopropanedimethanol (0.67 g) were dissolved in DMF (10 mL). The mixture was stirred at room temperature for 1 hour. After the reaction was complete, the mixture was diluted with water, extracted with EA, washed with saturated brine, dried with anhydrous sodium sulfate, filtered, concentrated, and purified by column chromatography (EA:DCM = 1:4) to obtain Compound 134-3 (0.8 g). ESI-MS m/z: 411.1 [M+H]⁺.

### Step 4: Synthesis of Compound 134-4

DMP (3 g) was added to a solution of Compound 134-3 (0.8 g) in DCM (20 mL) in batches. The mixture was stirred at room temperature for 3.0 h. After the reaction was complete, the mixture was quenched with saturated sodium sulfite, diluted with water, extracted with DCM. The aqueous phase was further extracted twice with DCM. The organic phases were combined, dried with anhydrous sodium sulfate, filtered, concentrated, and purified by column chromatography (PE:EA = 1:1) to obtain Compound 134-4 (0.67 g). ESI-MS m/z: 409.1 [M+H]⁺.

### Step 5: Synthesis of Compound 134-5

Compound 134-4 (0.67 g), M4 (0.9 g), potassium carbonate (0.93 g) and CataCxium A Pd G3 (0.21 g) were dissolved in 1,4-dioxane (5 mL) and H₂O (1 mL). Under nitrogen protection, the mixture was reacted at 90°C for 1 h. After the reaction was complete, the reaction solution was diluted with EA and H₂O, extracted with EA, washed once with saturated brine, dried with anhydrous sodium sulfate, concentrated, and purified by column chromatography (EA:DCM = 1:4) to obtain Compound 134-5 (0.27 g). ESI-MS m/z: 562.3 [M+H]⁺.

### Step 6: Synthesis of Compound 134

134-5 (141.0 mg) and M6 (120 mg) were dissolved in MeOH (5 mL). 1 M zinc chloride in tetrahydrofuran (0.3 mL) was added, and the mixture was reacted at 40°C for 1.5 hours. Sodium cyanoborohydride (45 mg) was added to the reaction solution, and the reaction was continued at 60°C for 8 hours. LCMS monitoring indicated the reaction was complete. Saturated ammonium chloride solution (5 mL) was added to the reaction solution. The organic phase was concentrated. DCM (10 mL) was added, and the mixture was washed once with saturated brine. The organic phase was dried with anhydrous sodium sulfate, filtered, concentrated, and purified by preparative liquid chromatography to obtain 70.7 mg of Compound 134. ESI-MS m/z: 637.8 1/2[M+2H]⁺.

Other Compounds in the present application, unless otherwise specified, were prepared by analogous methods to the Compound examples described above.

### Biological Experiments

### Biological Experiment 1: Cell Proliferation Assay

**Table 1**

| Cell Line | KRAS Mutation Type | Culture Medium | Seeding Density |
|---|---|---|---|
| AGS | G12D | 1640 + 10% FBS | 1000 cells/well |
| H358 | G12C | 1640 + 10% FBS | 1000 cells/well |
| SW620 | G12V | 1640 + 10% FBS | 1000 cells/well |
| Mia-Paca2 | G12C | 1640 + 10% FBS | 1000 cells/well |
| Panc0403 | G12D | 1640 + 10% FBS | 1000 cells/well |
| AsPC-1 | G12D | 1640 + 10% FBS | 1000 cells/well |
| MKN-1 | WT-Amp | 1640 + 10% FBS | 1000 cells/well |
| HCT116 | G13D | 1640 + 10% FBS | 2000 cells/well |

Cells with different mutations were seeded in low-attachment transparent 96-well plates at the seeding densities shown in Table 1, and incubated overnight in a cell culture incubator. After the cells adhered, the test compounds were added to the 96-well plates at final concentrations of 20000 nM, 5000 nM, 1250 nM, 312.5 nM, 78.13 nM, 19.53 nM, 4.88 nM, 1.22 nM, 0.31 nM, 0 nM (the final concentration of DMSO was 0.25% in all wells). After culturing at 37°C for 96 h, 50 µL of Cell-titer Glo working solution was added to each well. The plates were shaken to mix and then incubated at room temperature for 10 min. Luminescence values were read on a multimode microplate reader, and the luminescence data were converted into percentage inhibition. The percentage of cell proliferation inhibition was calculated according to the following formula: $Percentage inhibition = \left(1-\left(Test Value-Blank\right)/\left(Max Value-Blank\right)\right) \times 100 %$

("Max value" is from the 0.25% DMSO control wells, "Blank" is from the blank medium control wells, and "Test Value" is from the compound-treated wells).

Curve fitting was performed using GraphPad Prism software to obtain IC₅₀ (nM) values. The results are shown in Table 2.

**Table 2**

| Compound name | IC₅₀(nM) | | | | | | | |
|---|---|---|---|---|---|---|---|---|
| | AGS | SW620 | H358 | Mia-Paca2 | Panc0403 | AsPC-1 | MKN-1 | HCT116 |
| Compound 5 | 20 | 34 | 55 | | | | | |
| Compound 7 | 6.2 | 31 | / | | | | | |
| Compound 57 | 5 | 18 | 20 | | | | | |
| Compound 111 | 30 | 43 | 56 | | | | | |
| Compound 112 | 4.4 | 20 | / | | 6.5 | 2.6 | 0.4 | |
| Compound 113 | 80 | 325 | / | | | | | |
| Compound 114 | 2.7 | 10 | / | | | | | |
| Compound 115 | 0.9 | 8 | / | | 5.9 | 1.9 | 0.7 | 20 |
| Compound 131 | 4.4 | 15 | 8.4 | 5.3 | | | | |
| Compound 133 | 15 | 36 | 40 | 26 | | | | |
| Compound 134 | 34 | / | / | 67 | | | | |

| | | | | | | | | |
|---|---|---|---|---|---|---|---|---|
| "/" indicates not determined. | | | | | | | | |

### Biological Experiment 2: Compound 115 Western Blotting Experiment

### Cell Seeding

AGS cells in the logarithmic growth phase with good growth conditions were taken. The culture medium was discarded, and after one wash with PBS, the cells were digested with 0.25% Trypsin.

Culture medium was added to terminate digestion. The cells were collected in a 15 mL centrifuge tube, and centrifuged at 1000 rpm for 3 min. The culture medium was discarded, and 2 mL culture medium was added to resuspend the cells. The cells were counted and diluted with culture medium to 3.33 × 10⁵ cells/mL (3 × 10⁵ cells/0.9 mL/well).

The cells were seeded at 0.9 mL per well in a 12-well plate and cultured overnight at 37°C, 5% CO₂.

### Administration Treatment

Compound working solution preparation: A certain volume of stock solution was taken and diluted with DMSO to prepare a 1000× intermediate solution. Then, serial dilutions were performed using DMSO to obtain intermediate solutions at various concentrations. Then, 3 µL of each intermediate solution was added to 300 µL culture medium, and mixed by pipetting to make a 10× working solution.

Administration: 100 µL of the corresponding working solution was added to each well. After mixing, the 12-well plate was placed at 37°C, 5% CO₂ and cultured for 24 h.

### Protein Collection and Quantification

Cell lysis solution preparation: Prepared 10× RIPA lysis buffer, 10× phosphatase inhibitor and 25× protease inhibitor were taken out from -20°C in advance and thawed at room temperature. Cell lysis solution was prepared per mL as follows: 760 µL ddH₂O, 100 µL 10× RIPA lysis buffer, 100 µL 10× phosphatase inhibitor, 40 µL 25× protease inhibitor. The solution was mixed and set aside for later use.

Cell lysis: After the drug treatment, the culture medium in the wells was carefully aspirated. 500 µL PBS was added to each well to gently rinse, and the PBS was aspirated. 50 µL of cell lysis solution was slowly added to the center of the well. After lysis on ice for 10 to 15 min, the lysate was collected into a 1.5 mL centrifuge tube, and centrifuged at 15000 rpm for 15 min at 4°C. The supernatant was aspirated into another clean centrifuge tube.

Protein quantification: A clean transparent 96-well plate was taken. 20 µL PBS was added to each well. Then, 5 µL of the collected protein supernatant was added, respectively, and tested in a single well. Then, solution A and solution B of the BCA solution were pre-mixed at a ratio of 50:1. 200 µL of the mixed BCA colorimetric solution was added to each well. The 96-well plate was wrapped in aluminum foil and incubated at 37°C for 30 min. The OD value (562 nM) was measured using an Evisin instrument, and the protein concentration was calculated by reference to a standard curve.

### Performing WB Experiment

1. Adjusting protein concentration: According to the calculation formula, PBS was used to adjust the protein loading amount to 30 µg per well, with a final volume of 10 µL. 2 µL of 6× SDS loading buffer was added to each sample. The samples were boiled at 100°C for 5 min, then centrifuged and cooled to room temperature.
2. The electrophoresis tank was assembled, and electrophoresis buffer was added. 12 µL of each sample was loaded per well. 2.5 µL and 1.5 µL of marker were added to the left and right sides of the samples, respectively. The marker wells and the remaining wells were filled to 12 µL with 1× loading buffer. Electrophoresis was performed at 90 V for approximately 30 min. After the samples were compressed into a single band within the gel, the voltage was increased to 120 V until the blue loading buffer band reached the bottom of the gel.
3. Transfer buffer was prepared in advance according to the ratio 7:2:1 (ddH₂O:methanol:10× transfer buffer), with a total volume of 2 L to 3 L, and cooled at 4°C.
4. After electrophoresis ended, the gel cassette was carefully disassembled and the gel was removed. Unnecessary parts were cut off. The gel was placed on a transfer clamp lined with filter paper. Pre-cut PVDF membrane (PVDF membrane was pre-activated by soaking in methanol and then soaked in transfer buffer) was gently laid over the gel from one edge. A roller was carefully used to roll back and forth to remove bubbles between the gel and the membrane. Then, another layer of filter paper was placed on top. The transfer clamp was carefully tightened, inserted into the transfer tank, and transfer buffer was added. The transfer tank was placed in a slightly larger container surrounded by crushed ice. Transfer was performed at 400 mA for 120 to 150 min (depending on the molecular weight of the protein).
5. Preparing 5% BSA blocking solution: 2.5 g BSA was weighed, dissolved in TBS-T, and the volume was adjusted to 50 mL.
6. Blocking the membrane: After transfer ended, the PVDF membrane was carefully taken out and immersed in 5% BSA solution. The membrane was blocked on a shaker at room temperature for 1 h.
7. Preparing primary antibody: 5% BSA was diluted to 2% for antibody dilution. The primary antibody was diluted according to the recommended ratio in the antibody instructions.
8. The blocked PVDF membrane was transferred to the primary antibody solution and incubated overnight at 4°C.

### Secondary Antibody Incubation

1. The PVDF membrane was removed from the primary antibody solution and washed three times with TBS-T on a shaker for 10 min each time. The used primary antibody can be stored at -20°C for reuse.
2. According to the primary antibody instructions, the corresponding anti-rabbit or anti-mouse secondary antibody was diluted with 2% BSA at a dilution ratio of 4000:1. The PVDF membrane was placed in the secondary antibody solution and incubated at room temperature for 1 to 2 h.
3. After secondary antibody incubation, the membrane was washed three times with TBS-T for 10 min each time.

### Luminescence Procedure

1. A luminometer was turned on, and the BioSense mode was selected.
2. Luminescence solution A and solution B were mixed in equal proportions in a container. The PVDF membrane was dried with lint-free paper and placed in the container. A pipette was used to aspirate the luminescence solution and repeatedly pour it over the PVDF membrane. After the PVDF membrane was fully soaked with the luminescence solution, the membrane was placed in the instrument for exposure.

The remaining two cell lines, Mia-Paca2 and SW620, were tested according to the above method. The results are shown in Figure 1.

### Biological Experiment 3: PK Study of Compound 115 in ICR Mice

### Experimental Methods and Results:

Compound 115 was mixed with the vehicle 10% DMSO/5% Solutol/85% physiological saline, vortexed and sonicated to prepare a 0.6 mg/mL clear solution. Female ICR mice aged 6 to 7 weeks were selected, not fasted, and administered Compound 115 intravenously. Whole blood was collected at certain time points. Drug concentrations were analyzed by LC-MS/MS method, and pharmacokinetic parameters were calculated using Phoenix WinNonlin software (Pharsight, USA). The results are shown in Table 3.

**Table 3. Mouse PK data for intravenous single administration of Compound 115**

| Route | IV |
|---|---|
| Dosing Level (mg/kg) | 3 |
| Formulation | 10%DMSO/5%Solutol/85% physiological saline |
| Sex | female |
| Cl_obs (mL/min/kg) | 13.7 |
| t_{1/2} (h) | 13.5 |
| C₀ (ng/mL) | 7510 |
| AUCₗₐₛₜ (h*ng/mL) | 3714 |
| Vss_obs (L/kg) | 9.23 |

### Biological Experiment 4: PK Study of Compound 115 in SD Rats

### Experimental Methods and Results:

Compound 115 was mixed with the vehicle 10% DMSO/5% Solutol/85% physiological saline, vortexed and sonicated to prepare a 1.5 mg/mL clear solution. Male SD rats aged 6 to 7 weeks were selected, fasted for 8 h, and then administered Compound 115 intravenously. Whole blood was collected at certain time points. Drug concentrations were analyzed by LC-MS/MS method, and pharmacokinetic parameters were calculated using Phoenix WinNonlin software (Pharsight, USA). The results are shown in Table 4.

**Table 4. Rat PK data for intravenous single administration of Compound 115**

| Route | IV |
|---|---|
| Dosing Level (mg/kg) | 3 |
| Formulation | 10%DMSO/5%Solutol/85% physiological saline |
| Sex | male |
| Cl_obs (mL/min/kg) | 5.44 |
| t_{1/2} (h) | 12.7 |
| C₀ (ng/mL) | 17,275 |
| AUCₗₐₛₜ (h*ng/mL) | 9,172 |
| Vss_obs (L/kg) | 5.06 |

### Biological Experiment 5: PK Study of Compound 115 in Beagle Dogs

### Experimental Methods and Results:

Compound 115 was mixed with the vehicle 10% DMSO/5% Solutol/85% physiological saline, vortexed and sonicated to prepare a 1.5 mg/mL clear intravenous injection solution. Beagle dogs weighing approximately 10 kg (half male, half female) were selected, not fasted, and administered Compound 115 intravenously. Whole blood samples were collected intravenously at 5 min, 15 min, 30 min, 1 h, 2 h, 4 h, 6 h, 8 h, 10 h, 24 h, 48 h, and 72 h. Drug concentrations were analyzed by LC-MS/MS method, and pharmacokinetic parameters were calculated using Phoenix WinNonlin software (Pharsight, USA). The results are shown in Table 5. The administration experimental scheme and results are shown in Table 5.

**Table 5. Beagle Dog PK data for intravenous single administration of Compound 115**

| Route | IV |
|---|---|
| Dosing Level (mg/kg) | 3 |
| Formulation | 10%DMSO/5%Solutol/85% physiological saline |
| Sex | one female and one male |
| Cl_obs (mL/min/kg) | 1.96 |
| t_{1/2} (h) | 23.3 |
| C₀ (ng/mL) | 22657 |
| AUCₗₐₛₜ (h*ng/mL) | 20685 |
| Vss_obs (L/kg) | 1.82 |

Although the present application has been fully described by way of its embodiments, it should be noted that various changes and modifications will be apparent to those skilled in the art. Such changes and modifications are to be included within the scope of the appended claims of the present application.

## Claims

1. A compound having an F-L-M structure, or a tautomer, racemate, enantiomer, diastereomer thereof, or a mixture thereof, or a pharmaceutically acceptable salt thereof, wherein:
F is a KRAS protein binding fragment represented by general formula (I); 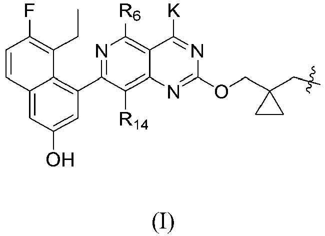
K is N-containing 6- to 12-membered heterocyclyl; K is optionally further substituted by one or more Rₐ;
R₁₄ is halogen;
R₆ is selected from the group consisting of H, hydroxy, amino, cyano, halogen, C₁₋₆ alkyl, C₁₋₆ haloalkyl, C₁₋₆ haloalkoxy and C₁₋₆ alkoxy; R₆ is preferably H; or
R₆ and K, together with the atoms to which they are bound, form 3- to 14-membered heterocyclyl; wherein the 3- to 14-membered heterocyclyl is optionally further substituted by one or more Rₐ;
L is a linking unit for linking F and M; L is selected from the group consisting of 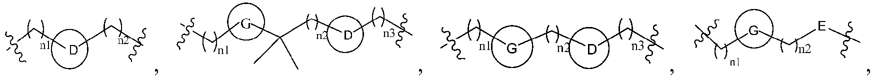 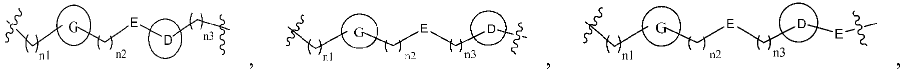 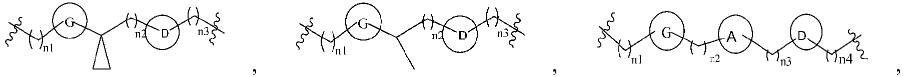 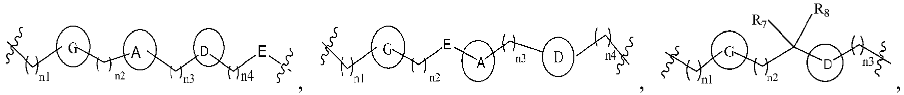 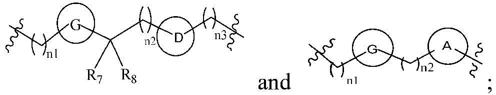
wherein,
ring G and ring D are each independently selected from the group consisting of C₆₋₁₄ aryl, 5-to 14-membered heteroaryl, C₃₋₁₄ cycloalkyl and 3- to 14-membered heterocyclyl; wherein the C₆₋₁₄ aryl, 5- to 14-membered heteroaryl, C₃₋₁₄ cycloalkyl or 3- to 14-membered heterocyclyl is optionally further substituted by one or more Rₐ;
each ring A is independently 3- to 7-membered N-containing heterocyclyl; wherein the 3- to 7-membered N-containing heterocyclyl is optionally further substituted by one or more Rₐ; preferably, each ring A is independently 3- to 6-membered N-containing heterocyclyl; the 3- to 6-membered N-containing heterocyclyl is optionally further substituted by one or more Rₐ;
each E is independently absent or selected from the group consisting of -O-, -NH-, 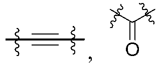 and -NCH₃-;
R₇ and R₈ are each independently selected from the group consisting of H, halogen, hydroxy, amino, oxo, cyano, halogen, C₁₋₆ alkyl, C₁₋₆ haloalkyl, C₁₋₆ haloalkoxy and C₁₋₆ alkoxy; or
R₇ and R₈, together with the atoms to which they are bound, form C₃₋₁₄ cycloalkyl or 3- to 14-membered heterocyclyl; wherein C₃₋₁₄ cycloalkyl or 3- to 14-membered heterocyclyl is optionally further substituted by one or more Rₐ;
n1, n2, n3, and n4 are each independently selected from the group consisting of integers ranging from 0 to 10, and are preferably each independently 0, 1, 2, 3, or 4;
M is a VHL binding fragment represented by general formula (II); 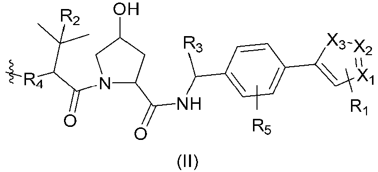
wherein, X₁ is selected from the group consisting of C, CH and N;
X₂ is selected from the group consisting of C, CH and N;
X₃ is selected from the group consisting of N, NH, O and S;
R₁ is selected from the group consisting of H, C₁₋₆ alkyl, C₁₋₆ haloalkyl, C₁₋₆ alkoxy, C₁₋₆ haloalkoxy, halogen and cyano; R₁ is preferably methyl, ethyl, F or cyano;
R₂ is selected from the group consisting of H, C₁₋₆ alkyl and C₁₋₆ haloalkyl; R₂ is preferably H or methyl;
R₃ is selected from the group consisting of H, C₁₋₆ alkyl, C₁₋₆ haloalkyl, C₁₋₆ hydroxyalkyl, -(CH₂)ₙN(R₉)₂, -(CH₂)ₙ-CO-OR₉ and -(CH₂)ₙ-CO-N(R₉)₂, wherein each R₉ is independently selected from the group consisting of H, C₁₋₆ alkyl, C₁₋₆ haloalkyl and C₁₋₆ alkoxy; preferably, the -(CH₂)ₙ-CO-N(R₉)₂ is -(CH₂)ₙ-CO-NH-(CH₂)ₙ-CH₃;
n is selected from the group consisting of 0, 1, 2 and 3;
R₄ is selected from the group consisting of 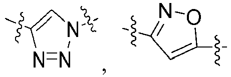 and -NH-;
R₅ is selected from the group consisting of H, C₁₋₆ alkyl and C₁₋₆ haloalkyl;
Rₐ is independently selected from the group consisting of H, hydroxy, amino, oxo, cyano, halogen, C₁₋₆ alkyl, C₁₋₆ haloalkyl, C₁₋₆ alkoxy, -C₀₋₃ alkylene-OR_{b}, -OC(=O)C₁₋₆ alkyl, -C₀₋₃ alkylene-SR_{b}, -C₀₋₃ alkylene-N(R_{b})₂, -C₀₋₃ alkylene-S(=O)R_{b}, -C₀₋₃ alkylene-S(=O)₂R_{d}, -C₀₋₃ alkylene-SR_{b}, -C₀₋₃ alkylene-S(R_{b})₅, -C₀₋₃ alkylene-C(=O)R_{b}, -C₀₋₃ alkylene-C(=O)OR_{b}, -C₀₋₃ alkylene-C(=O)N(R_{b})₂, C₂₋₆ alkenyl, 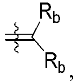 C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl and -C₀₋₃ alkylene-(5- to 18-membered heteroaryl); wherein the hydroxy, amino, C₁₋₆ alkyl, C₁₋₆ haloalkyl, C₁₋₆ alkoxy, C₀₋₃ alkylene, C₂₋₆ alkenyl, C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl or -C₀₋₃ alkylene-(5- to 18-membered heteroaryl) is optionally further substituted by one or more R_{b};
each R_{b} is independently H, halogen, hydroxy, amino, cyano, C₁₋₆ alkyl, C₃₋₆ cycloalkyl, C₁₋₆ haloalkyl, C₃₋₁₄ cycloalkyl, 3- to 14-membered heterocyclyl, C₆₋₁₈ aryl or 5- to 18-membered heteroaryl; wherein hydroxy, amino, C₁₋₆ alkyl, C₃₋₆ cycloalkyl, C₁₋₆ haloalkyl, C₃₋₁₄ cycloalkyl, 3- to 14-membered heterocyclyl, C₆₋₁₈ aryl or 5- to 18-membered heteroaryl is optionally further substituted by one or more halogen, C₁₋₆ alkyl or C₁₋₆ haloalkyl; or two R_{b} bound to one atom, together with the atom to which they are bound, form C₃₋₁₄ cycloalkyl, 3- to 14-membered heterocyclyl, C₆₋₁₈ aryl or 5- to 18-membered heteroaryl, and the C₃₋₁₄ cycloalkyl, 3- to 14-membered heterocyclyl, C₆₋₁₈ aryl or 5- to 18-membered heteroaryl is optionally further substituted by one or more halogen, amino, hydroxy, cyano, C₁₋₆ alkyl, C₃₋₆ cycloalkyl or C₁₋₆ haloalkyl.

2. The compound according to claim 1, or the tautomer, racemate, enantiomer, diastereomer thereof, or the mixture thereof, or the pharmaceutically acceptable salt thereof, wherein:
F is a KRAS protein binding fragment represented by general formula (I); 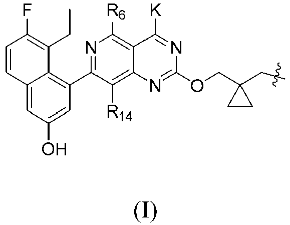
K is N-containing 6- to 12-membered heterocyclyl; K is optionally further substituted by one or more Rₐ;
R₁₄ is halogen;
R₆ is selected from the group consisting of H, hydroxy, amino, cyano, halogen, C₁₋₆ alkyl, C₁₋₆ haloalkyl and C₁₋₆ alkoxy; R₆ is preferably H; or
R₆ and K, together with the atoms to which they are bound, form 3- to 14-membered heterocyclyl; wherein 3- to 14-membered heterocyclyl is optionally further substituted by one or more Rₐ;
L is a linking unit for linking F and M; L is selected from the group consisting of 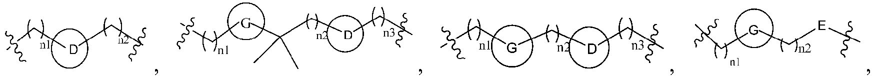 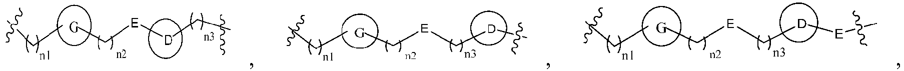 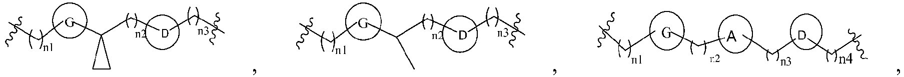 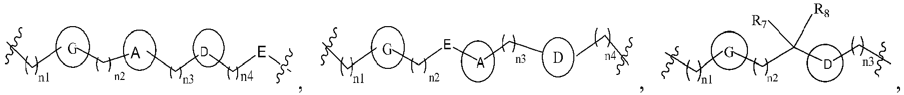 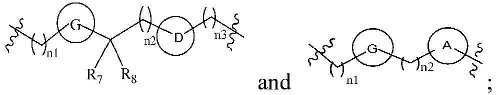
wherein,
ring G and ring D are each independently selected from the group consisting of C₆₋₁₄ aryl, 5-to 14-membered heteroaryl, C₃₋₁₄ cycloalkyl and 3- to 14-membered heterocyclyl; wherein the C₆₋₁₄ aryl, 5- to 14-membered heteroaryl, C₃₋₁₄ cycloalkyl or 3- to 14-membered heterocyclyl is optionally further substituted by one or more Rₐ;
each ring A is independently 3- to 7-membered N-containing heterocyclyl; wherein the 3- to 7-membered N-containing heterocyclyl is optionally further substituted by one or more Rₐ; preferably, each ring A is independently 3- to 6-membered N-containing heterocyclyl; wherein the 3- to 6-membered N-containing heterocyclyl is optionally further substituted by one or more Rₐ;
each E is independently absent or selected from the group consisting of -O-, -NH-, 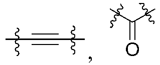 and -NCH₃-;
R₇ and R₈ are each independently selected from the group consisting of H, halogen, hydroxy, amino, oxo, cyano, halogen, C₁₋₆ alkyl, C₁₋₆ haloalkyl and C₁₋₆ alkoxy; or
R₇ and R₈, together with the atoms to which they are bound, form C₃₋₁₄ cycloalkyl or 3- to 14-membered heterocyclyl; wherein the C₃₋₁₄ cycloalkyl or 3- to 14-membered heterocyclyl is optionally further substituted by one or more Rₐ;
n1, n2, n3 and n4 are each independently selected from the group consisting of integers ranging from 0 to 10, and are preferably each independently 0, 1, 2, 3 or 4;
M is a VHL binding fragment represented by general formula (II); 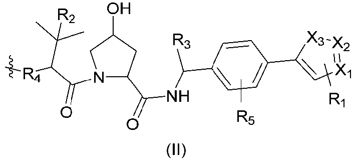
wherein, X₁ is selected from the group consisting of C, CH and N;
X₂ is selected from the group consisting of C, CH and N;
X₃ is selected from the group consisting of N, NH, O and S;
R₁ is selected from the group consisting of H, C₁₋₆ alkyl, halogen and cyano, and R₁ is preferably methyl, ethyl, F or cyano;
R₂ is selected from the group consisting of H and C₁₋₆ alkyl, and R₂ is preferably H or methyl;
R₃ is selected from the group consisting of H, C₁₋₆ alkyl, C₁₋₆ hydroxyalkyl, -(CH₂)ₙN(R₉)₂, -(CH₂)ₙ-CO-OR₉ and -(CH₂)ₙ-CO-N(R₉)₂, wherein each R₉ is independently selected from the group consisting of H, C₁₋₆ alkyl, C₁₋₆ haloalkyl and C₁₋₆ alkoxy; preferably, the -(CH₂)ₙ-CO-N(R₉)₂ is -(CH₂)ₙ-CO-NH-(CH₂)ₙ-CH₃;
n is selected from the group consisting of 0, 1, 2 and 3;
R₄ is selected from the group consisting of 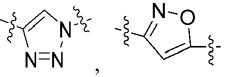 and -NH-;
R₅ is selected from the group consisting of H, C₁₋₆ alkyl and C₁₋₆ haloalkyl;
Rₐ is independently selected from the group consisting of H, hydroxy, amino, oxo, cyano, halogen, C₁₋₆ alkyl, C₁₋₆ haloalkyl, C₁₋₆ alkoxy, -C₀₋₃ alkylene-OR_{b}, -OC(=O)C₁₋₆ alkyl, -C₀₋₃ alkylene-SR_{b}, -C₀₋₃ alkylene-N(R_{b})₂, -C₀₋₃ alkylene-S(=O)R_{b}, -C₀₋₃ alkylene-S(=O)₂R_{d}, -C₀₋₃ alkylene-SR_{b}, -C₀₋₃ alkylene-S(R_{b})₅, -C₀₋₃ alkylene-C(=O)R_{b}, -C₀₋₃ alkylene-C(=O)OR_{b}, -C₀₋₃ alkylene-C(=O)N(R_{b})₂, C₂₋₆ alkenyl, 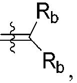 C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl and -C₀₋₃ alkylene-(5- to 18-membered heteroaryl); wherein C₁₋₆ alkyl, C₁₋₆ haloalkyl, C₁₋₆ alkoxy, C₂₋₆ alkenyl, C₂₋₆ alkynyl, -C₀₋₃ alkylene-C₃₋₁₄ cycloalkyl, -C₀₋₃ alkylene-(3- to 14-membered heterocyclyl), -C₀₋₃ alkylene-C₆₋₁₈ aryl or -C₀₋₃ alkylene-(5- to 18-membered heteroaryl) is optionally further substituted by one or more R_{b};
each R_{b} is independently H, halogen, hydroxy, cyano, C₁₋₆ alkyl, C₃₋₆ cycloalkyl, C₁₋₆ haloalkyl, C₃₋₁₄ cycloalkyl, 3- to 14-membered heterocyclyl, C₆₋₁₈ aryl or 5- to 18-membered heteroaryl; wherein the C₁₋₆ alkyl, C₃₋₆ cycloalkyl, C₁₋₆ haloalkyl, C₃₋₁₄ cycloalkyl, 3- to 14-membered heterocyclyl, C₆₋₁₈ aryl or 5- to 18-membered heteroaryl is optionally further substituted by one or more halogen, C₁₋₆ alkyl or C₁₋₆ haloalkyl; or two R_{b} bound to one atom, together with the atom to which they are bound, form C₃₋₁₄ cycloalkyl, 3- to 14-membered heterocyclyl, C₆₋₁₈ aryl or 5- to 18-membered heteroaryl, wherein C₃₋₁₄ cycloalkyl, 3- to 14-membered heterocyclyl, C₆₋₁₈ aryl or 5- to 18-membered heteroaryl is optionally further substituted by one or more halogen, amino, hydroxy, cyano, C₁₋₆ alkyl, C₃₋₆ cycloalkyl or C₁₋₆ haloalkyl.

3. The compound according to claim 1 or 2, or the tautomer, racemate, enantiomer, diastereomer thereof, or the mixture thereof, or the pharmaceutically acceptable salt thereof, wherein K is selected from the group consisting of

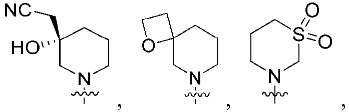

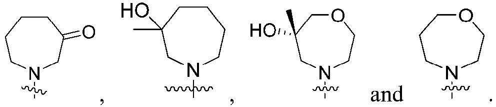

4. The compound according to any one of claims 1 to 3, or the tautomer, racemate, enantiomer, diastereomer thereof, or the mixture thereof, or the pharmaceutically acceptable salt thereof , wherein K is selected from the group consisting of

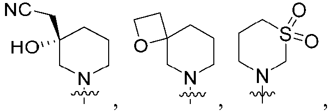

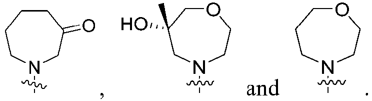

5. The compound according to any one of claims 1 to 4, or the tautomer, racemate, enantiomer, diastereomer thereof, or the mixture thereof, or the pharmaceutically acceptable salt thereof, wherein K is selected from the group consisting of 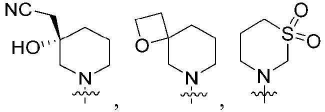 and

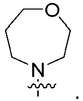

6. The compound according to any one of claims 1 to 5, or the tautomer, racemate, enantiomer, diastereomer thereof, or the mixture thereof, or the pharmaceutically acceptable salt thereof, wherein F is

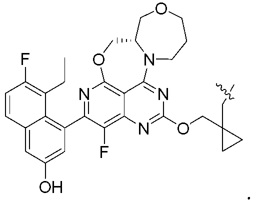

7. The compound according to any one of claims 1 to 6, or the tautomer, racemate, enantiomer, diastereomer thereof, or the mixture thereof, or the pharmaceutically acceptable salt thereof, wherein R₄ is

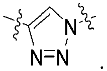

8. The compound according to any one of claims 1 to 6, or the tautomer, racemate, enantiomer, diastereomer thereof, or the mixture thereof, or the pharmaceutically acceptable salt thereof, wherein R₄ is

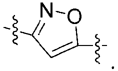

9. The compound according to any one of claims 1 to 6, or the tautomer, racemate, enantiomer, diastereomer thereof, or the mixture thereof, or the pharmaceutically acceptable salt thereof, wherein R₄ is -NH-.

10. The compound according to any one of claims 1 to 9, or the tautomer, racemate, enantiomer, diastereomer thereof, or the mixture thereof, or the pharmaceutically acceptable salt thereof, wherein ring G or ring D in L is each independently selected from the group consisting of C₆₋₁₄ aryl, 5- to 14-membered heteroaryl, C₃₋₁₄ cycloalkyl and 3- to 14-membered heterocyclyl; wherein the C₆₋₁₄ aryl, 5- to 14-membered heteroaryl, C₃₋₁₄ cycloalkyl or 3- to 14-membered heterocyclyl is optionally further substituted by one or more halogen or C₁₋₃ alkyl;
C₆₋₁₄ aryl is preferably 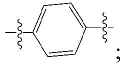
5- to 14-membered heteroaryl is preferably selected from the group consisting of 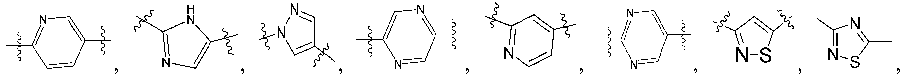 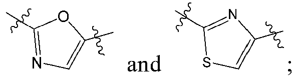
3- to 14-membered heterocyclyl is preferably selected from the group consisting of 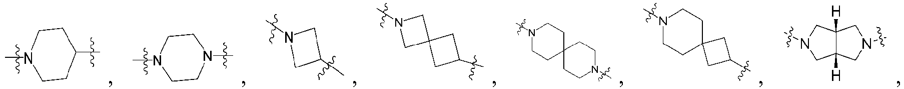 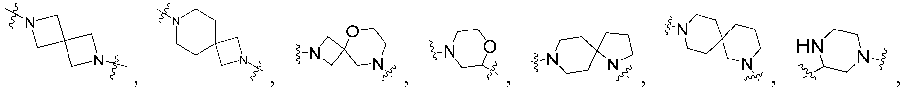 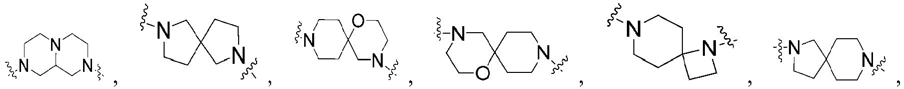 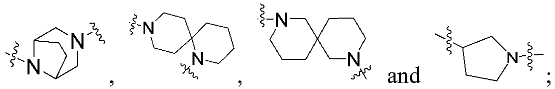
C₃₋₁₄ cycloalkyl is preferably selected from the group consisting of 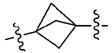
and

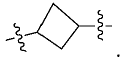

11. The compound according to any one of claims 1 to 10, or the tautomer, racemate, enantiomer, diastereomer thereof, or mixture thereof, or the pharmaceutically acceptable salt thereof, wherein each ring D is independently 5- to 6-membered nitrogen-containing heteroaryl; wherein the 5- to 6-membered nitrogen-containing heteroaryl is optionally further substituted by one or more Rₐ;
each ring G is independently selected from the group consisting of C₆₋₁₄ aryl, 5- to 14-membered heteroaryl, C₃₋₁₄ cycloalkyl and 3- to 14-membered heterocyclyl; wherein the C₆₋₁₄ aryl, 5- to 14-membered heteroaryl, C₃₋₁₄ cycloalkyl or 3- to 14-membered heterocyclyl is optionally further substituted by one or more Rₐ;
each ring A is independently 3- to 7-membered N-containing heterocyclyl; wherein the 3- to 7-membered N-containing heterocyclyl is optionally further substituted by one or more Rₐ;
each E is independently absent or selected from the group consisting of -O-, -NH-, 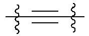 and -NCH₃-;
R₇ and R₈ are each independently selected from the group consisting of H, halogen, hydroxy, amino, oxo, cyano, halogen, C₁₋₆ alkyl, C₁₋₆ haloalkyl and C₁₋₆ alkoxy; or
R₇ and R₈, together with the atoms to which they are bound, form C₃₋₁₄ cycloalkyl or 3- to 14-membered heterocyclyl; wherein the C₃₋₁₄ cycloalkyl or 3- to 14-membered heterocyclyl is optionally further substituted by one or more Rₐ;
n1, n2, n3 and n4 are each independently selected from the group consisting of integers ranging from 0 to 10, and are preferably each independently 0, 1, 2, 3 or 4.

12. The compound according to any one of claims 1 to 11, or the tautomer, racemate, enantiomer, diastereomer thereof, or the mixture thereof, or the pharmaceutically acceptable salt thereof, wherein each ring A is independently 3- to 7-membered N-containing heterocyclyl; wherein the 3- to 7-membered N-containing heterocyclyl is optionally further substituted by one or more halogen or C₁₋₃ alkyl; preferably, each ring A is independently 3- to 6-membered N-containing heterocyclyl; wherein the 3- to 6-membered N-containing heterocyclyl is optionally further substituted by one or more halogen or C₁₋₃ alkyl.

13. The compound according to claim 12, or the tautomer, racemate, enantiomer, diastereomer thereof, or the mixture thereof, or the pharmaceutically acceptable salt thereof, wherein the 3- to 7-membered N-containing heterocyclyl is

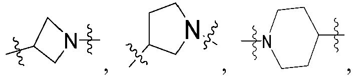

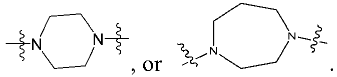

14. The compound according to any one of claims 1 to 13, or the tautomer, racemate, enantiomer, diastereomer thereof, or the mixture thereof, or the pharmaceutically acceptable salt thereof, wherein E is -O-.

15. The compound according to any one of claims 1 to 14, or the tautomer, racemate, enantiomer, diastereomer thereof, or the mixture thereof, or the pharmaceutically acceptable salt thereof, wherein E is -NH-.

16. The compound according to any one of claims 1 to 15, or the tautomer, racemate, enantiomer, diastereomer thereof, or the mixture thereof, or the pharmaceutically acceptable salt thereof, wherein E is -NCH₃-.

17. The compound according to any one of claims 1 to 16, or the tautomer, racemate, enantiomer, diastereomer thereof, or the mixture thereof, or the pharmaceutically acceptable salt thereof, wherein ring D is independently 5- to 6-membered nitrogen-containing heteroaryl, and the 5- to 6-membered heteroaryl is selected from the group consisting of 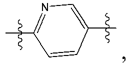 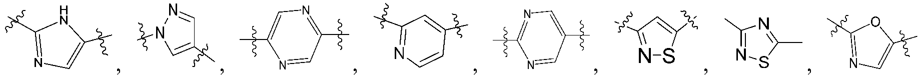 and 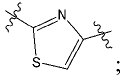 wherein, the 5- to 6-membered nitrogen-containing heteroaryl is optionally further substituted by one or more Rₐ; and Rₐ is selected from the group consisting of **H,** hydroxy, amino, oxo, cyano, halogen, C₁₋₆ alkyl, C₁₋₆ haloalkyl and C₁₋₆ alkoxy.

18. The compound according to any one of claims 1 to 17, or the tautomer, racemate, enantiomer, diastereomer thereof, or the mixture thereof, or the pharmaceutically acceptable salt thereof, wherein ring G is independently selected from the group consisting of C₆₋₁₄ aryl, 5- to 14-membered heteroaryl, C₃₋₁₄ cycloalkyl and 3- to 14-membered heterocyclyl; wherein the C₆₋₁₄ aryl, 5- to 14-membered heteroaryl, C₃₋₁₄ cycloalkyl or 3- to 14-membered heterocyclyl is optionally further substituted by one or more Rₐ;
C₆₋₁₄ aryl is preferably 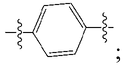
5- to 14-membered heteroaryl is preferably selected from the group consisting of 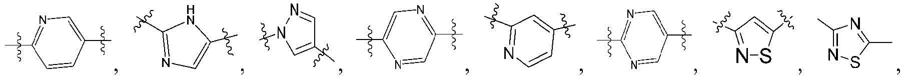 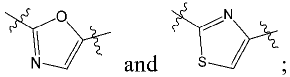 3- to 14-membered heterocyclyl is preferably selected from the group consisting of 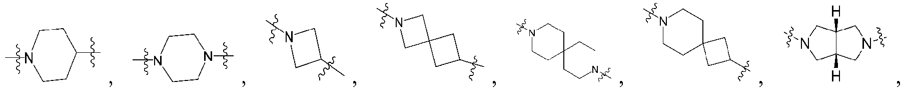 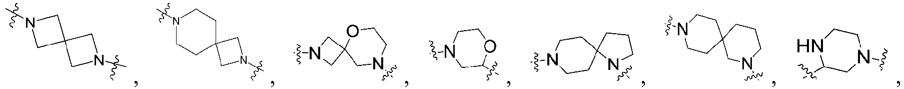 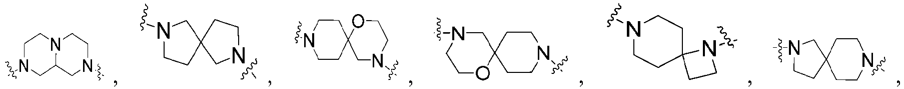 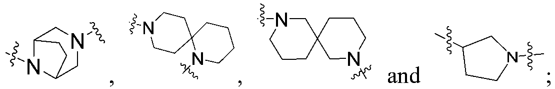
C₃₋₁₄ cycloalkyl is preferably selected from the group consisting of 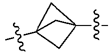 and 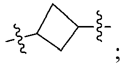
Rₐ is selected from the group consisting of H, hydroxy, amino, oxo, cyano, halogen, C₁₋₆ alkyl, C₁₋₆ haloalkyl and C₁₋₆ alkoxy.

19. The compound according to any one of claims 1 to 18, or the tautomer, racemate, enantiomer, diastereomer thereof, or the mixture thereof, or the pharmaceutically acceptable salt thereof, wherein ring G is independently 3- to 14-membered heterocyclyl, wherein 3- to 14-membered heterocyclyl is optionally further substituted by one or more Rₐ;
3- to 14-membered heterocyclyl is preferably selected from the group consisting of 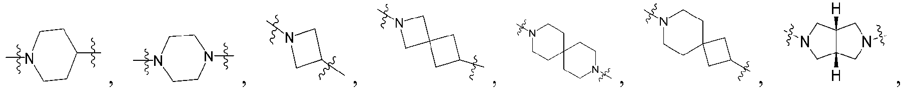 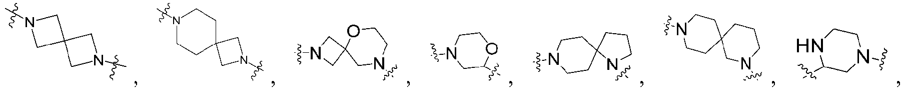 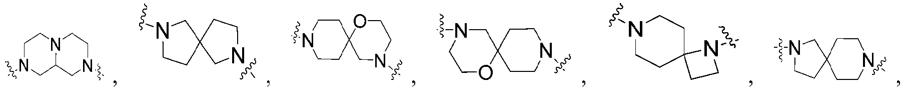 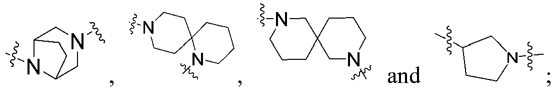
Rₐ is selected from the group consisting of H, hydroxy, amino, oxo, cyano, halogen, C₁₋₆ alkyl, C₁₋₆ haloalkyl and C₁₋₆ alkoxy.

20. The compound according to any one of claims 1 to 19, or the tautomer, racemate, enantiomer, diastereomer thereof, or the mixture thereof, or the pharmaceutically acceptable salt thereof, wherein L is selected from the group consisting of

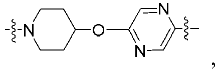

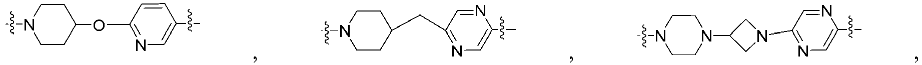

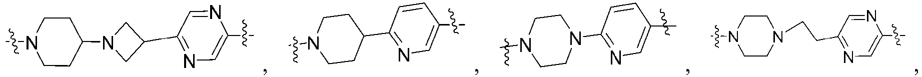

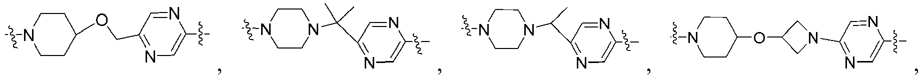

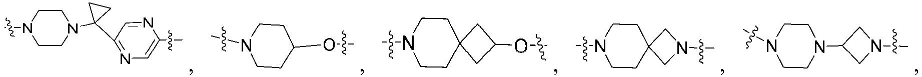

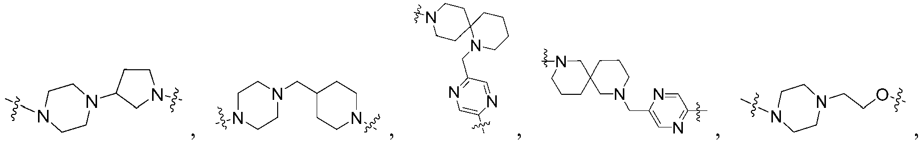

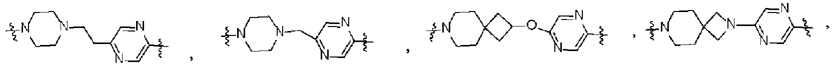

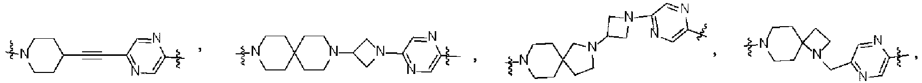

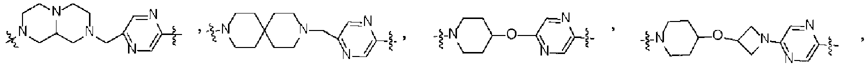

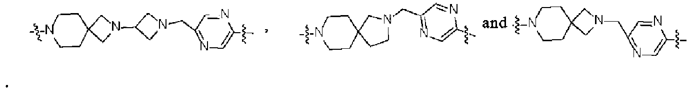

21. The compound according to any one of claims 1 to 19, or the tautomer, racemate, enantiomer, diastereomer thereof, or the mixture thereof, or the pharmaceutically acceptable salt thereof, wherein L is selected from the group consisting of

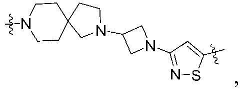

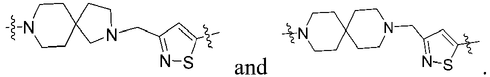

22. The compound according to any one of claims 1 to 21, or the tautomer, racemate, enantiomer, diastereomer thereof, or the mixture thereof, or the pharmaceutically acceptable salt thereof, wherein R₃ is selected from the group consisting of H, methyl, hydroxymethyl, -CH₂-CH₂-CO-O-CH₃, -CH₂-N(CH₃)₂ and -CH₂-CO-NH-CH₃.

23. The compound according to any one of claims 1 to 22, or the tautomer, racemate, enantiomer, diastereomer thereof, or the mixture thereof, or the pharmaceutically acceptable salt thereof, wherein M is selected from the group consisting of

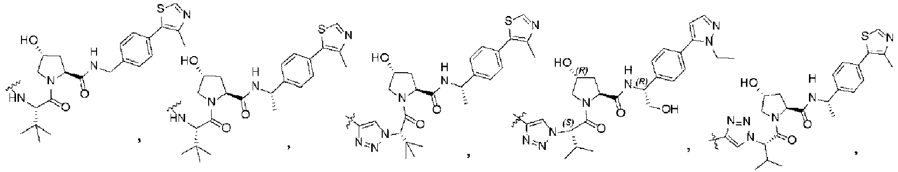

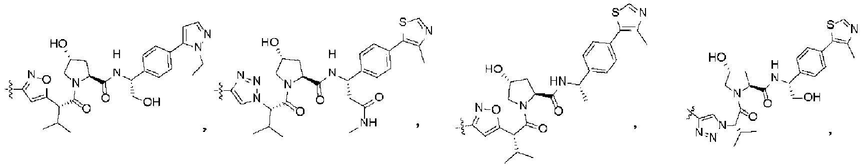

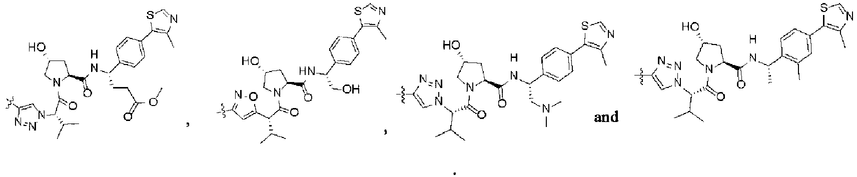

24. The compound according to claim 1, or the tautomer, racemate, enantiomer, diastereomer thereof, or the mixture thereof, or the pharmaceutically acceptable salt thereof, wherein the compound having the F-L-M structure is selected from the group consisting of the following compounds:

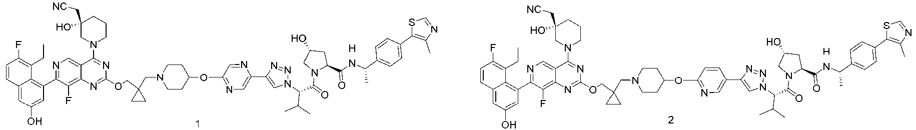

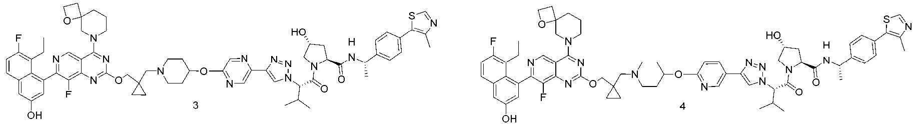

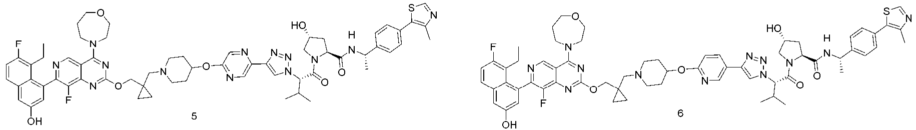

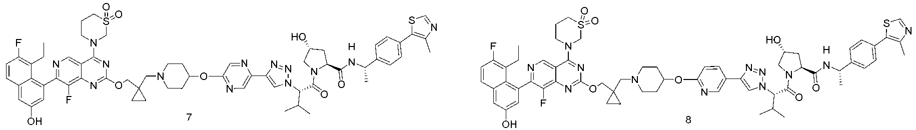

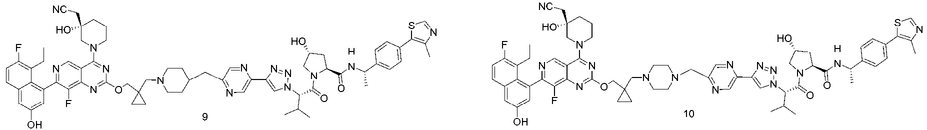

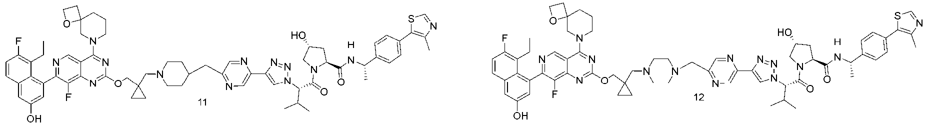

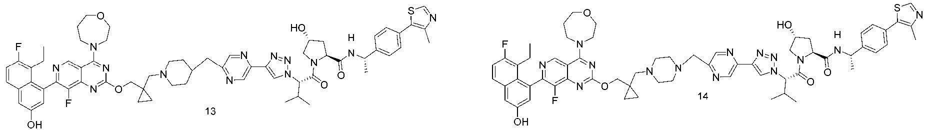

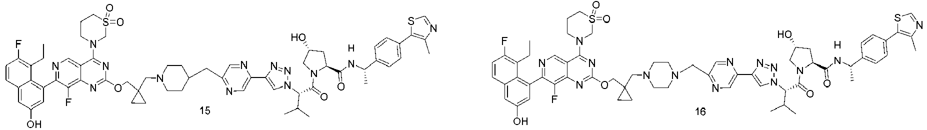

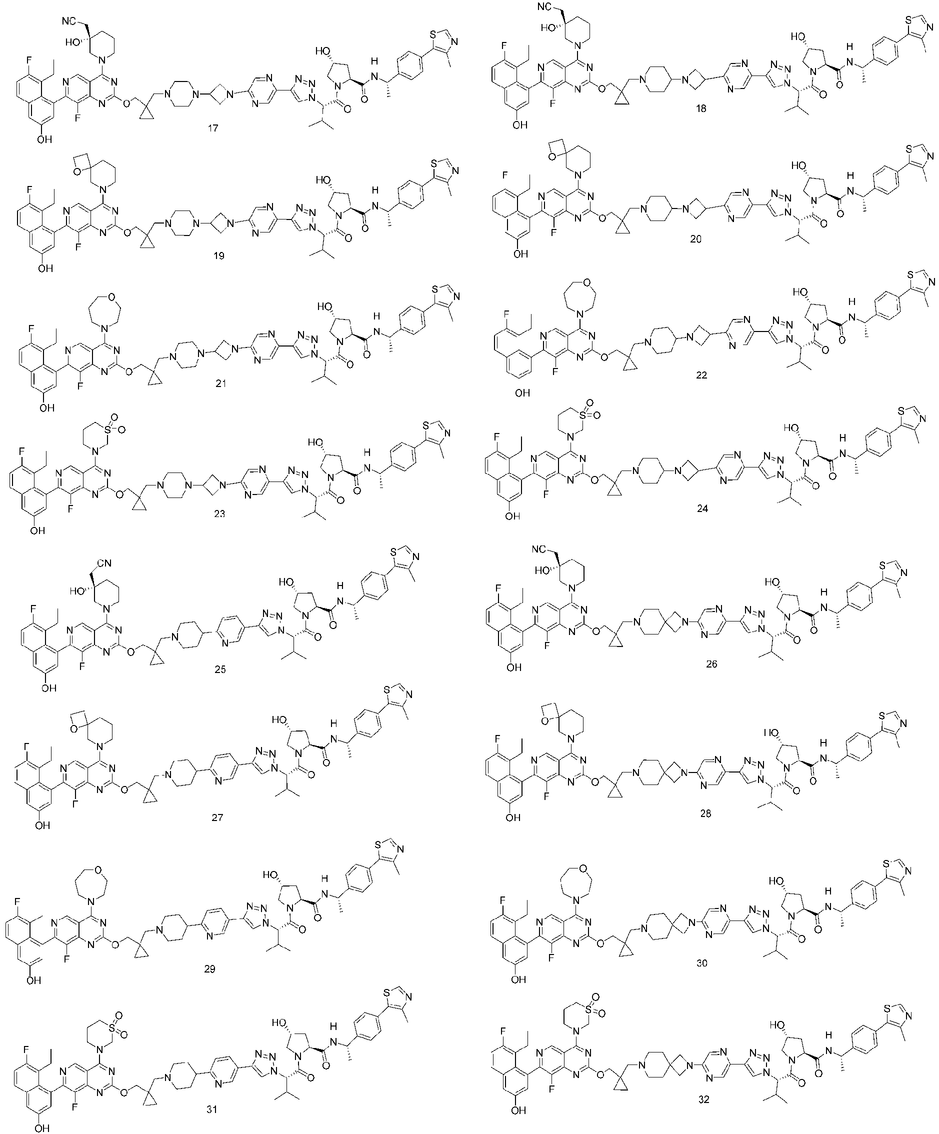

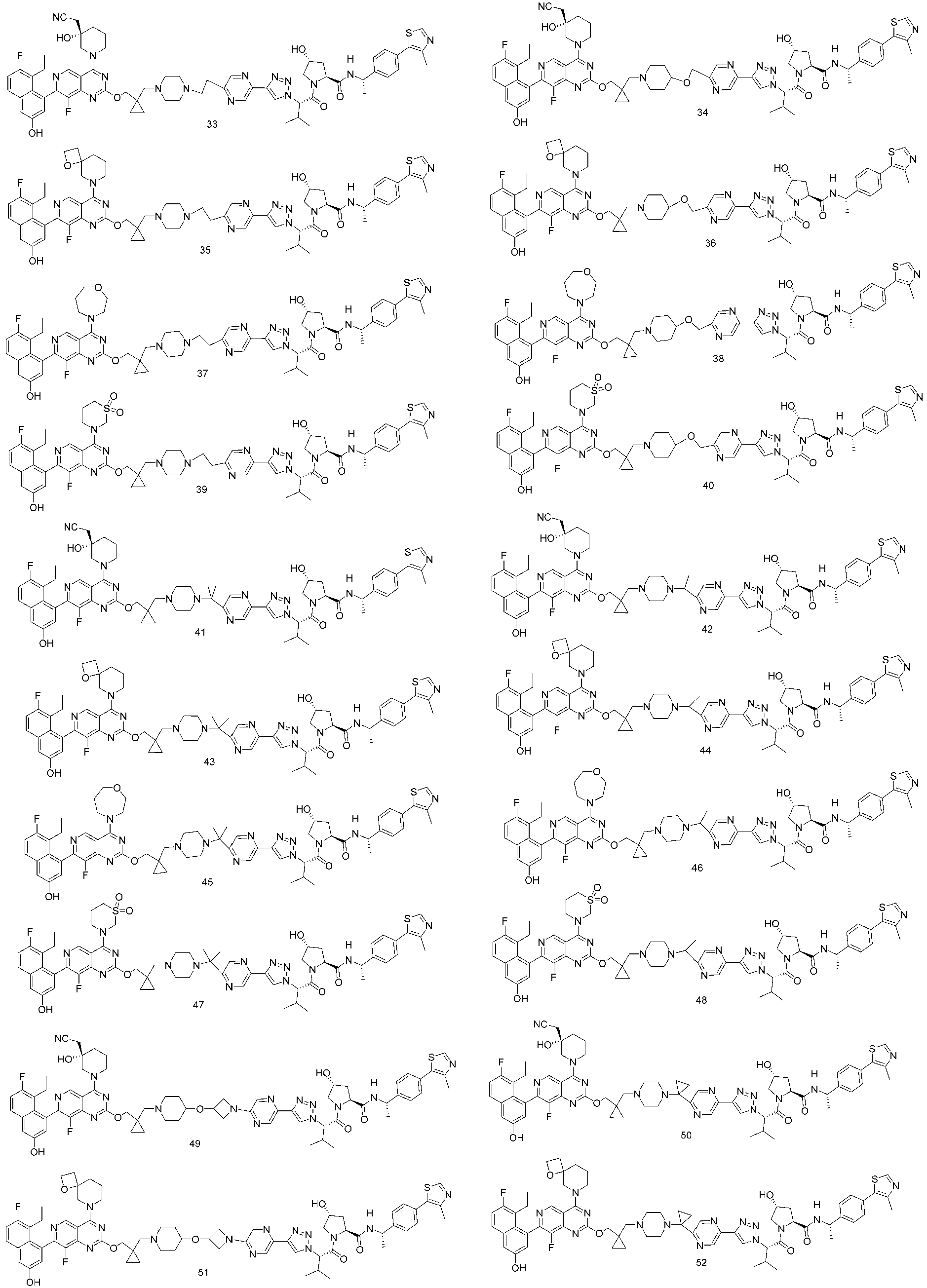

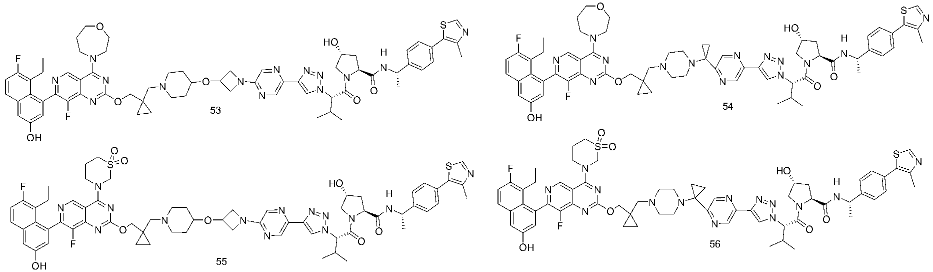

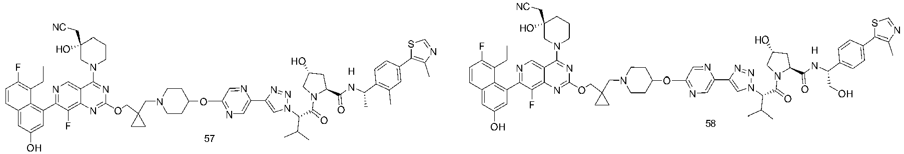

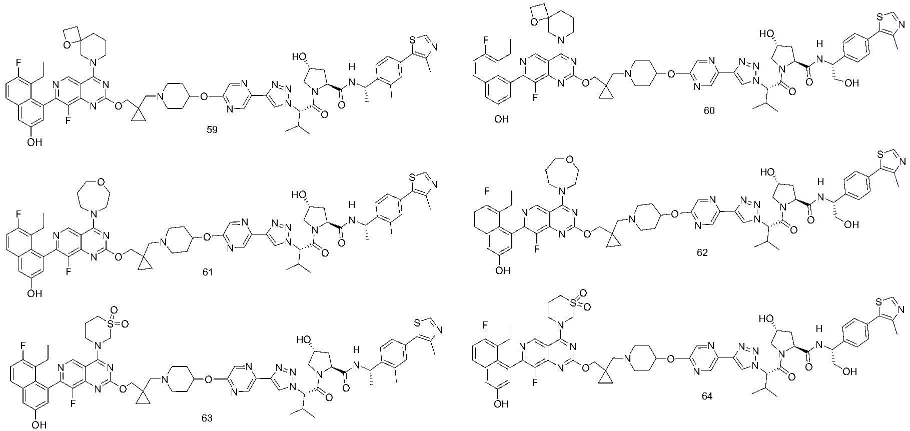

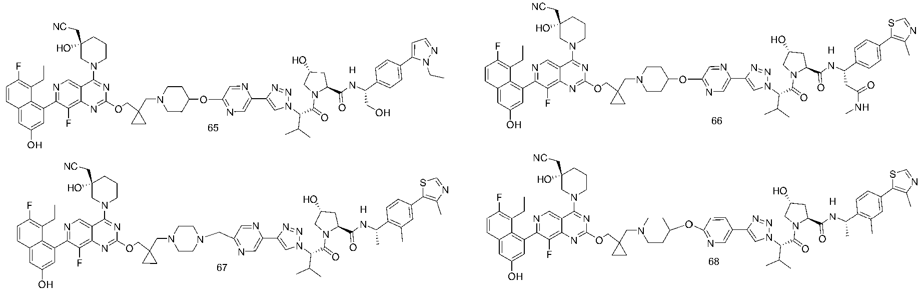

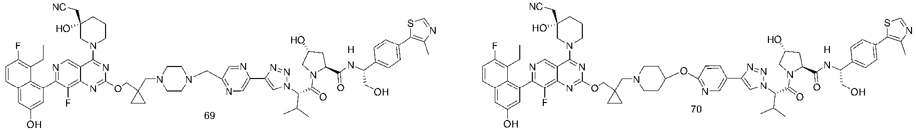

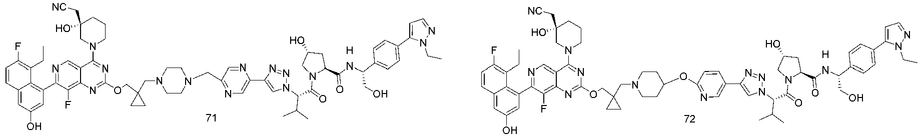

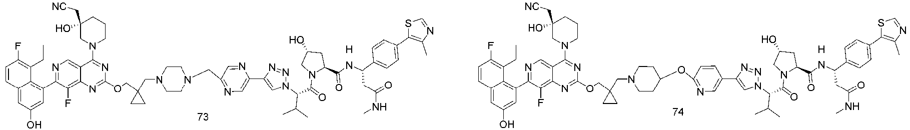

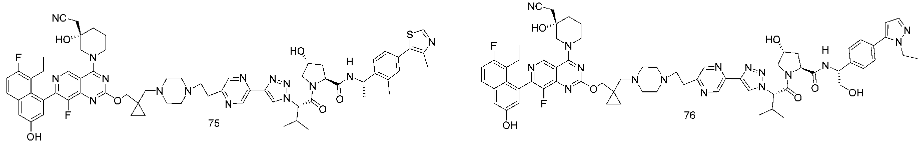

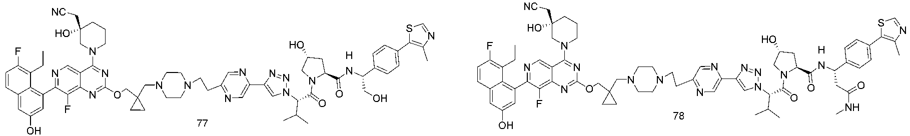

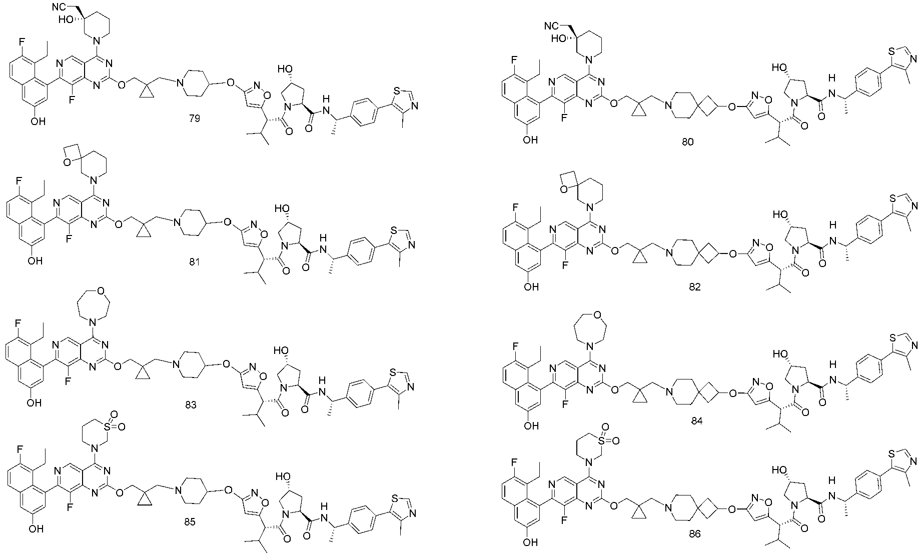

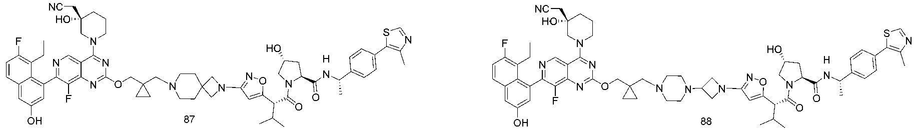

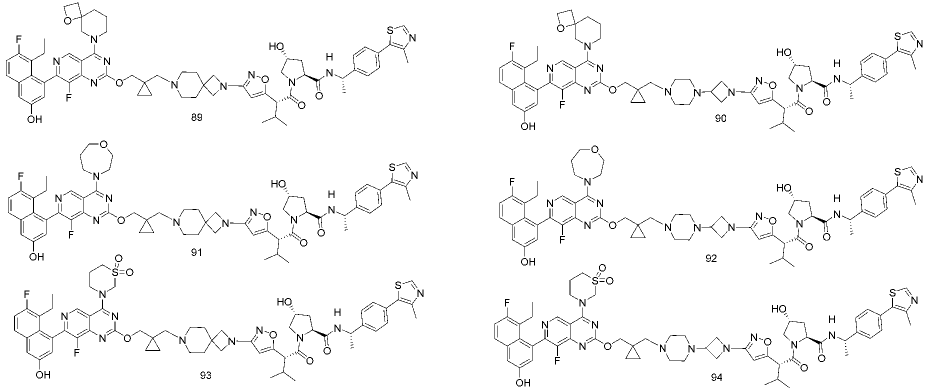

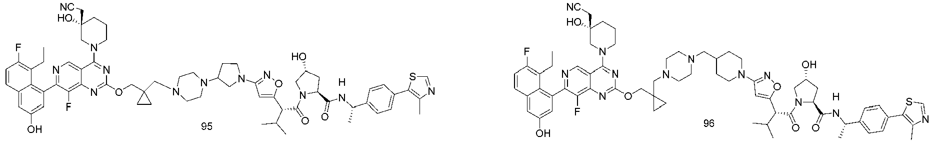

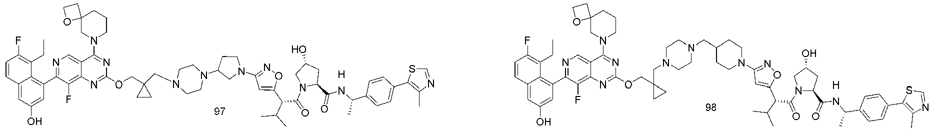

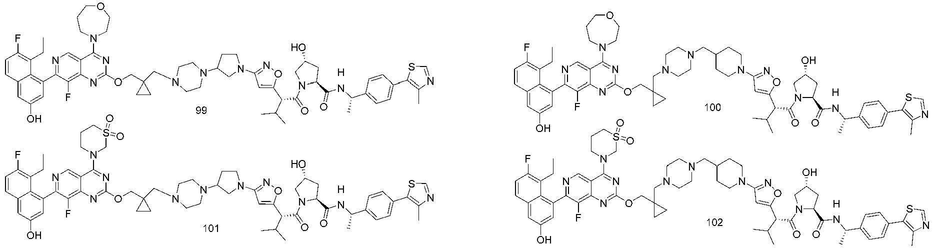

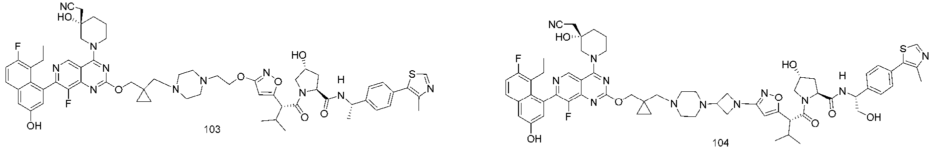

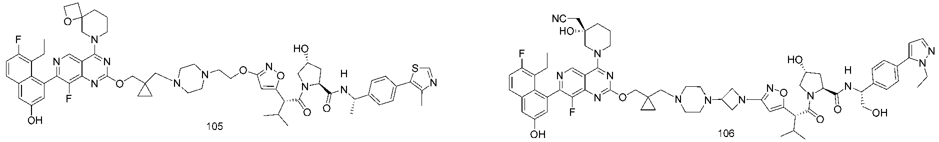

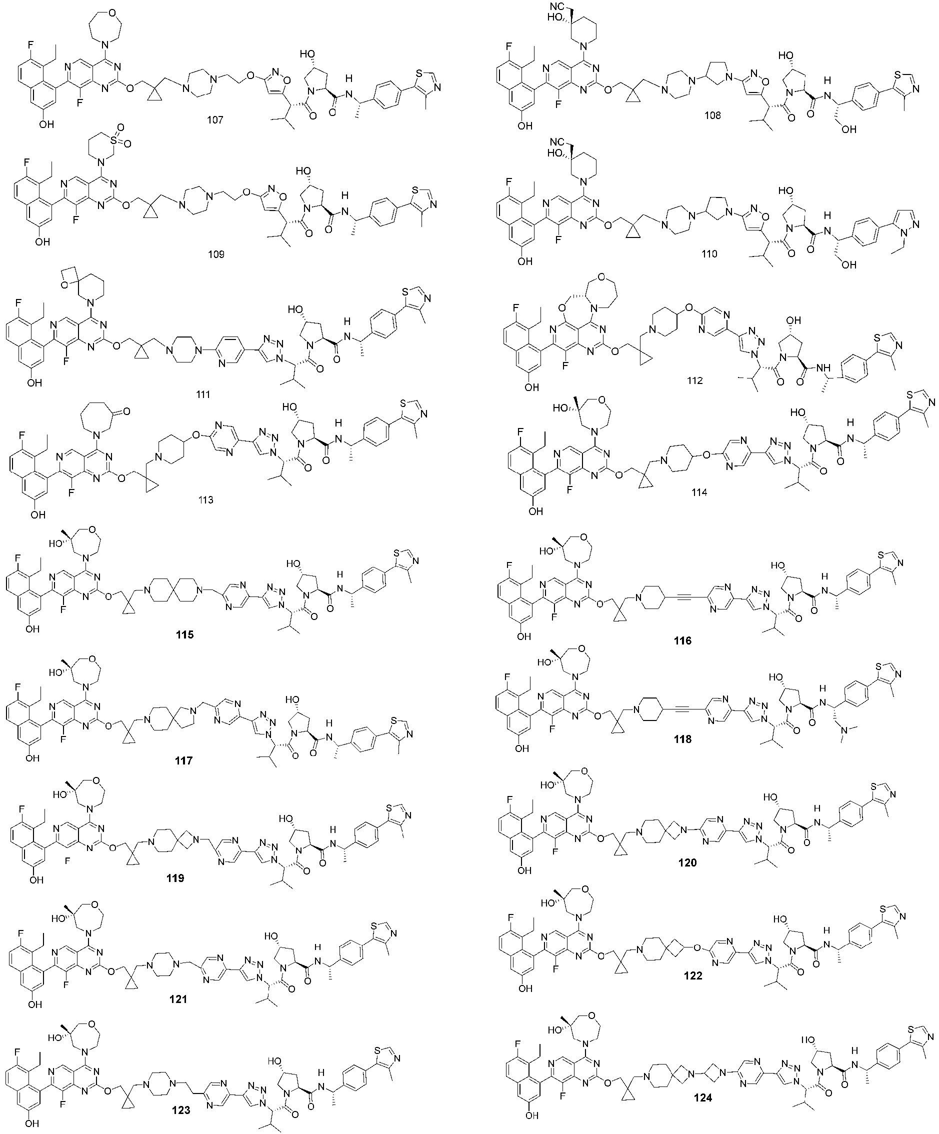

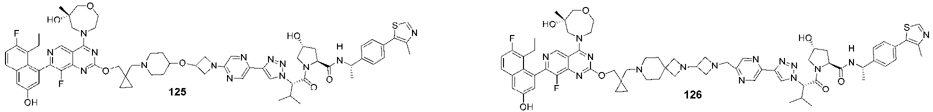

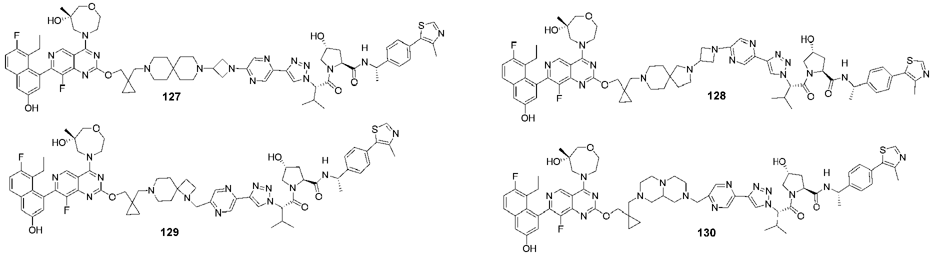

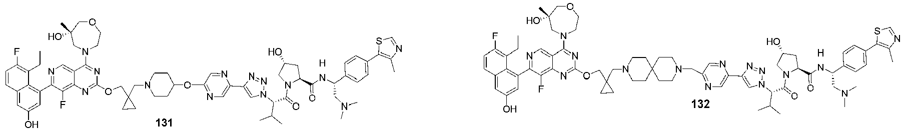

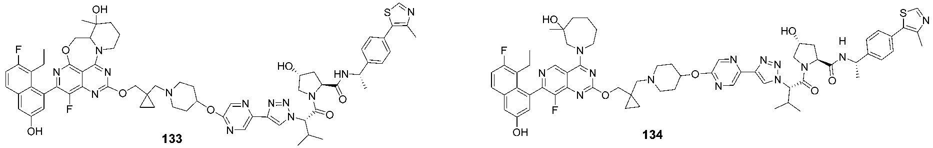

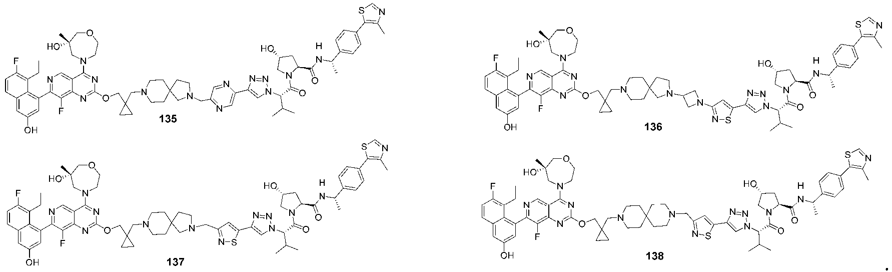

25. A pharmaceutical composition, wherein the pharmaceutical composition comprises the compound according to any one of claims 1 to 24, or the tautomer, racemate, enantiomer, diastereomer thereof, or the mixture thereof, or the pharmaceutically acceptable salt thereof.

26. Use of the compound according to any one of claims 1 to 24, or the tautomer, racemate, enantiomer, diastereomer thereof, or the mixture thereof, or the pharmaceutically acceptable salt thereof, or the pharmaceutical composition according to claim 25 in the manufacture of a medicament for regulating ubiquitination and degradation of KRAS protein in a subject.

27. Use of the compound according to any one of claims 1 to 24, or the tautomer, racemate, enantiomer, diastereomer thereof, or the mixture thereof, or the pharmaceutically acceptable salt thereof, or the pharmaceutical composition according to claim 25 in the manufacture of a medicament for treating and/or preventing a KRAS protein-mediated disease or a KRAS protein-dependent disease, wherein the KRAS protein-mediated disease is preferably a tumor.

28. The use according to claim 27, wherein the disease is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumor, intrahepatic cholangiocarcinoma, myelodysplastic syndrome, malignant glioma, prostate cancer, thyroid cancer, Schwann cell tumor, pulmonary squamous cell carcinoma, lichenoid keratosis, synovial sarcoma, skin cancer, pancreatic cancer, testicular cancer and liposarcoma.
